# EUROPEAN PATENT APPLICATION

(11) **EP 4 830 734 A1**
(43) Date of publication of application: **28.10.2026**
(21) Application number: 25172764.0
(22) Date of filing: 25.04.2025
(51) Int. Cl.: A61K 47/68, A61P 35/00

(54) **AN IMMUNOCONJUGATE COMPRISING AN ANTIBODY THAT BINDS TO EGFR AND MUC1 AND CORRESPONDING MEDICAL USES**

(71) Applicant: Merck Patent GmbH, 64293 Darmstadt (DE)
(72) Inventor: The designation of the inventor has not yet been filed
(74) Representative: Maiwald GmbH

(57) **Abstract**

An immunoconjugate comprising: (a) a bispecific antibody that binds to EGFR and MUC1, (b) a growth inhibiting agent, and (c) a linker covalently linking (a) and (b).

## Description

### Background

Despite the recent successes with targeted therapies and immune-oncology checkpoint inhibitors in the treatment of cancer, there is still a high medical need for improved chemotherapeutics used as mono- or combination therapies.

Epidermal growth factor receptor (EGFR; also known as ErbB1 or HER1) is a transmembrane protein that is overexpressed in several epithelial cancers. Some EGFR mutations, including deletion mutations, point mutations, insertion mutations, and gene amplifications have been associated with cancer. Some EGFR mutations, as well as EGFR overexpression, are associated with poor prognosis and/or resistance to targeted EGFR inhibitors and other receptor tyrosine kinase inhibitors. Several novel pathways leading to escape from anti-EGFR therapy have recently been reported, highlighting the challenges of anti-EGFR therapy.

MUC1, a Type I transmembrane glycoprotein, is expressed on many cancer cells, but also exhibits some expression in normal cells. In tumor cells, MUC1 co-localizes and interacts with EGFR, and their interaction blocks ligand-activated EGFR degradation. The bispecific antibody-drug conjugate of the pharmaceutical composition targets both MUC1 and EGFR. By targeting both MUC1 and EGFR with the same antibody, the presently disclosed antibody-drug conjugate not only enhance antibody internalization and tumor inhibition, they also enable higher specificity of binding to cancer cells, which may thereby reduce effects on normal cells.

It is an object of the present invention to provide a novel immunoconjugate or antibody drug conjugate (ADC) for the treatment of EGFR and/or MUC1 expressing cancers.

### Summary of the invention

The invention provides a new immunoconjugate that provides superior effects and addresses these needs. The inventors have found that the new immunoconjugate of the invention comprising specific components provides high efficacy against a variety of cancers.

The inventors have surprisingly found that the immunoconjugate of the present invention induces marked anti-tumour activity while minimizing toxicities. The inventors have surprisingly found that the immunoconjugate of the present invention induces marked anti-tumour activity while minimizing toxicities such as dermatological toxicities. This is even more surprising because the immunoconjugate of the invention comprises an anti-EGFR and MUC1 bispecific antibody in which the EGFR binding portion is not attenuated, i.e., the binding affinity to human EGFR is not attenuated.

In some embodiments, the immunoconjugate of the invention comprises:
(a) a bispecific antibody that binds to EGFR and MUC1,
(b) a growth inhibiting agent, and
(c) a linker covalently linking (a) and (b).

In some embodiments, the bispecific antibody comprises
- an EGFR binding portion comprising
   o a heavy chain variable region (VH) comprising
      a heavy chain complementary determining region (HCDR) 1 of SEQ ID NO: 1,
      a HCDR 2 of SEQ ID NO: 2, and
      a HCDR 3 of SEQ ID NO: 3, and
   ∘ a light chain variable region (VL) comprising
      a light chain complementarity determining region (LCDR) 1 of SEQ ID NO: 4,
      a LCDR 2 of SEQ ID NO: 5, and
      a LCDR 3 of SEQ ID NO: 6, and
- a MUC1 binding portion comprising
   o a heavy chain variable region (VH) comprising
      a heavy chain complementary determining region (HCDR) 1 of SEQ ID NO: 7,
      a HCDR 2 of SEQ ID NO: 8, and
      a HCDR 3 of SEQ ID NO: 9, and
   o a light chain variable region (VL) comprising
      a light chain complementarity determining region (LCDR) 1 of SEQ ID NO: 10,
      a LCDR 2 of SEQ ID NO: 11, and
      a LCDR 3 of SEQ ID NO: 12.

The immunoconjugate of claim 1 or 2, wherein the bispecific antibody comprises
- an EGFR binding portion comprising
   o a heavy chain variable region (VH) comprising
      an amino acid sequence that is at least 95%, 96%, 97%, 98%, 99% or 100% identical to the amino acid sequence of SEQ ID NO: 13, and
   ∘ alight chain variable region (VL) comprising
      an amino acid sequence that is at least 95%, 96%, 97%, 98%, 99% or 100% identical to the amino acid sequence of SEQ ID NO: 14,
- a MUC1 binding portion comprising
   o a heavy chain variable region (VH) comprising
      an amino acid sequence that is at least 95%, 96%, 97%, 98%, 99% or 100% identical to the amino acid sequence of SEQ ID NO: 15, and
   ∘ a light chain variable region (VL) comprising
      an amino acid sequence that is at least 95%, 96%, 97%, 98%, 99% or 100% identical to the amino acid sequence of SEQ ID NO: 16.

In some embodiments, the bispecific antibody binds to EGFR such as human EGFR with a binding affinity (K_{D}), preferably wherein the K_{D} is less than 10 nM such as less than 5 nM.

In some embodiments, the bispecific antibody comprises:
(i) a first polypeptide comprising a first Fc domain and a single-chain Fv fragment (scFv), wherein the scFv comprises the VH and VL of the MUC1 binding portion,
(ii) a second polypeptide comprising a second Fc domain and a heavy chain of a Fab fragment comprising the VH of the EGFR binding portion, and
(iii) a third polypeptide comprising a light chain of the Fab fragment comprising the VL of the EGFR binding portion.

In some embodiments,
- the first polypeptide comprises a T366W substitution, and the second polypeptide comprises at least one of T366S, L368A, and Y407V substitutions, or
- the second polypeptide comprises a T366W substitution, and the first polypeptide comprises at least one of T366S, L368A, and Y407V substitutions,
   and/or
- the first polypeptide comprises a S354C substitution, and the second polypeptide comprises a Y349C substitution, or
- the second polypeptide comprises a S354C substitution, and the first polypeptide comprises a Y349C substitution,
wherein EU numbering is used for said amino acid substitutions.

In some embodiments, the first and/or the second polypeptide comprises
- a C226S substitution,
- a L234A and L235A (LALA) substitution, and/or
- a D356E and L358M substitution,
wherein EU numbering is used for said amino acid substitutions.

In some embodiments,
- the first polypeptide comprises
   an amino acid sequence that is at least 90%, 95%, 96%, 97%, 98%, 99% or 100% identical to the amino acid sequence of SEQ ID NO: 17,
- the second polypeptide comprises
   an amino acid sequence that is at least 90%, 95%, 96%, 97%, 98%, 99% or 100% identical to the amino acid sequence of SEQ ID NO: 18, and
- the third polypeptide comprises
an amino acid sequence that is at least 90%, 95%, 96%, 97%, 98%, 99% or 100% identical to the amino acid sequence of SEQ ID NO: 19.

In some embodiments, the growth inhibitory agent is a topoisomerase I inhibitor such as exatecan.

In some embodiments, the linker is a cleavable linker, preferably a β-glucuronide linker.

In some embodiments, the linker and the growth inhibiting agent in combination represents a drug-linker compound, preferably wherein the drug-linker compound is conjugated to the bispecific antibody at an average drug-to-antibody ratio (DAR) from 2 to 8 or about 4 such as 3.5 to 4.5 or 3.8 to 4.2.

In some embodiments, the immunoconjugate comprises
(a) a bispecific antibody that binds to EGFR and MUC1 comprising an EGFR binding portion and a MUC1 binding portion,
(b) a growth inhibiting agent, and
(c) a linker covalently linking (a) and (b),

wherein the bispecific antibody comprises
   - a first polypeptide comprising a first Fc domain and a single-chain Fv fragment (scFv), wherein the scFv comprises a VH and a VL of the MUC1 binding portion,
   - a second polypeptide comprising a second Fc domain and a heavy chain of a Fab fragment comprising a VH of the EGFR binding portion, and
   - a third polypeptide comprising a light chain of the Fab fragment comprising a VL of the EGFR binding portion,
wherein the VH of the EGFR binding portion comprises an amino acid sequence that is at least 95% identical to the amino acid sequence of SEQ ID NO: 13, and comprises
   ∘ HCDR 1 of SEQ ID NO: 1,
   ∘ HCDR2 of SEQ ID NO: 2, and
   ∘ HCDR 3 of SEQ ID NO: 3, and
the VL of the EGFR binding portion comprises an amino acid sequence that is at least 95% identical to the amino acid sequence of SEQ ID NO: 14, and comprises
   ∘ LCDR 1 of SEQ ID NO: 4,
   ∘ LCDR2 of SEQ ID NO: 5, and
   ∘ LCDR 3 of SEQ ID NO: 6, and
wherein the VH of the MUC1 binding portion comprises an amino acid sequence that is at least 95% identical to the amino acid sequence of SEQ ID NO: 15, and comprises
   ∘ HCDR 1 of SEQ ID NO: 7,
   ∘ HCDR2 of SEQ ID NO: 8, and
   ∘ HCDR 3 of SEQ ID NO: 9, and
wherein the VL of the EGFR binding portion comprises an amino acid sequence that is at least 95% identical to the amino acid sequence of SEQ ID NO: 16, and comprises
   ∘ LCDR 1 of SEQ ID NO: 10,
   ∘ LCDR2 of SEQ ID NO: 11, and
   ∘ LCDR 3 of SEQ ID NO: 12, and
wherein the first Fc domain of the first polypeptide comprises at least one knob substitution, and the Fc domain of the second polypeptide comprises at least one hole substitution, or the second Fc domain of the second polypeptide comprises at least one knob substitution, and the Fc domain of the first polypeptide comprises at least one hole substitution,
wherein the growth inhibiting agent is exatecan, and the linker is a β-glucuronide linker comprising a maleimide moiety, wherein the immunoconjugate has the following formula **(II):** 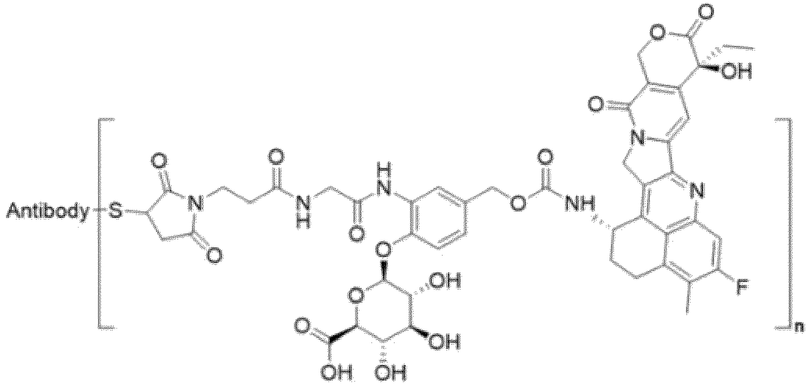
wherein "n" represents an average drug-to-antibody ratio (DAR), wherein the drug-linker compound is conjugated to the antibody at a DAR of below 8 or about 4 such as 3.5 to 4.5 or 3.8 to 4.2,
wherein the "S" is a sulfur atom of a cysteine of the bispecific antibody, wherein the cysteine of the bispecific antibody is one of the cysteines capable of forming an interchain disulfide bond.

In some embodiments, a pharmaceutical composition comprising the immunoconjugate and a pharmaceutically acceptable excipient is provided.

In some embodiments, the immunoconjugate or the pharmaceutical composition is for use in the treatment of cancer in a subject, wherein the subject is administered the pharmaceutical composition or the immunoconjugate, wherein the subject is human, preferably wherein the cancer is a MUC1 and/or EGFR expressing cancer.

In some embodiments, the immunoconjugate or the pharmaceutical composition is for use in the treatment of cancer in a subject, wherein the subject is administered at least 1 mg/kg of the immunoconjugate or from 1 mg/kg to 10mg/kg of the immunoconjugate, preferably qw, q2w, or q3w.

### Definitions

Terms are used herein as generally used in the art, unless otherwise defined in the following.

As used herein, the term "antigen binding molecule" refers in its broadest sense to a molecule that specifically binds an antigenic determinant. Examples of antigen binding molecules are immunoglobulins and derivatives, e.g. fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigenic determinant" is synonymous with "antigen" and "epitope" and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein can be any native form of the proteins from any vertebrate source, including mammals such as primates (e.g. humans), non-human primates (e.g. cynomolgus monkeys) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. In certain embodiments the antibody or bispecific antigen binding molecule in the immunoconjugate of the invention binds to an epitope of EGFR and/or MUC1. In particular embodiments, the antibody in the immunoconjugate of the invention binds to human EGFR and/or MUC1.

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed e.g. on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (KD) of ≤ 1 mM, ≤ 100 nM, ≤ 10 nM, ≤ 1 nM, ≤ 0.1 nM, ≤ 0.01 nM, or ≤ 0.001 nM (e.g. 10-8 M or less, e.g. from 10-8 M to 10-13 M, e.g., from 10-9 M to 10-13 M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (KD), which is the ratio of dissociation and association rate constants (koff and kon, respectively). Thus, equivalent affinities may comprise different rate constants, as long as the ratio of the rate constants remains the same. Affinity can be measured by well-established methods known in the art, including those described herein. A particular method for measuring affinity is Surface Plasmon Resonance (SPR) or biolayer interferometry (BLI).

"Reduced binding", for example reduced binding to an Fc receptor, refers to a decrease in affinity for the respective interaction, as measured for example by SPR. For clarity, the term includes also reduction of the affinity to zero (or below the detection limit of the analytic method), i.e. complete abolishment of the interaction. Conversely, "increased binding" refers to an increase in binding affinity for the respective interaction.

The term "EGFR," as used herein, refers to human EGFR e.g., as disclosed in SEQ ID NO: 23.

The term "MUC1" as used herein, refers to human MUC1 e.g., as disclosed in SEQ ID NO: 24.

The terms "anti-EGFR and MUC1 bispecific antibody" and "a bispecific antibody that binds to EGFR and MUC1" refer to a bispecific antibody that is capable of binding EGFR and MUC1 with sufficient affinity such that the bispecific antibody is useful as a diagnostic and/or therapeutic agent in targeting EGFR and MUC1. In one embodiment, the extent of binding of an anti-EGFR and MUC1 bispecific antibody to an unrelated, non-EGFR or non-MUC1 protein is less than about 10% of the binding of the bispecific antibody to EGFR and/or MUC1 as measured, e.g., by a radioimmunoassay (RIA) or by scatchard analysis or by surface plasmon resonance, such as, for example, BIAcoreTM. A particular method for measuring the extent of binding or "affinity" is Surface Plasmon Resonance (SPR) or biolayer interferometry (BLI). In certain embodiments, an antibody that binds to EGFR and MUC1 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g., from 10⁻⁸ M to 10⁻¹³ M, e.g., from 10⁻⁹ M to 10⁻¹³ M). In certain embodiments, an anti-EGFR and MUC1 bispecific antibody binds to an epitope of EGFR and/or MUC1 that is conserved among different species. In preferred embodiments, "anti-EGFR and MUC1 bispecific antibody" and "a bispecific antibody that binds to EGFR and MUC1" refer to an antibody that binds to human EGFR and human MUC1.

The term "antibody drug conjugate" (ADC) as used herein is equivalent to the term "immunoconjugate".

A "target cell antigen" as used herein refers to an antigenic determinant presented on the surface of a target cell, for example a cell in a tumor such as a cancer cell or a cell of the tumor stroma. In accordance with the invention, the target cell antigen is EGFR and/or MUC1, particularly human EGFR and/or MUC1.

By "fused" is meant that the components (e.g. a Fab molecule and an Fc domain subunit) are linked by peptide bonds, either directly or via one or more peptide linkers.

A "Fab molecule" refers to a protein consisting of the VH and CH1 domain of the heavy chain (the "Fab heavy chain") and the VL and CL domain of the light chain (the "Fab light chain") of an immunoglobulin.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable domain (VH), also called a variable heavy domain or a heavy chain variable region, followed by three constant domains (CH1, CH2, and CH3), also called a heavy chain constant region. Similarly, from N- to C-terminus, each light chain has a variable domain (VL), also called a variable light domain or a light chain variable region, followed by a constant light (CL) domain, also called a light chain constant region. The heavy chain of an immunoglobulin may be assigned to one of five types, called α (IgA), δ (IgD), ε (IgE), γ (IgG), or m (IgM), some of which may be further divided into subtypes, e.g. γ1 (IgG1), γ2 (IgG2), γ3 (IgG3), γ4 (IgG4), α1 (IgAi) and α2 (IgA2). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antibody" herein is used in the broadest sense and encompasses various antibody structures, including but not limited to monoclonal antibodies, polyclonal antibodies, multispecific antibodies (e.g. bispecific antibodies), and antibody fragments so long as they exhibit the desired antigen-binding activity.

The term "monoclonal antibody" as used herein refers to an antibody obtained from a population of substantially homogeneous antibodies, i.e. the individual antibodies comprised in the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The terms "full length antibody," "intact antibody," and "whole antibody" are used herein interchangeably to refer to an antibody having a structure substantially similar to a native antibody structure.

A "humanized" antibody refers to a chimeric antibody comprising amino acid residues from non-human HVRs and amino acid residues from human FRs. In certain embodiments, a humanized antibody will comprise at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. A single-domain antibody may be a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable domain (VL) and an antibody heavy chain variable domain (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity. As used herein in connection with variable region sequences, "Kabat numbering" refers to the numbering system set forth by Kabat et al., Sequences of Proteins of Immunological Interest, 5th Ed. Public Health Service, National Institutes of Health, Bethesda, MD (1991).

As used herein, the amino acid positions of all constant regions and domains of the heavy and light chain are numbered according to is according to the EU numbering system, also called the EU index of Kabat, as described in Kabat, E.A. et al., Sequences of Proteins of Immunological Interest, 5th ed., Public Health Service, National Institutes of Health, Bethesda, MD (1991), NIH Publication 91-3242.

The term "hypervariable region" or "HVR", as used herein, refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs; three in the VH (H1, H2, H3), and three in the VL (L1, L2, L3).

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H 1(L1)-FR2-H2(L2)-FR3-H3(L3)-FR4.

A "humanized" antibody refers to a chimeric antibody comprising amino acid residues from non-human HVRs and amino acid residues from human FRs. In certain embodiments, a humanized antibody will comprise at least one, and typically two, variable domains, in which all or substantially all of the HVRs (e.g., CDRs) correspond to those of a non-human antibody, and all or substantially all of the FRs correspond to those of a human antibody. Such variable domains are referred to herein as "humanized variable region". A humanized antibody optionally may comprise at least a portion of an antibody constant region derived from a human antibody. In some embodiments, some FR residues in a humanized antibody are substituted with corresponding residues from a non-human antibody (e.g., the antibody from which the HVR residues are derived), e.g., to restore or improve antibody specificity or affinity. A "humanized form" of an antibody, e.g. of a non-human antibody, refers to an antibody that has undergone humanization. Other forms of "humanized antibodies" according to the present disclosure are those in which the constant region has been additionally modified or changed from that of the original antibody to generate the properties according to the disclosure, especially in regard to C1q binding and/or Fc receptor (FcR) binding.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. A human antibody may be derived from a non-human transgenic mammal, for example a mouse, a rat, or a rabbit. A human antibody may also be derived from a hybridoma cell line. Antibodies or antibody fragments isolated from human antibody libraries are also considered human antibodies or human antibody fragments herein

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and m, respectively.

The terms "constant region derived from human origin" or "human constant region" as used in the current specification denotes a constant heavy chain region of a human antibody of the subclass IgG1, IgG2, IgG3, or IgG4 and/or a constant light chain kappa or lambda region. Such constant regions are well known in the state of the art and e.g., described by Kabat, E.A., et al., Sequences of Proteins of Immunological Interest, 5th ed., Public Health Service, National Institutes of Health, Bethesda, MD (1991) (see also, e.g., Johnson, G., and Wu, T.T., Nucleic Acids Res. 28 (2000) 214-218; Kabat, E.A., et al., Proc. Natl. Acad. Sci. USA 72 (1975) 2785-2788). Unless otherwise specified herein, numbering of amino acid residues in the constant region is according to the EU numbering system, also called the EU index of Kabat, as described in Kabat, E.A. et al., Sequences of Proteins of Immunological Interest, 5th ed., Public Health Service, National Institutes of Health, Bethesda, MD (1991), NIH Publication 91-3242.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain. When stating the mutations in the Fc region, the mutation is meant to be present in both subunits unless explicitly specified.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g., B cell receptor); and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G329, P329G, or Pro329Gly.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Thus, a sequence "at least 85% identical" to a reference sequence is a sequence having, over its entire length, 85% or more, for instance 90%, 91 %, 92%, 93%, 94%, 95%, 96%, 97%, 98% or 99% sequence identity with the entire length of the reference sequence. The percentage of "sequence identity" may thus be determined by comparing two such sequences over their entire length by global pairwise alignment using the algorithm of Needleman and Wunsch (J. Mol. Biol. 48:443 (1970)), e.g. using the program Needle (EMBOSS) with the BLOSUM62 matrix and the following parameters: gap open=10, gap extend=0.5, end gap penalty=false, end gap open=10, end gap extend=0.5 (which are standard settings).

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

The term "vector" or "expression vector" refers to a DNA molecule that is used to introduce and direct the expression of a specific gene to which it is operably associated in a cell. The term includes the vector as a self-replicating nucleic acid structure as well as the vector incorporated into the genome of a host cell into which it has been introduced. The expression vector of the present disclosure comprises an expression cassette. Expression vectors allow transcription of large amounts of stable mRNA. Once the expression vector is inside the cell, the ribonucleic acid molecule or protein that is encoded by the gene is produced by the cellular transcription and/or translation machinery. The expression vector of the present disclosure may comprise an expression cassette that comprises polynucleotide sequences that encode antibodies or bispecific antigen binding molecules of the invention or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the antibodies or bispecific antigen binding molecules of the present invention. Host cells include cultured cells, e.g. mammalian cultured cells, such as HEK cells, CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include a cancer that over-expresses EGFR and/or MUC1, which may include, for example, breast cancer and/or metastatic breast cancer, including Her2 negative breast cancers and/or triple negative breast cancers, pancreatic cancer, colon cancer, colorectal cancer, melanoma, ovarian cancer, non-small cell lung cancer (either squamous and/or non-squamous), gastric cancer, squamous cell cancer, small-cell lung cancer, adenocarcinoma of the lung, squamous carcinoma of the lung, cancer of the peritoneum, hepatocellular cancer, gastrointestinal cancer, glioma, cervical cancer, liver cancer, bladder cancer, hepatoma, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "EGFR-positive cancer" or "EGFR-expressing cancer" are used synonymously and refer to a cancer comprising cells that express EGFR on their surface. For the purposes of determining whether a cell expresses EGFR on the surface, EGFR mRNA expression is considered to correlate to EGFR expression on the cell surface. In some embodiments, expression of EGFR mRNA is determined by a method selected from in situ hybridization and RT-PCR (including quantitative RT-PCR). Alternatively, expression of EGFR on the cell surface can be determined, for example, using antibodies to EGFR in a method such as immunohistochemistry, FACS, etc. In some embodiments, a EGFR-positive cancer means a breast cancer, metastatic breast cancer, including Her2 negative breast cancers and/or triple negative breast cancers, pancreatic cancer, colon cancer, colorectal cancer, melanoma, ovarian cancer, non-small cell lung cancer (either squamous and/or non-squamous), or gastric cancer, each of which exhibits a high level of EGFR expression.

The term "MUC1-positive cancer" or "MUC1-expressing cancer" are used synonymously and refer to a cancer comprising cells that express MUC1 on their surface. For the purposes of determining whether a cell expresses MUC1 on the surface, MUC1 mRNA expression is considered to correlate to MUC1 expression on the cell surface. In some embodiments, expression of MUC1 mRNA is determined by a method selected from in situ hybridization and RT-PCR (including quantitative RT-PCR). Alternatively, expression of MUC1 on the cell surface can be determined, for example, using antibodies to MUC1 in a method such as immunohistochemistry, FACS, etc. In some embodiments, a MUC1-positive cancer means a breast cancer, metastatic breast cancer, including Her2 negative breast cancers and/or triple negative breast cancers, pancreatic cancer, colon cancer, colorectal cancer, melanoma, ovarian cancer, non-small cell lung cancer (either squamous and/or non-squamous), or gastric cancer, each of which exhibits a high level of MUC1 expression.

The term "pharmaceutical composition" refers to a preparation which is in such form as to permit the biological activity of an active ingredient contained therein to be effective, and which contains no additional components which are unacceptably toxic to a subject to which the composition would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies or bispecific antigen binding molecules of the invention are used to delay development of a disease or to slow the progression of a disease.

The term "package insert" is used to refer to instructions customarily included in commercial packages of therapeutic products, that contain information about the indications, usage, dosage, administration, combination therapy, contraindications and/or warnings concerning the use of such therapeutic products.

The term "growth inhibitory agent" (also referred to as an "anti-proliferative agent") refers to a molecule or compound or composition which inhibits growth of a cell, such as a tumor cell, in vitro and/or in vivo.

The term "cytotoxic drug" as used herein refers to a substance that directly or indirectly inhibits or prevents the function of cells and/or causes destruction of the cells. The term "cytotoxic drug" includes e.g. chemotherapeutic agents, enzymes, antibiotics, toxins such as small molecule toxins or enzymatically active toxins, toxoids, vincas, taxanes, maytansinoids or maytansinoid analogs, tomaymycin or pyrrolobenzodiazepine derivatives, cryptophycin derivatives, leptomycin derivatives, auristatin or dolastatin analogs, prodrugs, topoisomerase I inhibitors, topoisomerase II inhibitors, DNA alkylating agents, anti-tubulin agents, CC-1065 and CC-1065 analogs.

Topoisomerase I inhibitors are molecules or compounds that inhibit the human enzyme topoisomerase I which is involved in altering the topology of DNA by catalyzing the transient breaking and rejoining of a single strand of DNA. Topoisomerase I inhibitors are highly toxic to dividing cells e.g. of a mammal.

The term "radioactive moiety" refers to a chemical entity (such as a molecule, compound or composition) that comprises or consists of a radioactive isotope suitable for treating cancer, such as At²¹¹, Bi²¹², Er¹⁶⁹, I¹³¹, I¹²⁵, Y⁹⁰, In¹¹¹, P³², Re¹⁸⁶, Re¹⁸⁸, Sm¹⁵³, Sr⁸⁹, or radioactive isotopes of Lu. Such radioisotopes generally emit mainly beta-radiation. In some embodiments, the radioactive isotope is an alpha-emitter isotope, for example Thorium 227 which emits alpha-radiation. Immunoconjugates can be prepared e.g. as described in the application WO2004/091668.

"Linker", as used herein, means a chemical moiety comprising a covalent bond and/or any chain of atoms that covalently attaches the growth inhibitory agent to the antibody. Linkers are well known in the art and include e.g. disulfide groups, thioether groups, acid labile groups, photolabile groups, peptidase labile groups and esterase labile groups. Conjugation of an antibody of the invention with cytotoxic drugs or other growth inhibitory agents may be performed e.g. using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1 - pyrrolidinyl ester (nitro-SPDB), 4-(Pyridin-2-yldisulfanyl)-2-sulfo-butyric acid (sulfo-SPDB), N- succinimidyl (2-pyridyldithio) propionate (SPDP), succinimidyl (N-maleimidomethyl) cyclohexane-1 -carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1 ,5-difluoro-2,4-dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to an antibody (WO 94/11026).

In embodiments of the present invention, the linker may be a "cleavable linker", which may facilitate release of the cytotoxic drug or other growth inhibitory agent inside of or in the vicinity of a cell, e.g. a tumor cell.

The number n is also referred to as "drug-to-antibody ratio" (or "DAR"); this number n is always to be understood as an average number for any given (preparation of an) immunoconjugate.

### Brief Description of the Figures

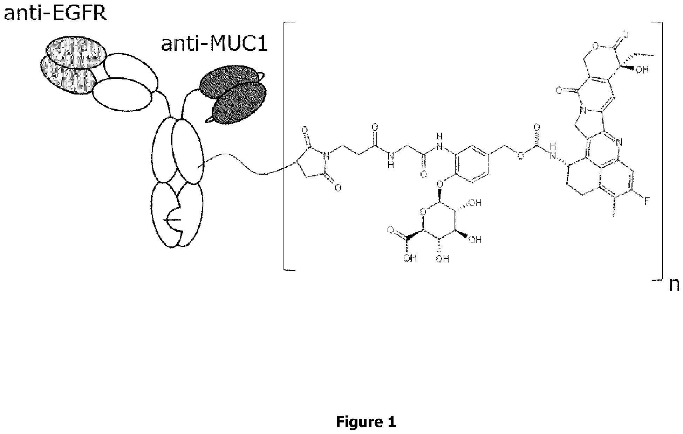

**Figure 1****:** A pictorial representation of the antibody of the invention conjugated to a drug linker compound. The drug linker compound comprises a β-glucuronide linker, and the growth inhibiting agent, exatecan resulting in a "β-glucuronide-exatecan", "drug linker compound" represented by the **formula (I).**
**Figure 2****:** Scheme of EGFR/MUC-MAB and strategy to conjugate drug-linker compound formula (I) resulting in EGFR/MUC-ADC3.
**Figure 3****:** Bispecific platform knob-into-hole in comparison to SEEDs.
**Figure 4****:** Bispecific platform knob-into-hole in comparison to SEEDs - showing design summary of EGFR/MUC-MAB1, EGFR/MUC-MAB2, EGFR/MUC-MAB3, EGFR/MUC-MAB4, EGFR/MUC-MAB5 and EGFR/MUC-MAB6.
**Figure 5****:** LC-MS analysis of reduced EGFR/MUC-ADC4 and EGFR/MUC-ADC5. +1, +2 refers to the number of attached to drug-linker compound formula (I).
**Figure 6****:** SDS PAGE analysis of EGFR/MUC-ADC4 and EGFR/MUC-ADC5.
**Figure 7****:** Selective conjugation of the interchain disulfide cysteines while preserving the integrity of the engineered disulfide.
**Figure 8A** **and** **8B****:** Optimization of reduction conditions for the conjugation of EGFR/MUC-MAB5 to achieve complete conjugation while minimizing the reduction of the engineered KIH 2nd disulfide.
**Figure 9****:** LC-MS profile (non-reduced) of exemplary EGFR/MUC-ADC6 batch confirming covalent linkage of heavy chains. Overall DAR calculated to 3.9.
**Figure 10****:** PK profiles of EGFR/MUC-ADC1 and EGFR-MUC-ADC6 in transgenic humanized FcRn TG32 mice.
**Figure 11****:** Schemes of comparative molecules of different valencies.
**Figure 12****:** Binding of fluorescently labelled molecules against different cell lines **(A)** MDA-MB-468 **(B)** HCC827 **(C)** OVCAR-3. Bispecific EGFR/MUC-fluo6 shows higher maximum signals compared to monospecific mono-and bivalents binders at lower concentration ranges.
**Figure 13****:** Efficacy in SCCHN PDX - HNX005 of EGFR/MUC-ADC6, and monoEGFR-ADC1.
**Figure 14****:** Efficacy in NSCLC PDX - LUX003 of EGFR/MUC-ADC6 and monoEGFR-ADC1.
**Figure 15****:** Efficacy in CDX - OVCAR3 of EGFR/MUC-ADC6 and monovEGFR-ADC1.
**Figure 16****:** Efficacy in SCCHN PDX - HNX005 of **(A)** EGFR/MUC-ADC6 and bivEGFR-ADC1 and **(B)** EGFR/MUC-ADC6, bivEGFR-ADC1, monoEGFR-ADC1.
**Figure 17****:** PK profiles of EGFR/cMET-ADC and EGFR/HER3-ADC compared to EGFR/MUC-ADC6 in transgenic humanized FcRn TG32 mice.
**Figure 18****:** Efficacy in CDX - OVCAR3 of EGFR/MUC-ADC6 and EGFR-ADC-MMAE.
**Figure 19****:** Efficacy in NSCLC PDX - LUX003 of EGFR/MUC-ADC6 and EGFR-ADC-MMAE.
**Figure 20** Efficacy in SCCHN PDX **(A)** ST3718 - of EGFR/MUC-ADC6 and EGFR-ADC-MMAE **(B)** ST497B - of EGFR/MUC-ADC6 and EGFR-ADC-MMAE **(C)** ST2652 - of EGFR/MUC-ADC6 and EGFR-ADC-MMAE **Figure 21****:** In vitro potency of EGFR/MUC-ADC6 and EGFR/HER3 ADC (DAR8, TOP1i payload).
**Figure 22****:** Efficacy in NSCLC PDX - OD29498 of EGFR/MUC-ADC6 and EGFR/HER3 ADC.

### Brief Description of Sequences

### Amino acid sequences

**SEQ ID NO: 1** - an EGFR HCDR1 of the bispecific anti-EGFRxMUC1 antibody
   GFSLTNYG
**SEQ ID NO: 2** - an EGFR HCDR2 of the bispecific anti-EGFRxMUC1 antibody
   IWSGGNT
**SEQ ID NO: 3** - an EGFR HCDR3 of the bispecific anti-EGFRxMUC1 antibody
   ARALTYYDYEFAY
**SEQ ID NO: 4** - an EGFR LCDR1 of the bispecific anti-EGFRxMUC1 antibody
   QSIGTN
**SEQ ID NO: 5** - an EGFR LCDR2 of the bispecific anti-EGFRxMUC1 antibody
   YAS
**SEQ ID NO: 6** - an EGFR LCDR3 of the bispecific anti-EGFRxMUC1 antibody
   QQNNNWPTT
**SEQ ID NO: 7 -** a MUC1 HCDR1 of the bispecific anti-EGFRxMUC1 antibody
   GYSFTSHF
**SEQ ID NO: 8 -** a MUC1 HCDR2 of the bispecific anti-EGFRxMUC1 antibody
   IDPVTGGT
**SEQ ID NO: 9 -** a MUC1 HCDR3 of the bispecific anti-EGFRxMUC1 antibody
   AREARADRGQFDK
**SEQ ID NO: 10** - a MUC1 LCDR 1 of the bispecific anti-EGFRxMUC1 antibody
   NIGSKS
**SEQ ID NO: 11** - a MUC1 LCDR 2 of the bispecific anti-EGFRxMUC1 antibody
   YGS
**SEQ ID NO: 12** - a MUC1 LCDR 3 of the bispecific anti-EGFRxMUC1 antibody
   QVWDSSSDWV
**SEQ ID NO: 13** - an EGFR heavy chain variable region (VH) 
EVQLVQSGAEVKKPGASVKVSCKASGFSLTNYGVHWMRQAPGQGLEWIGVIWSGGNTDYNTPFTSRVTITSD
KSTSTAYMELSSLRSEDTAVYYCARALTYYDYEFAYWGQGTLVTVSS
**SEQ ID NO: 14** - an EGFR light chain variable region (VL) 
DIQMTQSPSSLSASVGDRVTITCRASQSIGTNIHWYQQKPGKAPKLLIKYASESISGVPSRFSGSGYGTDFTLTIS
SLQPEDVATYYCQQNNNWPTTFGQGTKVEIK
**SEQ ID NO: 15** - a MUC1 heavy chain variable region (VH) 
QMQLVQSEAELKKPGASVKVSCKASGYSFTSHFMHWVRQAPGQGLEWMGWIDPVTGGTKYAQNFQGWVT
MTRDTSIRTAYLELSRLRSDDTAMYYCAREARADRGQFDKWGQGTLVTVAS
**SEQ ID NO: 16** - a MUC1 light chain variable region (VL) 
QSVLTQPPSVSVAPGKTARITCGGNNIGSKSVHWYQQKPGQAPALVIYYGSNRPSGIPERFSGSNSGNTATLTIS
RVEAGDEADYYCQVWDSSSDWVFGGGTKLTVL
**SEQ ID NO: 17** - first polypeptide comprising a first Fc domain and a single-chain Fv fragment (scFv) that binds to MUC1 
QMQLVQSEAELKKPGASVKVSCKASGYSFTSHFMHWVRQAPGQGLEWMGWIDPVTGGTKYAQNFQGWVT
MTRDTSIRTAYLELSRLRSDDTAMYYCAREARADRGQFDKWGQGTLVTVASGGGGSGGGGSGGGGSQSVLT
QPPSVSVAPGKTARITCGGNNIGSKSVHWYQQKPGQAPALVIYYGSNRPSGIPERFSGSNSGNTATLTISRVEAG
DEADYYCQVWDSSSDWVFGGGTKLTVLEPKSSDKTHTSPPCPAPEAAGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKALPAPIEKTIS
KAKGQPREPQVYTLPPCREEMTKNQVSLWCLVKGFYPSDIAVEWESNGQPENNYKTTPPVLDSDGSFFLYSKLTV
DKSRWQQGNVFSCSVMHEALHNHYTQKSLSLSPGK
**SEQ ID NO: 18** - second polypeptide comprising a second Fc domain and a heavy chain of a Fab fragment comprising the EGFR VH 
EVQLVQSGAEVKKPGASVKVSCKASGFSLTNYGVHWMRQAPGQGLEWIGVIWSGGNTDYNTPFTSRVTITSD
KSTSTAYMELSSLRSEDTAVYYCARALTYYDYEFAYWGQGTLVTVSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEPKSC
DKTHTSPPCPAPEAAGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVCTLPPSREEMTKNQVSLSCAVKG
FYPSDIAVEWESNGQPENNYKTTPPVLDSDGSFFLVSKLTVDKSRWQQGNVFSCSVMHEALHNHYTQKSLSLSP
GK
**SEQ ID NO: 19** - third polypeptide comprising a light chain of the Fab fragment comprising the EGFR VL 
DIQMTQSPSSLSASVGDRVTITCRASQSIGTNIHWYQQKPGKAPKLLIKYASESISGVPSRFSGSGYGTDFTLTIS
SLQPEDVATYYCQQNNNWPTTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC
**SEQ ID NO: 20** - Human kappa CL domain
**SEQ ID NO: 21** - Human lambda CL domain 
QPKAAPSVTLFPPSSEELQANKATLVCLISDFYPGAVTVAWKADSSPVKAGVETTTPSKQSNNKYAASSYLSLTPE
QWKSHRSYSCQVTHEGSTVEKTVAPTECS
**SEQ ID NO: 22** - Human IgG1 heavy chain constant region (CH1-CH2-CH3) 
ASTKGPSVFPLAPSSKSTSGGTAALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSP
**SEQ ID NO: 23 -** Human EGFR
**SEQ ID NO: 24** - Human MUC1 
MTPGTQSPFFLLLLLTVLTVVTGSGHASSTPGGEKETSATQRSSVPSSTEKNAVSMTSSVLSSHSPGSGSSTTQGQ
DVTLAPATEPASGSAATWGQDVTSVPVTRPALGSTTPPAHDVTSAPDNKPAPGSTAPPAHGVTSAPDTRPAPGST
 
APPAHGVTSAPDTRPAPGSTAPPAHGVTSAPDTRPAPGSTAPPAHGVTSAPDTRPAPGSTAPPAHGVTSAPDTRP
APGSTAPPAHGVTSAPDTRPAPGSTAPPAHGVTSAPDTRPAPGSTAPPAHGVTSAPDTRPAPGSTAPPAHGVTSA
PDTRPAPGSTAPPAHGVTSAPDTRPAPGSTAPPAHGVTSAPDTRPAPGSTAPPAHGVTSAPDTRPAPGSTAPPAH
GVTSAPDTRPAPGSTAPPAHGVTSAPDTRPAPGSTAPPAHGVTSAPDTRPAPGSTAPPAHGVTSAPDTRPAPGST
APPAHGVTSAPDTRPAPGSTAPPAHGVTSAPDTRPAPGSTAPPAHGVTSAPDTRPAPGSTAPPAHGVTSAPDTRP
APGSTAPPAHGVTSAPDTRPAPGSTAPPAHGVTSAPDTRPAPGSTAPPAHGVTSAPDTRPAPGSTAPPAHGVTSA
PDTRPAPGSTAPPAHGVTSAPDTRPAPGSTAPPAHGVTSAPDTRPAPGSTAPPAHGVTSAPDTRPAPGSTAPPAH
GVTSAPDTRPAPGSTAPPAHGVTSAPDTRPAPGSTAPPAHGVTSAPDTRPAPGSTAPPAHGVTSAPDTRPAPGST
APPAHGVTSAPDTRPAPGSTAPPAHGVTSAPDTRPAPGSTAPPAHGVTSAPDTRPAPGSTAPPAHGVTSAPDTRP
APGSTAPPAHGVTSAPDTRPAPGSTAPPAHGVTSAPDTRPAPGSTAPPAHGVTSAPDTRPAPGSTAPPAHGVTSA
PDTRPAPGSTAPPAHGVTSAPDTRPAPGSTAPPAHGVTSAPDNRPALGSTAPPVHNVTSASGSASGSASTLVHNG
TSARATTTPASKSTPFSIPSHHSDTPTTLASHSTKTDASSTHHSSVPPLTSSNHSTSPQLSTGVSFFFLSFHISNLQF
NSSLEDPSTDYYQELQRDISEMFLQIYKQGGFLGLSNIKFRPGSVVVQLTLAFREGTINVHDVETQFNQYKTEAAS
RYNLTISDVSVSDVPFPFSAQSGAGVPGWGIALLVLVCVLVALAIVYLIALAVCQCRRKNYGQLDIFPARDTYHPMS
EYPTYHTHGRYVPPSSTDRSPYEKVSAGNGGSSLSYTNPAVAATSANL
**SEQ ID NO: 25** - Transglutaminase 
MGSGSGSGTGEEKRSYAETHRLTADDVDDINALNESAPAASSAGPSFRAPDSDERVTPPAEPLDRMPDPYRPSYG
RAETIVNNYIRKWQQVYSHRDGRKQQMTEEQREWLSYGCVGVTWVNSGQYPTNRLAFAFFDEDKYKNELKNGR
PRSGETRAEFEGRVAKDSFDEAKGFQRARDVASVMNKALENAHDEGAYLDNLKKELANGNDALRNEDARSPFYS
ALRNTPSFKDRNGGNHDPSKMKAVIYSKHFWSGQDRSGSSDKRKYGDPEAFRPDRGTGLVDMSRDRNIPRSPTS
PGESFVNFDYGWFGAQTEADADKTVWTHGNHYHAPNGSLGAMHVYESKFRNWSDGYSDFDRGAYVVTFVPKS
WNTAPDKVTQGWP
**SEQ ID NO: 26** - Transglutaminase (activated, version1) 
FRAPDSDERVTPPAEPLDRMPDPYRPSYGRAETIVNNYIRKWQQVYSHRDGRKQQMTEEQREWLSYGCVGVTW
VNSGQYPTNRLAFAFFDEDKYKNELKNGRPRSGETRAEFEGRVAKDSFDEAKGFQRARDVASVMNKALENAHDE
GAYLDNLKKELANGNDALRNEDARSPFYSALRNTPSFKDRNGGNHDPSKMKAVIYSKHFWSGQDRSGSSDKRKY
GDPEAFRPDRGTGLVDMSRDRNIPRSPTSPGESFVNFDYGWFGAQTEADADKTVWTHGNHYHAPNGSLGAMH
VYESKFRNWSDGYSDFDRGAYVVTFVPKSWNTAPDKVTQGWP
**SEQ ID NO: 27** - Transglutaminase (activated 1, from S. mobaraensis) 
FRAPDSDDRVTPPAEPLDRMPDPYRPSYGRAETVVNNYIRKWQQVYSHRDGRKQQMTEEQREWLSYGCVGVT
WVNSGQYPTNRLAFASFDEDRFKNELKNGRPRSGETRAEFEGRVAKESFDEEKGFQRAREVASVMNRALENAHD
ESAYLDNLKKELANGNDALRNEDARSPFYSALRNTPSFKERNGGNHDPSRMKAVIYSKHFWSGQDRSSSADKRK
YGDPDAFRPAPGTGLVDMSRDRNIPRSPTSPGEGFVNFDYGWFGAQTEADADKTVWTHGNHYHAPNGSLGAM
HVYESKFRNWSEGYSDFDRGAYVITFIPKSWNTAPDKVKQGWPLE
**SEQ ID NO: 28** - Transglutaminase (activated, version 3) 
DSDERVTPPAEPLDRMPDPYRPSYGRAETIVNNYIRKWQQVYSHRDGRKQQMTEEQREWLSYGCVGVTWVNSG
QYPTNRLAFAFFDEDKYKNELKNGRPRSGETRAEFEGRVAKDSFDEAKGFQRARDVASVMNKALENAHDEGAYL
DNLKKELANGNDALRNEDARSPFYSALRNTPSFKDRNGGNHDPSKMKAVIYSKHFWSGQDRSGSSDKRKYGDPE
AFRPDRGTGLVDMSRDRNIPRSPTSPGESFVNFDYGWFGAQTEADADKTVWTHGNHYHAPNGSLGAMHVYESK
FRNWSDGYSDFDRGAYVVTFVPKSWNTAPDKVTQGWPLE
**SEQ ID NO: 29** - Transglutaminase (activated, version 4) 
DSDERVTPPAEPLDRMPDPYRPSYGRAETIVNNYIRKWQQVYSHRDGRKQQMTEEQREWLSYGCVGVTWVNSG
QYPTNRLAFAFFDEDKYKNELKNGRPRSGETRAEFEGRVAKDSFDEAKGFQRARDVASVMNKALENAHDEGAYL
DNLKKELANGNDALRNEDARSPFYSALRNTPSFKDRNGGNHDPSKMKAVIYSKHFWSGQDRSGSSDKRKYGDPE
 
AFRPDRGTGLVDMSRDRNIPRSPTSPGESFVNFDYGWFGAQTEADADKTVWTHGNHYHAPNGSLGAMHVYESK
FRNWSDGYSDFDRGAYVVTFVPKSWNTAPDKVTQGW
**SEQ ID NO: 30** - Transglutaminase (activated, version 2 from S. Ladakanum) 
FRAPDSDERVTPPAEPLDRMPDPYRPSYGRAETIVNNYIRKWQQVYSHRDGRKQQMTEEQREWLSYGCVGVTW
VNSGQYPTNRLAFAFFDEDKYKNELKNGRPRSGETRAEFEGRVAKDSFDEAKGFQRARDVASVMNKALENAHDE
GAYLDNLKKELANGNDALRNEDARSPFYSALRNTPSFKDRNGGNHDPSKMKAVIYSKHFWSGQDRSGSSDKRKY
GDPEAFRPDRGTGLVDMSRDRNIPRSPTSPGESFVNFDYGWFGAQTEADADKTVWTHGNHYHAPNGSLGAMH
VYESKFRNWSDGYSDFDRGAYVVTFVPKSWNTAPDKVTQGWPLE
**SEQ ID NO: 31** - MTG_5 (activated 2 from S. mobaraensis) 
FRAPDSDDRVTPPAEPLDRMPDPYRPSYGRAETVVNNYIRKWQQVYSHRDGRKQQMTEEQREWLSYGCVGVT
WVNSGQYPTNRLAFASFDEDRFKNELKNGRPRSGETRAEFEGRVAKESFDEEKGFQRAREVASVMNRALENAHD
ESAYLDNLKKELANGNDALRNEDARSPFYSALRNTPSFKERNGGNHDPSRMKAVIYSKHFWSGQDRSSSADKRK
YGDPDAFRPAPGTGLVDMSRDRNIPRSPTSPGEGFVNFDYGWFGAQTEADADKTVWTHGNHYHAPNGSLGAM
HVYESKFRNWSEGYSDFDRGAYVITFIPKSWNTAPDKVKQGWP
**SEQ ID NO: 32** - MTG_6 (activated 3 from S. mobaraensis) 
DSDDRVTPPAEPLDRMPDPYRPSYGRAETVVNNYIRKWQQVYSHRDGRKQQMTEEQREWLSYGCVGVTWVNS
GQYPTNRLAFASFDEDRFKNELKNGRPRSGETRAEFEGRVAKESFDEEKGFQRAREVASVMNRALENAHDESAYL
DNLKKELANGNDALRNEDARSPFYSALRNTPSFKERNGGNHDPSRMKAVIYSKHFWSGQDRSSSADKRKYGDPD
AFRPAPGTGLVDMSRDRNIPRSPTSPGEGFVNFDYGWFGAQTEADADKTVWTHGNHYHAPNGSLGAMHVYESK
FRNWSEGYSDFDRGAYVITFIPKSWNTAPDKVKQGWPLE
**SEQ ID NO: 33** - MTG_7 (activated 4 from S. mobaraensis) 
DSDDRVTPPAEPLDRMPDPYRPSYGRAETVVNNYIRKWQQVYSHRDGRKQQMTEEQREWLSYGCVGVTWVNS
GQYPTNRLAFASFDEDRFKNELKNGRPRSGETRAEFEGRVAKESFDEEKGFQRAREVASVMNRALENAHDESAYL
DNLKKELANGNDALRNEDARSPFYSALRNTPSFKERNGGNHDPSRMKAVIYSKHFWSGQDRSSSADKRKYGDPD
AFRPAPGTGLVDMSRDRNIPRSPTSPGEGFVNFDYGWFGAQTEADADKTVWTHGNHYHAPNGSLGAMHVYESK
FRNWSEGYSDFDRGAYVITFIPKSWNTAPDKVKQGWP
**SEQ ID NO: 34** - MTG_8 (from S. mobaraensis) V1 
MDNGAGEETKSYAETYRLTADDVANINALNESAPAASSAGPSFRAPDSDDRVTPPAEPLDRMPDPYRPSYGRAET
VVNNYIRKWQQVYSHRDGRKQQMTEEQREWLSYGCVGVTWVNSGQYPTNRLAFASFDEDRFKNELKNGRPRS
GETRAEFEGRVAKESFDEEKGFQRAREVASVMNRALENAHDESAYLDNLKKELANGNDALRNEDARSPFYSALRN
TPSFKERNGGNHDPSRMKAVIYSKHFWSGQDRSSSADKRKYGDPDAFRPAPGTGLVDMSRDRNIPRSPTSPGEG
FVNFDYGWFGAQTEADADKTVWTHGNHYHAPNGSLGAMHVYESKFRNWSEGYSDFDRGAYVITFIPKSWNTAP
DKVKQGWP
**SEQ ID NO: 35** - mTG tag
   GGTLQSPP
**SEQ ID NO: 36** - mTG tag
   TLQSG
**SEQ ID NO: 37** - mTG tag
   TLQSPP
**SEQ ID NO: 38** - mTG tag
   GGTLQSG
**SEQ ID NO: 39** - mTG tag
   TLQSA
**SEQ ID NO: 40** - Heavy chain 2 (HC2) of monoEGFR, monoEGFR-ADC1 and monoEGFR-fluo1 of Table 10 
EPKSSDKTHTSPPCPAPEAAGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLPPCREEMTKNQVSLW
CLVKGFYPSDIAVEWESNGQPENNYKTTPPVLDSDGSFFLYSKLTVDKSRWQQGNVFSCSVMHEALHNHYTQKS
LSLSPGK
**SEQ ID NO: 41 -** Heavy chain (HC) of monoMUC-ADC1, and mono-MUC fluo1 of Table 10 
QMQLVQSEAELKKPGASVKVSCKASGYSFTSHFMHWVRQAPGQGLEWMGWIDPVTGGTKYAQNFQGWVTMT
RDTSIRTAYLELSRLRSDDTAMYYCAREARADRGQFDKWGQGTLVTVASGGGGSGGGGSGGGGSQSVLTQPPS
VSVAPGKTARITCGGNNIGSKSVHWYQQKPGQAPALVIYYGSNRPSGIPERFSGSNSGNTATLTISRVEAGDEAD
YYCQVWDSSSDWVFGGGTKLTVLEPKSSDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEKTISKAKGQP
FRPEVHLLPPSREEMTKNQVSLTCLARGFYPKDIAVEWESNGQPENNYKTTPSRQEPSQGTTTFAVTSKLTVDKS
RWQQGNVFSCSVMHEALHNHYTQKTISLSPGK
**SEQ ID NO: 42-** - Heavy chain 2 (HC2) of monoMUC-ADC1, and mono-MUC fluo1 of Table 10 
EPKSSDKTHTCPPCPAPELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLPPPSEELALNELVTLTCL
VKGFYPSDIAVEWLQGSQELPREKYLTWAPVLDSDGSFFLYSILRVAAEDWKKGDTFSCSVMHEALHNHYTQKSL
DRSPGK
**SEQ ID NO: 43** - Heavy chain (HC) of monoMUC-ADC2 of Table 10 
EPKSSDKTHTCPPCPAPEAAGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVCTLPPSREEMTKNQVSLSC
AVKGFYPSDIAVEWESNGQPENNYKTTPPVLDSDGSFFLVSKLTVDKSRWQQGNVFSCSVMHEALHNHYTQKSL
SLSPGK
**SEQ ID NO: 44** - Heavy chain 2 (HC2) of monoMUC-ADC2 of Table 10 
QMQLVQSEAELKKPGASVKVSCKASGYSFTSHFMHWVRQAPGQGLEWMGWIDPVTGGTKYAQNFQGWVTMT
RDTSIRTAYLELSRLRSDDTAMYYCAREARADRGQFDKWGQGTLVTVASGGGGSGGGGSGGGGSQSVLTQPPS
VSVAPGKTARITCGGNNIGSKSVHWYQQKPGQAPALVIYYGSNRPSGIPERFSGSNSGNTATLTISRVEAGDEAD
YYCQVWDSSSDWVFGGGTKLTVLEPKSSDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIEKTISKAKGQ
PREPQVYTLPPCREEMTKNQVSLWCLVKGFYPSDIAVEWESNGQPENNYKTTPPVLDSDGSFFLYSKLTVDKSRW
QQGNVFSCSVMHEALHNHYTQKSLSLSPGK
**SEQ ID NO: 45** - Heavy chain (HC) of bivEGFR, bivEGFR-ADC1, bivEGFR-fluo1 
EVQLVQSGAEVKKPGASVKVSCKASGFSLTNYGVHWMRQAPGQGLEWIGVIWSGGNTDYNTPFTSRVTITSDKS
TSTAYMELSSLRSEDTAVYYCARALTYYDYEFAYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKRVEPKSSDKTH
TCPPCPAPEAAGGPSVFLFPPKPKDTLMISRTPEVTCVVVDVSHEDPEVKFNWYVDGVEVHNAKTKPREEQYNST
 
YRVVSVLTVLHQDWLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYTQKSLSLSPGK
**SEQ ID NO: 46** - Heavy chain (HC) of bivMUC, bivMUC-ADC1, bivMUC-fluo1 
QMQLVQSEAELKKPGASVKVSCKASGYSFTSHFMHWVRQAPGQGLEWMGWIDPVTGGTKYAQNFQGWVTMT
RDTSIRTAYLELSRLRSDDTAMYYCAREARADRGQFDKWGQGTLVTVASASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKRVEPKS
SDKTHTCPPCPAPEAAGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFSCSVMHEALHNHYTQKSLSLSP
GK
**SEQ ID NO: 47** - Light chain (LC) of bivMUC, bivMUC-ADC1, bivMUC-fluo1 
QSVLTQPPSVSVAPGKTARITCGGNNIGSKSVHWYQQKPGQAPALVIYYGSNRPSGIPERFSGSNSGNTATLTISRV
EAGDEADYYCQVWDSSSDWVFGGGTKLTVLGQPKAAPSVTLFPPSSEELQANKATLVCLISDFYPGAVTVAWKAD
SSPVKAGVETTTPSKQSNNKYAASSYLSLTPEQWKSHKSYSCQVTHEGSTVEKTVAPTESS
**SEQ ID NO: 48** - Heavy chain (HC) of iso-MAB1, iso-ADC1, and iso-fluo1 
QVQLVESGGGLVKPGGSLRLSCAASGFTFSDYAMSWIRQAPGKGLEWVASINIGATYAYYPDSVKGRFTISRDNA
KNSLYLQMNSLRAEDTAVYYCARPGSPYEYDKAYYSMAYWGQGTTVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKRVEPK
SCDKTHTCPPCPAPEAAGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEALHNHYTQKSLSL
SPGK
**SEQ ID NO: 49** - Light chain (LC) of iso-MAB1, iso-ADC1, and iso-fluo1 
DIQMTQSPSSLSASVGDRVTITCRASQDIKNYLNWYQQKPGKAPKLLIYYSSTLLSGVPSRFSGSGSGTDFTLTISS
LQPEDFATYYCQQSITLPPTFGGGTKVEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC
**SEQ ID NO: 50** - Heavy chain (HC) of iso-MAB2, iso-ADC2, and iso-fluo2 
QVQLQQPGAELVKPGASVKMSCKASGYTFTSYNMHWVKQTPGRGLEWIGAIYPGNGDTSYNQKFKGKATLTAD
KSSSTAYMQLSSLTSEDSAVYYCARSTYYGGDWYFNVWGAGTTVTVSAASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEPKSC
DKTHTCPPCPAPEAAGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSVMHEALHNHYTQKSLSLSPG
K
**SEQ ID NO: 51** - Light chain (LC) of iso-MAB2, iso-ADC2, and iso-fluo2 
QIVLSQSPAILSASPGEKVTMTCRASSSVSYIHWFQQKPGSSPKPWIYATSNLASGVPVRFSGSGSGTSYSLTISRV
EAEDAATYYCQQWTSNPPTFGGGTKLEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC
**SEQ ID NO: 52** - MTG_5 (from S. mobaraensis) 
DSDDRVTPPAEPLDRMPDPYRPSYGRAETVVNNYIRKWQQVYSHRDGRKQQMTEEQREWLSYGCVGVTWVNS
GQYPTNRLAFASFDEDRFKNELKNGRPRSGETRAEFEGRVAKESFDEEKGFQRAREVASVMNRALENAHDESAYL
 
DNLKKELANGNDALRNEDARSPFYSALRNTPSFKERNGGNHDPSRMKAVIYSKHFWSGQDRSSSADKRKYGDPD
AFRPAPGTGLVDMSRDRNIPRSPTSPGEGFVNFDYGWFGAQTEADADKTVWTHGNHYHAPNGSLGAMHVYESK
FRNWSEGYSDFDRGAYVITFIPKSWNTAPDKVKQGWP
**SEQ ID NO: 53** - MTG_7 (from S. mobaraensis) 
FRAPDSDDRVTPPAEPLDRMPDPYRPSYGRAETVVNNYIRKWQQVYSHRDGRKQQMTEEQREWLSYGCVGVT
WVNSGQYPTNRLAFASFDEDRFKNELKNGRPRSGETRAEFEGRVAKESFDEEKGFQRAREVASVMNRALENAHD
ESAYLDNLKKELANGNDALRNEDARSPFYSALRNTPSFKERNGGNHDPSRMKAVIYSKHFWSGQDRSSSADKRK
YGDPDAFRPAPGTGLVDMSRDRNIPRSPTSPGEGFVNFDYGWFGAQTEADADKTVWTHGNHYHAPNGSLGAM
HVYESKFRNWSEGYSDFDRGAYVITFIPKSWNTAPDKVKQGWP
**SEQ ID NO: 54** - MTG_8 (from S. mobaraensis) 
FRAPDSDDRVTPPAEPLDRMPDPYRPSYGRAETVVNNYIRKWQQVYSHRDGRKQQMTEEQREWLSYGCVGVT
WVNSGQYPTNRLAFASFDEDRFKNELKNGRPRSGETRAEFEGRVAKESFDEEKGFQRAREVASVMNRALENAHD
ESAYLDNLKKELANGNDALRNEDARSPFYSALRNTPSFKERNGGNHDPSRMKAVIYSKHFWSGQDRSSSADKRK
YGDPDAFRPAPGTGLVDMSRDRNIPRSPTSPGEGFVNFDYGWFGAQTEADADKTVWTHGNHYHAPNGSLGAM
HVYESKFRNWSEGYSDFDRGAYVITFIPKSWNTAPDKVKQGWPLE
**SEQ ID NO: 55** - Human EGFR-His 
LEEKKVCQGTSNKLTQLGTFEDHFLSLQRMFNNCEVVLGNLEITYVQRNYDLSFLKTIQEVAGYVLIALNTVERIPL
ENLQIIRGNMYYENSYALAVLSNYDANKTGLKELPMRNLQEILHGAVRFSNNPALCNVESIQWRDIVSSDFLSNMS
MDFQNHLGSCQKCDPSCPNGSCWGAGEENCQKLTKIICAQQCSGRCRGKSPSDCCHNQCAAGCTGPRESDCLV
CRKFRDEATCKDTCPPLMLYNPTTYQMDVNPEGKYSFGATCVKKCPRNYVVTDHGSCVRACGADSYEMEEDGVR
KCKKCEGPCRKVCNGIGIGEFKDSLSINATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRSLKEISDGDVIISGNKNLCYANTINWKK
LFGTSGQKTKIISNRGENSCKATGQVCHALCSPEGCWGPEPRDCVSCRNVSRGRECVDKCKLLEGEPREFVENSE
CIQCHPECLPQAMNITCTGRGPDNCIQCAHYIDGPHCVKTCPAGVMGENNTLVWKYADAGHVCHLCHPNCTYGC
TGPGLEGCPTNGPKHHHHHH
**SEQ ID NO: 56** - Cyno EGFR-His 
LEEKKVCQGTSNKLTQLGTFEDHFLSLQRMFNNCEVVLGNLEITYVQRNYDLSFLKTIQEVAGYVLIALNTVERIPL
ENLQIIRGNMYYENSYALAVLSNYDANKTGLKELPMRNLQEILHGAVRFSNNPALCNVESIQWRDIVSSEFLSNMS
MDFQNHLGSCQKCDPSCPNGSCWGAGEENCQKLTKIICAQQCSGRCRGKSPSDCCHNQCAAGCTGPRESDCLV
CRKFRDEATCKDTCPPLMLYNPTTYQMDVNPEGKYSFGATCVKKCPRNYVVTDHGSCVRACGADSYEMEEDGVR
KCKKCEGPCRKVCNGIGIGEFKDTLSINATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRSLKEISDGDVIISGNKNLCYANTINWKK
LFGTSSQKTKIISNRGENSCKATGQVCHALCSPEGCWGPEPRDCVSCQNVSRGRECVDKCNILEGEPREFVENSE
CIQCHPECLPQVMNITCTGRGPDNCIQCAHYIDGPHCVKTCPAGVMGENNTLVWKYADAGHVCHLCHPNC
TYGCTGPGLEGCARNGPKIPSHHHHHH
**SEQ ID NO: 57** - 14MUC_GGGS_VHH

MAPDTRPAPGSTAPPAGGGGSGGGGSGGGGSEVQLVESGGGLVQPGGSLRLSCAASGRSLSNYYMGWFRQAPG
KGRELLGNISWRGYNIYYKDSVKGRFTISRDDSKNTIYLQMNSLKTEDTAVYYCAASILPLSDDPGWNTYWGQGT
QVTVSSHHHHHH

### Detailed Description of the Invention

The invention provides a novel immunoconjugate with a molecular structure that maximizes the therapeutic efficacy and safety.

In some embodiments, an immunoconjugate is provided comprising:
(a) a bispecific antibody that binds to EGFR and MUC1,
(b) a growth inhibiting agent, and
(c) a linker covalently linking (a) and (b).

The components of immunoconjugates (i.e., antibody, growth inhibiting agent, and linker), the immunoconjugates, methods for preparing the immunoconjugates, pharmaceutical compositions comprising the immunoconjugates, medical uses of the immunoconjugates, dosing regimens of the immunoconjugates, and specific embodiments and examples thereof are described in the detailed description of the invention hereinafter:

| | |
|---|---|
| Anti-EGFR and MUC1 bispecific antibody of the immunoconjugate | 26 |
| Bispecific antibody characterized by HCDR and LCDR regions | 26 |
| Bispecific antibody characterized by VH and VL regions | 31 |
| Constant and Fc region of the bispecific antibody | 39 |
| Types of Antibodies | 46 |
| Glycosylation variants | 49 |
| Cysteine engineered antibody variants | 51 |
| Multispecificity of the antibody | 51 |
| Growth inhibiting agent of the immunoconjugate | 53 |
| Linker and drug linker compound of the immunoconjugate | 54 |
| Immunoconjugate | 60 |
| Methods of preparing immunoconjugates | 66 |
| Pharmaceutical compositions | 69 |
| Medical use and methods of treatment of the immunoconjugate | 72 |
| Use of the immunoconjugate for the manufacture of a medicament | 74 |
| Method of treating cancer | 74 |
| Immunoconjugate for use as a medicament or for use in treating cancer | 75 |
| Selected advantages of the medical use and methods of treatment | 75 |
| Dosing regimen | 76 |
| Embodiments of the invention | 80 |
| Examples | 143 |
| Summary of molecules used in the Examples | 143 |
| Example 1 - Drug Linker compound Formula (I) synthesis | 146 |
| Example 2 - Production of EGFR/MUC-MAB2 and EGFR/MUC-MAB3 | 153 |
| Example 3- EGFR/MUC-MAB3 conjugation to yield EGFR/MUC ADC3 | 154 |
| Example 4 - Pharmacokinetics of SEED-based antibodies and ADCs | 156 |
| Example 5 - KiH (knob-into-hole) design of EGFR/MUC-MAB4, EGFR/MUC-MAB5 - production and conjugation to yield EGFR/MUC-ADC4 and EGFR/MUC-ADC5 | 157 |
| Example 6 - In vivo studies to evaluate pharmacokinetics properties of EGFR/MUC-ADC4 EGFR/MUC-ADC5 | and 160 |
| Example 7 - Design and production and upscaling of knob-into-hole based exatecan EGFR/MUC-ADC6. | 160 |
| Example 8 - In vivo studies to compare pharmacokinetic properties of EGFR/MUC-ADC6 | and |
| EGFR/MUC-ADC1 | 162 |
| Example 9 - Target binding of EGFRxMUC1 bispecific ADCs | 162 |
| Example 10 - Generation of mono-specific comparative molecules of different valency | 163 |
| Example 11 - Cell binding of anti EGFRxMUC1 molecules | 163 |
| Example 12 - In vivo efficacy studies | 167 |
| Example 13: Comparison of EGFR/MUC-ADC6 with EGFR-ADC-MMAE, EGFR/cMet-ADC, EGFR/HER3-ADC | 168 |
| Example 14 - Dose-range finding toxicity studies in NHPs (cynomolgus monkeys) | 170 |

### Anti-EGFR and MUC1 bispecific antibody of the immunoconjugate

The invention provides a novel immunoconjugate with a molecular structure that maximizes the therapeutic efficacy and safety.

In some embodiments, an immunoconjugate is provided comprising:
(a) a bispecific antibody that binds to EGFR and MUC1,
(b) a growth inhibiting agent, and
(c) a linker covalently linking (a) and (b).

### Bispecific antibody characterized by HCDR and LCDR regions

In some embodiments, the bispecific antibody that binds to EGFR and MUC1 comprises an EGFR binding portion comprising a heavy chain variable region (VH) comprising a heavy chain complementary determining region (HCDR) 1 of SEQ ID NO: 1.

In some embodiments, the bispecific antibody that binds to EGFR and MUC1 comprises an EGFR binding portion comprising a heavy chain variable region (VH) comprising an HCDR 2 of SEQ ID NO: 2.

In some embodiments, the bispecific antibody that binds to EGFR and MUC1 comprises an EGFR binding portion comprising a heavy chain variable region (VH) comprising an HCDR 3 of SEQ ID NO: 3.

In some embodiments, the bispecific antibody that binds to EGFR and MUC1 comprises an EGFR binding portion comprising a heavy chain variable region (VH) comprising a heavy chain complementary determining region (HCDR) 1 of SEQ ID NO: 1, a HCDR 2 of SEQ ID NO: 2, and a HCDR 3 of SEQ ID NO: 3.

In some embodiments, the bispecific antibody that binds to EGFR and MUC1 comprises an EGFR binding portion comprising a light chain variable region (VL) comprising a light chain complementarity determining region (LCDR) 1 of SEQ ID NO: 4.

In some embodiments, the bispecific antibody that binds to EGFR and MUC1 comprises an EGFR binding portion comprising a light chain variable region (VL) comprising a LCDR 2 of SEQ ID NO: 5.

In some embodiments, the bispecific antibody that binds to EGFR and MUC1 comprises an EGFR binding portion comprising a light chain variable region (VL) comprising at least one LCDR 3 of SEQ ID NO: 6.

In some embodiments, the bispecific antibody that binds to EGFR and MUC1 comprises
- an EGFR binding portion comprising
   a heavy chain variable region (VH) comprising
   a heavy chain complementary determining region (HCDR) 1 of SEQ ID NO: 1,
   a HCDR 2 of SEQ ID NO: 2, and
   a HCDR 3 of SEQ ID NO: 3, and
      a light chain variable region (VL) comprising
   a light chain complementarity determining region (LCDR) 1 of SEQ ID NO: 4,
   a LCDR 2 of SEQ ID NO: 5, and
   a LCDR 3 of SEQ ID NO: 6.

In some embodiments, the bispecific antibody that binds to EGFR and MUC1 comprises a MUC1 binding portion comprising a heavy chain variable region (VH) comprising a heavy chain complementary determining region (HCDR) 1 of SEQ ID NO: 7.

In some embodiments, the bispecific antibody that binds to EGFR and MUC1 comprises a MUC1 binding portion comprising a heavy chain variable region (VH) comprising an HCDR 2 of SEQ ID NO: 8.

In some embodiments, the bispecific antibody that binds to EGFR and MUC1 comprises a MUC1 binding portion comprising a heavy chain variable region (VH) comprising an HCDR 3 of SEQ ID NO: 9.

In some embodiments, the bispecific antibody that binds to EGFR and MUC1 comprises a MUC1 binding portion comprising a heavy chain variable region (VH) comprising a heavy chain complementary determining region (HCDR) 1 of SEQ ID NO: 7, a HCDR 2 of SEQ ID NO: 8, and a HCDR 3 of SEQ ID NO: 9.

In some embodiments, the bispecific antibody that binds to EGFR and MUC1 comprises a MUC1 binding portion comprising a light chain variable region (VL) comprising a light chain complementarity determining region (LCDR) 1 of SEQ ID NO: 10.

In some embodiments, the bispecific antibody that binds to EGFR and MUC1 comprises a MUC1 binding portion comprising a light chain variable region (VL) comprising a LCDR 2 of SEQ ID NO: 11.

In some embodiments, the bispecific antibody that binds to EGFR and MUC1 comprises a MUC1 binding portion comprising a light chain variable region (VL) comprising at least one LCDR 3 of SEQ ID NO: 12.

In some embodiments, the bispecific antibody that binds to EGFR and MUC1 comprises
- a MUC1 binding portion comprising a
   heavy chain variable region (VH) comprising
   a heavy chain complementary determining region (HCDR) 1 of SEQ ID NO: 7,
   a HCDR 2 of SEQ ID NO: 8, and
   a HCDR 3 selected from the group consisting of SEQ ID NO: 9, and
   a light chain variable region (VL) comprising
   a light chain complementarity determining region (LCDR) 1 of SEQ ID NO: 10,
   a LCDR 2 of SEQ ID NO: 11, and
   a LCDR 3 of SEQ ID NO: 12.

In some embodiments, the bispecific antibody that binds to EGFR and MUC1 comprises
- an EGFR binding portion comprising
   a heavy chain variable region (VH) comprising
   a heavy chain complementary determining region (HCDR) 1 of SEQ ID NO: 1,
   a HCDR 2 of SEQ ID NO: 2, and
   a HCDR 3 selected from the group consisting of SEQ ID NO: 3, and
   a light chain variable region (VL) comprising
   a light chain complementarity determining region (LCDR) 1 of SEQ ID NO: 4,
   a LCDR 2 of SEQ ID NO: 5, and
   a LCDR 3 of SEQ ID NO: 6,
   and
- a MUC1 binding portion comprising
   a heavy chain variable region (VH) comprising
   a heavy chain complementary determining region (HCDR) 1 of SEQ ID NO: 7,
   a HCDR 2 of SEQ ID NO: 8, and
   a HCDR 3 selected from the group consisting of SEQ ID NO: 9, and
   a light chain variable region (VL) comprising
   a light chain complementarity determining region (LCDR) 1 of SEQ ID NO: 10,
   a LCDR 2 of SEQ ID NO: 11, and
   a LCDR 3 of SEQ ID NO: 12.

In some embodiments, any of the above-described CDR sequences contain substitutions (e.g., conservative substitutions), insertions, or deletions relative to the reference CDR sequence, preferably wherein the binding affinity (K_{D}) of the bispecific antibody to EGFR and/or MUC1 such as human EGFR and/or MUC1, respectively, is not altered by more than 10% preferably as measured by biolayer interferometry (BLI) or Surface Plasmon Resonance (SPR).

In certain embodiments, at least one amino acid has been substituted, inserted and/or deleted in any of the CDRs described above, preferably wherein the binding affinity (K_{D}) of the bispecific antibody to EGFR and MUC1 such as human EGFR and/or MUC1, respectively, is not altered by more than 10% (compared to an antibody with the reference CDR sequences without substitution/insertion/deletion), preferably as measured by biolayer interferometry (BLI) or Surface Plasmon Resonance (SPR).

In certain embodiments, at least one amino acid has been substituted, inserted and/or deleted in at least one of HCDR1, HCDR2, HCDR3 of EGFR described above, preferably wherein the binding affinity (K_{D}) of the bispecific antibody to EGFR such as human EGFR, respectively, is not altered by more than 10%, preferably as measured by biolayer interferometry (BLI) or Surface Plasmon Resonance (SPR). In certain embodiments, at least one amino acid has been substituted, inserted and/or deleted in at least one of LCDR1, LCDR2, LCDR3 of EGFR described above, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In certain embodiments, at least two amino acid has been substituted, inserted and/or deleted in at least one of HCDR1, HCDR2, HCDR3 of EGFR described above, preferably wherein the binding affinity (K_{D}) of the bispecific antibody to EGFR such as human EGFR, respectively, is not altered by more than 10%, preferably as measured by biolayer interferometry (BLI) or Surface Plasmon Resonance (SPR). In certain embodiments, at least two amino acid has been substituted, inserted and/or deleted in at least one of LCDR1, LCDR2, LCDR3 of EGFR described above, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In certain embodiments, at least three amino acid has been substituted, inserted and/or deleted in at least one of HCDR1, HCDR2, HCDR3 of EGFR described above, preferably wherein the binding affinity (K_{D}) of the bispecific antibody to EGFR such as human EGFR, respectively, is not altered by more than 10%, preferably as measured by biolayer interferometry (BLI) or Surface Plasmon Resonance (SPR). In certain embodiments, at least three amino acid has been substituted, inserted and/or deleted in at least one of LCDR1, LCDR2, LCDR3 of EGFR described above, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In certain embodiments, at least one amino acid has been substituted, inserted and/or deleted in at least one of HCDR1, HCDR2, HCDR3 of MUC1 described above, preferably wherein the binding affinity (K_{D}) of the bispecific antibody to MUC1 such as human MUC1, respectively, is not altered by more than 10%, preferably as measured by biolayer interferometry (BLI) or Surface Plasmon Resonance (SPR). In certain embodiments, at least one amino acid has been substituted, inserted and/or deleted in at least one of LCDR1, LCDR2, LCDR3 of MUC1 described above, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In certain embodiments, at least two amino acid has been substituted, inserted and/or deleted in at least one of HCDR1, HCDR2, HCDR3 of MUC1 described above, preferably wherein the binding affinity (K_{D}) of the bispecific antibody to MUC1 such as human MUC1, respectively, is not altered by more than 10%, preferably as measured by biolayer interferometry (BLI) or Surface Plasmon Resonance (SPR). In certain embodiments, at least two amino acid has been substituted, inserted and/or deleted in at least one of LCDR1, LCDR2, LCDR3 of MUC1 described above, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In certain embodiments, at least three amino acid has been substituted, inserted and/or deleted in at least one of HCDR1, HCDR2, HCDR3 of MUC1 described above, preferably wherein the binding affinity (K_{D}) of the bispecific antibody to MUC1 such as human MUC1, respectively, is not altered by more than 10%, preferably as measured by biolayer interferometry (BLI) or Surface Plasmon Resonance (SPR). In certain embodiments, at least three amino acid has been substituted, inserted and/or deleted in at least one of LCDR1, LCDR2, LCDR3 of MUC1 described above, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, the binding affinity (KD) of the bispecific antibody to EGFR such as human EGFR is not attenuated.

In some embodiments, the bispecific antibody binds to EGFR with a binding affinity (KD), preferably wherein the KD is less than 10 nM such as less than 5 nM. In some embodiments, the bispecific antibody binds to EGFR with a binding affinity (KD), preferably wherein the KD is less than 10 nM. In some embodiments, the bispecific antibody binds to EGFR with a binding affinity (KD), preferably wherein the KD is less than 5 nM. In some embodiments, the bispecific antibody binds to EGFR with a binding affinity (KD), preferably wherein the KD is less than 10 nM such as from 2nM to 5 nM. In some embodiments, the bispecific antibody binds to EGFR with a binding affinity (KD), preferably wherein the KD is less than 10 nM. In some embodiments, the bispecific antibody binds to EGFR with a binding affinity (KD), preferably wherein the KD is from 1.5 to 2 nM. In some embodiments, the bispecific antibody binds to EGFR with a binding affinity (KD), preferably wherein the KD is from 1.5 to 2 nM.

In some embodiments, the bispecific antibody binds to human EGFR with a binding affinity (KD), preferably wherein the KD is less than 10 nM such as less than 5 nM. In some embodiments, the bispecific antibody binds to human EGFR with a binding affinity (KD), preferably wherein the KD is less than 10 nM. In some embodiments, the bispecific antibody binds to human EGFR with a binding affinity (KD), preferably wherein the KD is less than 5 nM. In some embodiments, the bispecific antibody binds to human EGFR with a binding affinity (KD), preferably wherein the KD is less than 10 nM such as from 2nM to 5 nM. In some embodiments, the bispecific antibody binds to human EGFR with a binding affinity (KD), preferably wherein the KD is less than 10 nM. In some embodiments, the bispecific antibody binds to human EGFR with a binding affinity (KD), preferably wherein the KD is from 1.5 to 2 nM. In some embodiments, the bispecific antibody binds to human EGFR with a binding affinity (KD), preferably wherein the KD is from 1.5 to 2 nM.

### Bispecific antibody characterized by VH and VL regions

This section describes the heavy chain variable region (VH) and light chain variable region (VL) of the anti-EGFR and MUC1 bispecific antibody in the immunoconjugate. The embodiments in this section define the bispecific antibody both independent of the embodiments in the previous section and in combination with the embodiments in the previous section.

In some embodiments, the EGFR VH is a humanized VH and/or the EGFR VL is a humanized VL. In some embodiments, the antibody comprises CDRs as in any of the above embodiments, and further comprises an acceptor human framework, e.g. a human immunoglobulin framework or a human consensus framework.

In some embodiments, the MUC1 VH is a fully human VH and/or the MUC1 VL is a fully human VL.

In some embodiments, the bispecific antibody comprises an EGFR binding portion comprising a VH comprising an amino acid sequence that is at least 90%, 95%, 96%, 97%, 98%, 99% or 100% identical to the amino acid sequence of SEQ ID NO: 13.

In some embodiments, the bispecific antibody comprises an EGFR binding portion comprising a VH comprising an amino acid sequence that is at least 90% identical to the amino acid sequence of SEQ ID NO: 13.

In some embodiments, the bispecific antibody comprises an EGFR binding portion comprising a VH comprising an amino acid sequence that is at least 95% identical to the amino acid sequence of SEQ ID NO: 13.

In some embodiments, the bispecific antibody comprises an EGFR binding portion comprising a VH comprising an amino acid sequence that is at least 99% identical to the amino acid sequence of SEQ ID NO: 13.

In some embodiments, the bispecific antibody comprises an EGFR binding portion comprising a VH comprising an amino acid sequence of SEQ ID NO: 13.

In some embodiments, the above-described percentage identities to the EGFR VH are in combination with 100% identity to the EGFR HCDR1, HCDR2, and HCDR3 of SEQ ID NO: 1, 2, and 3, respectively.

In some embodiments, the bispecific antibody comprises an EGFR binding portion comprising a VH comprising an amino acid sequence with a percentage identity to an amino acid sequence of SEQ ID NO: 13 as described above, wherein said VH comprises a HCDR1 of SEQ ID NO: 1, a HCDR2 of SEQ ID NO: 2, and a HCDR3 of SEQ ID NO: 3.

In some embodiments, the bispecific antibody comprises an EGFR binding portion comprising a VL comprising an amino acid sequence that is at least 90%, 95%, 96%, 97%, 98%, 99% or 100% identical to the amino acid sequence of SEQ ID NO: 14.

In some embodiments, the bispecific antibody comprises an EGFR binding portion comprising a VL comprising an amino acid sequence that is at least 90% identical to the amino acid sequence of SEQ ID NO: 14.

In some embodiments, the bispecific antibody comprises an EGFR binding portion comprising a VL comprising an amino acid sequence that is at least 95% identical to the amino acid sequence of SEQ ID NO: 14.

In some embodiments, the bispecific antibody comprises an EGFR binding portion comprising a EGFR VL comprising an amino acid sequence that is at least 99% identical to the amino acid sequence of SEQ ID NO: 14.

In some embodiments, the bispecific antibody comprises an EGFR binding portion comprising a VL comprising an amino acid sequence of SEQ ID NO: 14.

In some embodiments, the above-described percentage identities to the EGFR VL are in combination with 100% identity to the LCDR1, LCDR2, and LCDR3 of SEQ ID NO: 4, 5, and 6, respectively.

In some embodiments, the bispecific antibody comprises an EGFR binding portion comprising a VL comprising an amino acid sequence with a percentage identity to an amino acid sequence of SEQ ID NO: 14 as described above, wherein said VL comprises a LCDR1 of SEQ ID NO: 4, a LCDR2 of SEQ ID NO: 5, and a LCDR3 of SEQ ID NO: 6.

In some embodiments, when the % identity is less than 100%, the binding affinity (K_{D}) of the bispecific antibody to EGFR such as human EGFR is not altered by more than 10%, preferably as measured by biolayer interferometry (BLI) or Surface Plasmon Resonance (SPR).

In some embodiments, the bispecific antibody comprises an EGFR binding portion comprising a VH comprising an amino acid sequence that is at least 90%, 95%, 96%, 97%, 98%, 99% or 100% identical to the amino acid sequence selected from the group of SEQ ID NO: 13 and a VL comprising an amino acid sequence that is at least 90%, 95%, 96%, 97%, 98%, 99% or 100% identical to the amino acid sequence of SEQ ID NO: 14.

In some embodiments, the bispecific antibody comprises an EGFR binding portion comprising a VH comprising an amino acid sequence that is at least 90% identical to the amino acid sequence selected from the group of SEQ ID NO: 13 and a VL comprising an amino acid sequence that is at least 90% identical to the amino acid sequence of SEQ ID NO: 14.

In some embodiments, the bispecific antibody comprises an EGFR binding portion comprising a VH comprising an amino acid sequence that is at least 95% identical to the amino acid sequence selected from the group of SEQ ID NO: 13 and a VL comprising an amino acid sequence that is at least 95% identical to the amino acid sequence of SEQ ID NO: 14.

In some embodiments, the bispecific antibody comprises an EGFR binding portion comprising a VH comprising an amino acid sequence that is at least 99% identical to the amino acid sequence selected from the group of SEQ ID NO: 13 and a VL comprising an amino acid sequence that is at least 99% identical to the amino acid sequence of SEQ ID NO: 14.

In some embodiments, the bispecific antibody comprises an EGFR binding portion comprising a VH sequence selected from the group of SEQ ID NO: 13, and a VL sequence of SEQ ID NO: 14.

In some embodiments, the above-described percentage identities to the EGFR VH are in combination with 100% identity to the HCDR1 (SEQ ID NO: 1), HCDR2 (SEQ ID NO: 2), and HCDR3 (SEQ ID NO: 3) and the above-described percentage identities to the EGFR VL are in combination with 100% identity to the LCDR1 (SEQ ID NO: 4), LCDR2 (SEQ ID NO: 5), and LCDR3 (SEQ ID NO: 6).

In some embodiments, the bispecific antibody comprises an EGFR binding portion comprising,
- a VH comprising an amino acid sequence with a percentage identity to an amino acid sequence of SEQ ID NO: 13 as described above, wherein said VH comprises a HCDR1 of SEQ ID NO: 1, a HCDR2 of SEQ ID NO: 2, and a HCDR3 of SEQ ID NO: 3; and
- a VL comprising an amino acid sequence with a percentage identity to an amino acid sequence of SEQ ID NO: 14 as described above, wherein said VL comprises a LCDR1 of SEQ ID NO: 4, a LCDR2 of SEQ ID NO: 5, and a LCDR3 of SEQ ID NO: 6.

In some embodiments, when the % identity is less than 100%, the binding affinity (K_{D}) of the bispecific antibody to EGFR such as human EGFR is not altered by more than 10%, preferably as measured by biolayer interferometry (BLI) or Surface Plasmon Resonance (SPR).

In some embodiments, a VH or VL sequence having at least 90%, 95%, 96%, 97%, 98%, or 99% identity contains substitutions (e.g., conservative substitutions), insertions, or deletions relative to the reference sequence, preferably wherein the binding affinity (K_{D}) of the bispecific antibody to EGFR such as human EGFR is not altered by more than 10%(compared to a sequence with 100% sequence identity), preferably as measured by biolayer interferometry (BLI) or Surface Plasmon Resonance (SPR).

In some embodiments, a VH or VL sequence having at least 90% contains substitutions (e.g., conservative substitutions), insertions, or deletions relative to the reference sequence, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In some embodiments, a VH or VL sequence having at least 95% contains substitutions (e.g., conservative substitutions), insertions, or deletions relative to the reference sequence, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In some embodiments, a VH or VL sequence having at least 99% contains substitutions (e.g., conservative substitutions), insertions, or deletions relative to the reference sequence, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In certain embodiments, a total of 1 to 10 amino acids have been substituted, inserted and/or deleted in SEQ ID NO: 13 and/or a total of 1 to 10 amino acids have been substituted, inserted and/or deleted in SEQ ID NO: 14, preferably wherein the binding affinity (K_{D}) of the bispecific antibody to EGFR such as human EGFR is not altered by more than 10%, preferably as measured by biolayer interferometry (BLI) or Surface Plasmon Resonance (SPR).

In certain embodiments, all substitutions, insertions, or deletions occur in regions outside the CDRs.

In some embodiments, the bispecific antibody comprises a MUC1 binding portion comprising a VH comprising an amino acid sequence that is at least 90%, 95%, 96%, 97%, 98%, 99% or 100% identical to the amino acid sequence of SEQ ID NO: 15.

In some embodiments, the bispecific antibody comprises a MUC1 binding portion comprising a VH comprising an amino acid sequence that is at least 90% identical to theamino acid sequence of SEQ ID NO: 15.

In some embodiments, the bispecific antibody comprises a MUC1 binding portion comprising a VH comprising an amino acid sequence that is at least 95% identical to the amino acid sequence of SEQ ID NO: 15.

In some embodiments, the bispecific antibody comprises a MUC1 binding portion comprising a VH comprising an amino acid sequence that is at least 99% identical to the amino acid sequence of SEQ ID NO: 15.

In some embodiments, the bispecific antibody comprises a MUC1 binding portion comprising a VH comprising an amino acid sequence of SEQ ID NO: 15.

In some embodiments, the above-described percentage identities to the MUC1 VH are in combination with 100% identity to the MUC1 HCDR1, HCDR2, and HCDR3 of SEQ ID NO: 7, 8, and 9, respectively.

In some embodiments, the bispecific antibody comprises a MUC1 binding portion comprising an amino acid sequence with a percentage identity to an amino acid sequence of SEQ ID NO: 15 as described above, wherein said VH comprises a HCDR1 of SEQ ID NO: 7, a HCDR2 of SEQ ID NO: 8, and a HCDR3 of SEQ ID NO: 9.

In some embodiments, the bispecific antibody comprises a MUC1 binding portion comprising a VL comprising an amino acid sequence that is at least 90%, 95%, 96%, 97%, 98%, 99% or 100% identical to the amino acid sequence of SEQ ID NO: 16.

In some embodiments, the bispecific antibody comprises a MUC1 binding portion comprising a VL comprising an amino acid sequence that is at least 90% identical to the amino acid sequence of SEQ ID NO: 16.

In some embodiments, the bispecific antibody comprises a MUC1 binding portion comprising a VL comprising an amino acid sequence that is at least 95% identical to the amino acid sequence of SEQ ID NO: 16.

In some embodiments, the bispecific antibody comprises a MUC1 binding portion comprising a VL comprising an amino acid sequence that is at least 99% identical to the amino acid sequence of SEQ ID NO: 16.

In some embodiments, the bispecific antibody comprises a MUC1 binding portion comprising a VL comprising an amino acid sequence of SEQ ID NO: 16.

In some embodiments, the above-described percentage identities to the MUC1 VL are in combination with 100% identity to the LCDR1, LCDR2, and LCDR3 of SEQ ID NO: 10, 11, and 12, respectively.

In some embodiments, the antibody comprises a VL comprising an amino acid sequence with a percentage identity to an amino acid sequence of SEQ ID NO: 16 as described above, wherein said VL comprises a LCDR1 of SEQ ID NO: 10, a LCDR2 of SEQ ID NO: 11, and a LCDR3 of SEQ ID NO: 12.

In some embodiments, when the % identity is less than 100%, the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, the bispecific antibody comprises a MUC1 binding portion comprising a VH comprising an amino acid sequence that is at least 90%, 95%, 96%, 97%, 98%, 99% or 100% identical to the amino acid sequence selected from the group of SEQ ID NO: 15 and a VL comprising an amino acid sequence that is at least 90%, 95%, 96%, 97%, 98%, 99% or 100% identical to the amino acid sequence of SEQ ID NO: 16.

In some embodiments, the bispecific antibody comprises a MUC1 binding portion comprising a VH comprising an amino acid sequence that is at least 90% identical to the amino acid sequence selected from the group of SEQ ID NO: 15 and a VL comprising an amino acid sequence that is at least 90% identical to the amino acid sequence of SEQ ID NO: 16.

In some embodiments, the bispecific antibody comprises a MUC1 binding portion comprising a VH comprising an amino acid sequence that is at least 95% identical to the amino acid sequence selected from the group of SEQ ID NO: 15 and a VL comprising an amino acid sequence that is at least 95% identical to the amino acid sequence of SEQ ID NO: 16.

In some embodiments, the bispecific antibody comprises a MUC1 binding portion comprising a VH comprising an amino acid sequence that is at least 99% identical to the amino acid sequence selected from the group of SEQ ID NO: 15 and a VL comprising an amino acid sequence that is at least 99% identical to the amino acid sequence of SEQ ID NO: 16.

In some embodiments, the bispecific antibody comprises a MUC1 binding portion comprising a VH of SEQ ID NO: 15, and a VL sequence of SEQ ID NO: 16.

In some embodiments, the above-described percentage identities to the MUC1 VH are in combination with 100% identity to the HCDR1 (SEQ ID NO: 7), HCDR2 (SEQ ID NO: 8), and HCDR3 (SEQ ID NO: 9) and the above-described percentage identities to the MUC1 VL are in combination with 100% identity to the LCDR1 (SEQ ID NO: 10), LCDR2 (SEQ ID NO: 11), and LCDR3 (SEQ ID NO: 12).

In some embodiments, the bispecific antibody comprises a MUC1 binding portion comprising
- a VH comprising an amino acid sequence with a percentage identity to an amino acid sequence of SEQ ID NO: 15 as described above, wherein said VH comprises a HCDR1 of SEQ ID NO: 7, a HCDR2 of SEQ ID NO: 8, and a HCDR3 of SEQ ID NO: 9; and
- a VL comprising an amino acid sequence with a percentage identity to an amino acid sequence of SEQ ID NO: 16 as described above, wherein said VL comprises a LCDR1 of SEQ ID NO: 10, a LCDR2 of SEQ ID NO: 11, and a LCDR3 of SEQ ID NO: 12.

In some embodiments, when the % identity is less than 100%, the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, a VH or VL sequence having at least 90%, 95%, 96%, 97%, 98%, or 99% identity contains substitutions (e.g., conservative substitutions), insertions, or deletions relative to the reference sequence, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, a VH or VL sequence having at least 90% contains substitutions (e.g., conservative substitutions), insertions, or deletions relative to the reference sequence, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, a VH or VL sequence having at least 95% contains substitutions (e.g., conservative substitutions), insertions, or deletions relative to the reference sequence, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In some embodiments, a VH or VL sequence having at least 99% contains substitutions (e.g., conservative substitutions), insertions, or deletions relative to the reference sequence, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In certain embodiments, a total of 1 to 10 amino acids have been substituted, inserted and/or deleted in SEQ ID NO: 15 and/or a total of 1 to 10 amino acids have been substituted, inserted and/or deleted in SEQ ID NO: 16, preferably wherein the binding affinity (K_{D}) of the bispecific antibody to MUC1 such as human MUC1 is not altered by more than 10%, preferably as measured by biolayer interferometry (BLI) or Surface Plasmon Resonance (SPR).

In certain embodiments, all substitutions, insertions, or deletions occur in regions outside the CDRs.

Optionally, the bispecific antibody comprises the VH sequence in SEQ ID NO: 15 and/or the VL sequence in SEQ ID NO: 16, including post-translational modifications of that sequence.

In some embodiments, the bispecific antibody comprises
- an EGFR binding portion comprising
   a VH comprising an amino acid sequence that is at least 90%, 95%, 96%, 97%, 98%, 99% or 100% identical to the amino acid sequence of SEQ ID NO: 13, and
   a VL comprising an amino acid sequence that is at least 90%, 95%, 96%, 97%, 98%, 99% or 100% identical to the amino acid sequence of SEQ ID NO: 14, and
- a MUC1 binding portion comprising
   a VH comprising an amino acid sequence that is at least 90%, 95%, 96%, 97%, 98%, 99% or 100% identical to the amino acid sequence of SEQ ID NO: 15, and
   a VL comprising an amino acid sequence that is at least 90%, 95%, 96%, 97%, 98%, 99% or 100% identical to the amino acid sequence of SEQ ID NO: 16.

In some embodiments, the bispecific antibody comprises
- an EGFR binding portion comprising
   a VH comprising an amino acid sequence that is at least 90% identical to the amino acid sequence of SEQ ID NO: 13, and
   a VL comprising an amino acid sequence that is at least 90% identical to the amino acid sequence of SEQ ID NO: 14, and
- a MUC1 binding portion comprising
   a VH comprising an amino acid sequence that is at least 90% identical to the amino acid sequence of SEQ ID NO: 15, and
   a VL comprising an amino acid sequence that is at least 90% identical to the amino acid sequence of SEQ ID NO: 16.

In some embodiments, the bispecific antibody comprises
- an EGFR binding portion comprising
   a VH comprising an amino acid sequence that is at least 95% identical to the amino acid sequence of SEQ ID NO: 13, and
   a VL comprising an amino acid sequence that is at least 95% identical to the amino acid sequence of SEQ ID NO: 14, and
- a MUC1 binding portion comprising
   a VH comprising an amino acid sequence that is at least 95% identical to the amino acid sequence of SEQ ID NO: 15, and
   a VL comprising an amino acid sequence that is at least 95% identical to the amino acid sequence of SEQ ID NO: 16.

In some embodiments, when the % identity is less than 100%, the binding affinity (K_{D}) of the bispecific antibody to EGFR and/or MUC1 such as human EGFR and/or MUC1 is not altered by more than 10%, preferably as measured by biolayer interferometry (BLI) or Surface Plasmon Resonance (SPR).

In some embodiments, the above-described percentage identities to the EGFR VH are in combination with 100% identity to the HCDR1 (SEQ ID NO: 1), HCDR2 (SEQ ID NO: 2), and HCDR3 (SEQ ID NO: 3) and the above-described percentage identities to the EGFR VL are in combination with 100% identity to the LCDR1 (SEQ ID NO: 4), LCDR2 (SEQ ID NO: 5), and LCDR3 (SEQ ID NO: 6). In some embodiments, the above-described percentage identities to the MUC1 VH are in combination with 100% identity to the HCDR1 (SEQ ID NO: 7), HCDR2 (SEQ ID NO: 8), and HCDR3 (SEQ ID NO: 9) and the above-described percentage identities to the MUC1 VL are in combination with 100% identity to the LCDR1 (SEQ ID NO: 10), LCDR2 (SEQ ID NO: 11), and LCDR3 (SEQ ID NO: 12).

In some embodiments, the bispecific antibody comprises an EGFR binding portion and a MUC1 binding portion,
wherein the EGFR binding portion comprises
   - a VH comprising an amino acid sequence with a percentage identity to an amino acid sequence of SEQ ID NO: 13 as described above, wherein said VH comprises a HCDR1 of SEQ ID NO: 1, a HCDR2 of SEQ ID NO: 2, and a HCDR3 of SEQ ID NO: 3; and
   - a VL comprising an amino acid sequence with a percentage identity to an amino acid sequence of SEQ ID NO: 14 as described above, wherein said VL comprises a LCDR1 of SEQ ID NO: 4, a LCDR2 of SEQ ID NO: 5, and a LCDR3 of SEQ ID NO: 6, and
wherein the MUC1 binding portion comprises,
   - a VH comprising an amino acid sequence with a percentage identity to an amino acid sequence of SEQ ID NO: 15 as described above, wherein said VH comprises a HCDR1 of SEQ ID NO: 7, a HCDR2 of SEQ ID NO: 8, and a HCDR3 of SEQ ID NO: 9; and
   - a VL comprising an amino acid sequence with a percentage identity to an amino acid sequence of SEQ ID NO: 16 as described above, wherein said VL comprises a LCDR1 of SEQ ID NO: 10, a LCDR2 of SEQ ID NO: 11, and a LCDR3 of SEQ ID NO: 12.

### Constant and Fc region of the bispecific antibody

This section describes the constant and Fc region of the bispecific antibody in the immunoconjugate. The embodiments in this section define the bispecific antibody both independent of the embodiments in the previous section and in combination with the embodiments in the previous section.

In some embodiments, the bispecific antibody comprises a human constant region. In some embodiments, the bispecific antibody comprises an Fc region. In some embodiments, the bispecific antibody comprises an Fc region that comprises two CH2 domains and two CH3 domains.

In some embodiments, the bispecific antibody comprises a heavy chain constant region comprising an Fc domain. In some embodiments, the bispecific antibody comprises a heavy chain constant region comprising an Fc domain comprising a single CH2 domain and a single CH3 domain. In some embodiments, the bispecific antibody comprises at least one Fc domain. In some embodiments, the bispecific antibody comprises two Fc domains.

In some embodiments, the bispecific antibody comprises an Fc region comprising two Fc domains, wherein one Fc domain comprises at least one knob substitution and the other Fc domain comprises at least one hole substitution. In some embodiments, one Fc domain comprises a T366W substitution, and the other Fc domain comprises **at least one** of T366S, L368A, and Y407V substitutions. In some embodiments, one Fc domain comprises a T366W substitution, and the second Fc domain comprises T366S, L368A, and Y407V substitutions. In some embodiments, one Fc domain comprises a S354C substitution, and the other Fc domain comprises a Y349C substitution. In some embodiments, one Fc domain comprises a T366W substitution and a S354C substitution, and the other Fc domain comprises a Y349C substitution and T366S, L368A, and Y407V substitutions.

In some embodiments, the bispecific antibody comprises an Fc region comprising two Fc domains, wherein at least one of the Fc domains comprises at least one of the following substitutions:
- a C226S substitution;
- a D356E and L358M substitution;
- L234A and L235A (LALA mutation).

In some embodiments, the bispecific antibody comprises an Fc region comprising two Fc domains, wherein at least one of the Fc domains comprises at least two of the following substitutions:
- a C226S substitution;
- a D356E and L358M substitution;
- L234A and L235A (LALA mutation).

In some embodiment, the bispecific antibody comprises an Fc region comprising two Fc domains, wherein at least one of the Fc domains comprises the following substitutions:
- a C226S substitution;
- a D356E and L358M substitution;
- L234A and L235A (LALA mutation).

In some embodiments, the bispecific antibody comprises an Fc region comprising two Fc domains, wherein both Fc domains comprise at least one of the following substitutions:
- a C226S substitution;
- a D356E and L358M substitution;
- L234A and L235A (LALA mutation).

In some embodiments, the bispecific antibody comprises an Fc region comprising two Fc domains, wherein both Fc domains comprise at least two of the following substitutions:
- a C226S substitution;
- a D356E and L358M substitution;
- L234A and L235A (LALA mutation).

In some embodiment, the bispecific antibody comprises an Fc region comprising two Fc domains, wherein both Fc domains comprise the following substitutions:
- a C226S substitution;
- a D356E and L358M substitution;
- L234A and L235A (LALA mutation).

In some embodiments, the bispecific antibody comprises an Fc region comprising two Fc domains, wherein at least one of the two Fc domains comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the bispecific antibody comprises an Fc region comprising two Fc domains, wherein both Fc domains comprise at least one of the following substitutions:
- L234A and L235A and P329G (LALA-PG mutation);
- L235A and G237A (LAGA mutation);
- M252Y and S254T and T256E (YTE mutation);
- K222R

In some embodiments, the bispecific antibody comprises:
- a first polypeptide comprising a first Fc domain and the VH and VL of the MUC1 binding portion,
- a second polypeptide comprising a second Fc domain and the VH of the EGFR binding portion.

In some embodiments, the bispecific antibody comprises:
- a first polypeptide comprising a first Fc domain and the VH and VL of the MUC1 binding portion as described in the previous section,
- a second polypeptide comprising a second Fc domain and the VH of the EGFR binding portion as described in the previous section.

In some embodiments, the first Fc domain comprises at least one knob substitution and the second Fc domain comprises at least one hole substitution. In some embodiments, the first Fc domain comprises a T366W substitution, and the second Fc domain comprises **at least one** of T366S, L368A, and Y407V substitutions. In some embodiments, the first Fc domain comprises a T366W substitution, and the second Fc domain comprises T366S, L368A, and Y407V substitutions. In some embodiments, the first Fc domain comprises a S354C substitution, and the second Fc domain comprises a Y349C substitution. In some embodiments, the first Fc domain comprises a T366W substitution and a S354C substitution, and the second Fc domain comprises a Y349C substitution and T366S, L368A, and Y407V substitutions.

In some embodiments, the first Fc domain comprises at least one hole substitution and the second Fc domain comprises at least one knob substitution. In some embodiments, the second Fc domain comprises a T366W substitution, and the first Fc domain comprises **at least one** of T366S, L368A, and Y407V substitutions. In some embodiments, the second Fc domain comprises a T366W substitution, and the first Fc domain comprises T366S, L368A, and Y407V substitutions. In some embodiments, the second Fc domain comprises a S354C substitution, and the first Fc domain comprises a Y349C substitution. In some embodiments, the second Fc domain comprises a T366W substitution and a S354C substitution, and the first Fc domain comprises a Y349C substitution and T366S, L368A, and Y407V substitutions.

In some embodiments, at least one of the Fc domains comprises at least one of the following substitutions:
- a C226S substitution;
- a D356E and L358M substitution;
- L234A and L235A (LALA mutation).

In some embodiments, at least one of the Fc domains comprises at least two of the following substitutions:
- a C226S substitution;
- a D356E and L358M substitution;
- L234A and L235A (LALA mutation).

In some embodiment, at least one of the Fc domains comprises the following substitutions:
- a C226S substitution;
- a D356E and L358M substitution;
- L234A and L235A (LALA mutation).

In some embodiments, the first and the second Fc domains comprise at least one of the following substitutions:
- a C226S substitution;
- a D356E and L358M substitution;
- L234A and L235A (LALA mutation).

In some embodiments, the first and the second Fc domains comprise at least two of the following substitutions:
- a C226S substitution;
- a D356E and L358M substitution;
- L234A and L235A (LALA mutation).

In some embodiment, the first and the second Fc domains comprise the following substitutions:
- a C226S substitution;
- a D356E and L358M substitution;
- L234A and L235A (LALA mutation).

In some embodiments, at least one of the first and the second Fc domains comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the first and the second Fc domains each comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the first polypeptide comprises at least one knob substitution and the second polypeptide comprises at least one hole substitution. In some embodiments, the first polypeptide comprises a T366W substitution, and the second polypeptide comprises **at least one** of T366S, L368A, and Y407V substitutions. In some embodiments, the first polypeptide comprises a T366W substitution, and the second polypeptide comprises T366S, L368A, and Y407V substitutions. In some embodiments, the first polypeptide comprises a S354C substitution, and the second polypeptide comprises a Y349C substitution. In some embodiments, the first polypeptide comprises a T366W substitution and a S354C substitution, and the second polypeptide comprises a Y349C substitution and T366S, L368A, and Y407V substitutions.

In some embodiments, the first polypeptide comprises at least one hole substitution and the second polypeptide comprises at least one knob substitution. In some embodiments, the second polypeptide comprises a T366W substitution, and the first polypeptide comprises **at least one** of T366S, L368A, and Y407V substitutions. In some embodiments, the second polypeptide comprises a T366W substitution, and the first polypeptide comprises T366S, L368A, and Y407V substitutions. In some embodiments, the second polypeptide comprises a S354C substitution, and the first polypeptide comprises a Y349C substitution. In some embodiments, the second polypeptide comprises a T366W substitution and a S354C substitution, and the first polypeptide comprises a Y349C substitution and T366S, L368A, and Y407V substitutions.

In some embodiments, at least one of the first and the second polypeptides comprises at least one of the following substitutions:
- a C226S substitution;
- a D356E and L358M substitution;
- L234A and L235A (LALA mutation).

In some embodiments, at least one of the first and the second polypeptides comprises at least two of the following substitutions:
- a C226S substitution;
- a D356E and L358M substitution;
- L234A and L235A (LALA mutation).

In some embodiment, at least one of the first and the second polypeptides comprises the following substitutions:
- a C226S substitution;
- a D356E and L358M substitution;
- L234A and L235A (LALA mutation).

In some embodiments, the first and the second polypeptides comprise at least one of the following substitutions:
- a C226S substitution;
- a D356E and L358M substitution;
- L234A and L235A (LALA mutation).

In some embodiments, the first and the second polypeptides comprise at least two of the following substitutions:
- a C226S substitution;
- a D356E and L358M substitution;
- L234A and L235A (LALA mutation).

In some embodiment, the first and the second polypeptides comprise the following substitutions:
- a C226S substitution;
- a D356E and L358M substitution;
- L234A and L235A (LALA mutation).

In some embodiments, at least one of the first and the second polypeptides comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the first and the second polypeptides each comprises at least one of the following substitutions:
- L234A and L235A and P329G (LALA-PG mutation);
- L235A and G237A (LAGA mutation);
- M252Y and S254T and T256E (YTE mutation);
- K222R.

Exemplary sequences of human constant domains are given in SEQ ID NO:20 and SEQ ID NO: 21 (human kappa and lambda CL domains, respectively) and SEQ ID NO: 22 (human IgG1 heavy chain constant domains CH1-CH2-CH3).

In some embodiments, the bispecific antibody comprises a light chain constant region comprising an amino acid sequence that is at least 95%, 96%, 97%, 98%, 99% or 100% identical to the amino acid sequence of SEQ ID NO: 20 or SEQ ID NO:21. In some embodiments, the bispecific antibody comprises a light chain constant region comprising an amino acid sequence that is at least 95%, 96%, 97%, 98%, 99% or 100% identical to the amino acid sequence of SEQ ID NO: 20.

In some embodiments, the bispecific antibody comprises
- a first polypeptide comprising a first Fc domain and a single-chain Fv fragment (scFv), wherein the scFv comprises the VH and VL of the MUC1 binding portion as described above,
- a second polypeptide comprising a second Fc domain and a heavy chain of a Fab fragment comprising the VH of the EGFR binding portion as described above, and
- a third polypeptide comprising a light chain of the Fab fragment comprising the VL of the EGFR binding portion as described above.

In some embodiments, the first polypeptide comprises at least one knob substitution and the second polypeptide comprises at least one hole substitution. In some embodiments, the first polypeptide comprises a T366W substitution, and the second polypeptide comprises **at least one** of T366S, L368A, and Y407V substitutions. In some embodiments, the first polypeptide comprises a T366W substitution, and the second polypeptide comprises T366S, L368A, and Y407V substitutions. In some embodiments, the first polypeptide comprises a S354C substitution, and the second polypeptide comprises a Y349C substitution. In some embodiments, the first polypeptide comprises a T366W substitution and a S354C substitution, and the second polypeptide comprises a Y349C substitution and T366S, L368A, and Y407V substitutions.

In some embodiments, the first polypeptide comprises at least one hole substitution and the second polypeptide comprises at least one knob substitution. In some embodiments, the second polypeptide comprises a T366W substitution, and the first polypeptide comprises **at least one** of T366S, L368A, and Y407V substitutions. In some embodiments, the second polypeptide comprises a T366W substitution, and the first polypeptide comprises T366S, L368A, and Y407V substitutions. In some embodiments, the second polypeptide comprises a S354C substitution, and the first polypeptide comprises a Y349C substitution. In some embodiments, the second polypeptide comprises a T366W substitution and a S354C substitution, and the first polypeptide comprises a Y349C substitution and T366S, L368A, and Y407V substitutions.

In some embodiments, at least one of the first and the second polypeptides comprises at least one of the following substitutions:
- a C226S substitution;
- a D356E and L358M substitution;
- L234A and L235A (LALA mutation).

In some embodiments, at least one of the first and the second polypeptides comprises at least two of the following substitutions:
- a C226S substitution;
- a D356E and L358M substitution;
- L234A and L235A (LALA mutation).

In some embodiment, at least one of the first and the second polypeptides comprises the following substitutions:
- a C226S substitution;
- a D356E and L358M substitution;
- L234A and L235A (LALA mutation).

In some embodiments, the first and the second polypeptides comprise at least one of the following substitutions:
- a C226S substitution;
- a D356E and L358M substitution;
- L234A and L235A (LALA mutation).

In some embodiments, the first and the second polypeptides comprise at least two of the following substitutions:
- a C226S substitution;
- a D356E and L358M substitution;
- L234A and L235A (LALA mutation).

In some embodiment, the first and the second polypeptides comprise the following substitutions:
- a C226S substitution;
- a D356E and L358M substitution;
- L234A and L235A (LALA mutation).

In some embodiments, at least one of the first and the second polypeptides comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the first and the second polypeptides each comprises at least one of the following substitutions:
- L234A and L235A and P329G (LALA-PG mutation);
- L235A and G237A (LAGA mutation);
- M252Y and S254T and T256E (YTE mutation);
- K222R.

In some embodiments, the third polypeptide comprises a light chain constant region comprising an amino acid sequence that is at least 95%, 96%, 97%, 98%, 99% or 100% identical to the amino acid sequence of SEQ ID NO: 20 or SEQ ID NO:21. In some embodiments, the third polypeptide that comprises a light chain constant region comprising an amino acid sequence that is at least 95%, 96%, 97%, 98%, 99% or 100% identical to the amino acid sequence of SEQ ID NO: 20. In some embodiments, the third polypeptide that comprises a light chain constant region comprising an amino acid sequence that is at least 95%, 96%, 97%, 98%, 99% or 100% identical to the amino acid sequence of SEQ ID NO: 20.

In some embodiments, the bispecific antibody comprises an Fc region, particularly an IgG Fc region, more particularly an IgG1 Fc region. In some embodiments, the bispecific antibody comprises a variant of an IgG1 Fc region. In one embodiment the Fc region is a human Fc region. In some embodiments, the bispecific antibody comprises an Fc region, particularly an IgG Fc region, more particularly an IgG1, IgG2, IgG3, or IgG4 Fc region. In some embodiments, the bispecific antibody comprises a variant of an IgG1, IgG2, IgG3, or IgG4 Fc region. In one embodiment the Fc region is a human Fc region.

### Types of Antibodies

This section describes the type of the bispecific antibody in the immunoconjugate. The embodiments in this section define the bispecific antibody both independent of the embodiments in the previous section and in combination with the embodiments in the previous section.

In some embodiments, the bispecific antibody comprises an antibody fragment selected from the group of an Fv molecule, a scFv molecule, a Fab molecule, and a F(ab')2 molecule; particularly a Fab molecule that comprises the EGFR VH and VL as described previously. In another embodiment, the bispecific antibody comprises an antibody fragment that is a diabody, a triabody or a tetrabody that comprises the EGFR VH and VL as described previously.

In some embodiments, the bispecific antibody comprises an antibody fragment selected from the group of an Fv molecule, a scFv molecule, a Fab molecule, and a F(ab')2 molecule; particularly a Fab molecule that comprises the MUC1 VH and VL as described previously. In another embodiment, the bispecific antibody comprises an antibody fragment that is a diabody, a triabody or a tetrabody that comprises the MUC1 VH and VL as described previously.

In some embodiments, the bispecific antibody comprises:
- a first polypeptide comprising a first Fc domain as described above and a single-chain Fv fragment (scFv), wherein the scFv comprises the VH and VL of the MUC1 binding portion as described above.

In some embodiments the scFV comprises a MUC1 binding portion comprising the VH and VL as described above preferably fused with a linker. In some embodiments, the linker is a GS linker.

In some embodiments, the first polypeptide comprises an amino acid sequence that is at least 80%, 85%, 90%, 95%, 96%, 97%, 98%, 99% or 100% identical to the amino acid sequence of SEQ ID NO: 17.

In some embodiments, the first polypeptide comprises an amino acid sequence that is at least 80% identical to the amino acid sequence of SEQ ID NO: 17.

In some embodiments, the first polypeptide comprises an amino acid sequence that is at least 85% identical to the amino acid sequence of SEQ ID NO: 17.

In some embodiments, the first polypeptide comprises an amino acid sequence that is at least 90% identical to the amino acid sequence of SEQ ID NO: 17.

In some embodiments, the first polypeptide comprises an amino acid sequence that is at least 95% identical to the amino acid sequence of SEQ ID NO: 17.

In some embodiments, the first polypeptide comprises an amino acid sequence that is at least 99% identical to the amino acid sequence of SEQ ID NO: 17.

In some embodiments, the first polypeptide comprises an amino acid sequence of SEQ ID NO: 17.

In some embodiments, the bispecific antibody comprises:
- a second polypeptide comprising a second Fc domain and a heavy chain of a Fab fragment comprising the VH of the EGFR binding portion as described above.

In some embodiments, the second polypeptide comprises an amino acid sequence that is at least 80%, 85%, 90%, 95%, 96%, 97%, 98%, 99% or 100% identical to the amino acid sequence of SEQ ID NO: 18.

In some embodiments, the second polypeptide comprises an amino acid sequence that is at least 80% identical to the amino acid sequence of SEQ ID NO: 18.

In some embodiments, the second polypeptide comprises an amino acid sequence that is at least 85% identical to the amino acid sequence of SEQ ID NO: 18.

In some embodiments, the second polypeptide comprises an amino acid sequence that is at least 90% identical to the amino acid sequence of SEQ ID NO: 18.

In some embodiments, the second polypeptide comprises an amino acid sequence that is at least 95% identical to the amino acid sequence of SEQ ID NO: 18.

In some embodiments, the second polypeptide comprises an amino acid sequence that is at least 99% identical to the amino acid sequence of SEQ ID NO: 18.

In some embodiments, the second polypeptide comprises an amino acid sequence of SEQ ID NO: 18.

In some embodiments, the bispecific antibody comprises:
- a third polypeptide comprising a light chain of the Fab fragment comprising the VL of the EGFR binding portion as described above.

In some embodiments, the third polypeptide comprises an amino acid sequence that is at least 80%, 85%, 90%, 95%, 96%, 97%, 98%, 99% or 100% identical to the amino acid sequence of SEQ ID NO: 19.

In some embodiments, the third polypeptide comprises an amino acid sequence that is at least 80% identical to the amino acid sequence of SEQ ID NO: 19.

In some embodiments, the third polypeptide comprises an amino acid sequence that is at least 85% identical to the amino acid sequence of SEQ ID NO: 19.

In some embodiments, the third polypeptide comprises an amino acid sequence that is at least 90% identical to the amino acid sequence of SEQ ID NO: 19.

In some embodiments, the third polypeptide comprises an amino acid sequence that is at least 95% identical to the amino acid sequence of SEQ ID NO: 19.

In some embodiments, the third polypeptide comprises an amino acid sequence that is at least 99% identical to the amino acid sequence of SEQ ID NO: 19.

In some embodiments, the third polypeptide comprises an amino acid sequence of SEQ ID NO: 19.

In some embodiments, the bispecific antibody comprises:
- a first polypeptide comprising a first Fc domain and a single-chain Fv fragment (scFv), wherein the scFv comprises the VH and VL of the MUC1 binding portion as described above.
- a second polypeptide comprising a second Fc domain and a heavy chain of a Fab fragment comprising the VH of the EGFR binding portion as described above, and
- a third polypeptide comprising a light chain of the Fab fragment comprising the VL of the EGFR binding portion as described above.

In some embodiments, the Fab fragment comprises the VH and the VL of the EGFR binding portion as described above. In some embodiments, the Fab fragment comprises a light chain constant region as described above.

In some embodiments, the first polypeptide comprises an amino acid sequence that is at least 80%, 85%, 90%, 95%, 96%, 97%, 98%, 99% or 100% identical to the amino acid sequence of SEQ ID NO: 17, the second polypeptide comprises an amino acid sequence that is at least 80%, 85%, 90%, 95%, 96%, 97%, 98%, 99% or 100% identical to the amino acid sequence of SEQ ID NO: 18, and the third polypeptide comprises an amino acid sequence that is at least 80%, 85%, 90%, 95%, 96%, 97%, 98%, 99% or 100% identical to the amino acid sequence of SEQ ID NO: 19.

In some embodiments, the first polypeptide comprises an amino acid sequence that is at least 80% identical to the amino acid sequence of SEQ ID NO: 17, the second polypeptide comprises an amino acid sequence that is at least 80% to the amino acid sequence of SEQ ID NO: 18, and the third polypeptide comprises an amino acid sequence that is at least 80% identical to the amino acid sequence of SEQ ID NO: 19.

In some embodiments, the first polypeptide comprises an amino acid sequence that is at least 85% identical to the amino acid sequence of SEQ ID NO: 17, the second polypeptide comprises an amino acid sequence that is at least 85% to the amino acid sequence of SEQ ID NO: 18, and the third polypeptide comprises an amino acid sequence that is at least 85% identical to the amino acid sequence of SEQ ID NO: 19.

In some embodiments, the first polypeptide comprises an amino acid sequence that is at least 90% identical to the amino acid sequence of SEQ ID NO: 17, the second polypeptide comprises an amino acid sequence that is at least 90% to the amino acid sequence of SEQ ID NO: 18, and the third polypeptide comprises an amino acid sequence that is at least 90% identical to the amino acid sequence of SEQ ID NO: 19.

In some embodiments, the first polypeptide comprises an amino acid sequence that is at least 95% identical to the amino acid sequence of SEQ ID NO: 17, the second polypeptide comprises an amino acid sequence that is at least 95% to the amino acid sequence of SEQ ID NO: 18, and the third polypeptide comprises an amino acid sequence that is at least 95% identical to the amino acid sequence of SEQ ID NO: 19.

In some embodiments, the first polypeptide comprises an amino acid sequence that is at least 99% identical to the amino acid sequence of SEQ ID NO: 17, the second polypeptide comprises an amino acid sequence that is at least 99% to the amino acid sequence of SEQ ID NO: 18, and the third polypeptide comprises an amino acid sequence that is at least 99% identical to the amino acid sequence of SEQ ID NO: 19.

In some embodiments, the first polypeptide comprises an amino acid sequence of SEQ ID NO: 17, the second polypeptide comprises an amino acid sequence of SEQ ID NO: 18, and the third polypeptide comprises an amino acid sequence of SEQ ID NO: 19.

### Glycosylation variants

This section describes the glycosylation variants of the anti-EGFR and MUC1 bispecific antibody in the immunoconjugate. The embodiments in this section define the bispecific antibody both independent of the embodiments in the previous section and in combination with the embodiments in the previous section.

In some embodiments, the bispecific antibody is glycosylated. In some embodiments, the bispecific antibody is altered to increase or decrease the extent to which the bispecific antibody is glycosylated. Addition or deletion of glycosylation sites to the bispecific antibody may be conveniently accomplished by altering the amino acid sequence such that one or more glycosylation sites is created or removed.

Where the bispecific antibody comprises an Fc region, the oligosaccharide attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in the bispecific antibody may be made in order to create antibody variants in the immunoconjugate with certain improved properties.

In some embodiments, the immunoconjugate comprises bispecific antibody variants having a non-fucosylated oligosaccharide, i.e. an oligosaccharide structure that lacks fucose attached (directly or indirectly) to an Fc region. Such non-fucosylated oligosaccharide (also referred to as "afucosylated" oligosaccharide) particularly is an N-linked oligosaccharide which lacks a fucose residue attached to the first GlcNAc in the stem of the biantennary oligosaccharide structure.

In some embodiments, the immunoconjugate comprises bispecific antibody variants having an increased proportion of non-fucosylated oligosaccharides in the Fc region as compared to a native or parent antibody. For example, the proportion of non-fucosylated oligosaccharides may be at least about 20%, at least about 40%, at least about 60%, at least about 80%, or even about 100% (i.e. no fucosylated oligosaccharides are present). The percentage of non-fucosylated oligosaccharides is the (average) amount of oligosaccharides lacking fucose residues, relative to the sum of all oligosaccharides attached to Asn 297 (e. g. complex, hybrid and high mannose structures) as measured by MALDI-TOF mass spectrometry, as described in WO 2006/082515, for example.

In a further embodiment, the immunoconjugate comprises bispecific antibody variants with bisected oligosaccharides, e.g., in which a biantennary oligosaccharide attached to the Fc region of the bispecific antibody is bisected by GlcNAc. Such antibody variants may have reduced fucosylation and/or improved ADCC function as described above. Examples of such antibody variants are described, e.g., in Umana et al., Nat Biotechnol 17, 176-180 (1999); Ferrara et al., Biotechn Bioeng 93, 851-861 (2006); WO 99/54342; WO 2004/065540, WO 2003/011878.

In some embodiments, the immunoconjugate comprises antibody variants with at least one galactose residue in the oligosaccharide attached to the Fc region. Such antibody variants may have improved CDC function. Such antibody variants are described, e.g., in WO 1997/30087; WO 1998/58964; and WO 1999/22764.

### Cysteine engineered antibody variants

This section describes the cysteine engineered variants of the anti-EGFR and MUC1 bispecific antibody in the immunoconjugate. The embodiments in this section define the bispecific antibody both independent of the embodiments in the previous section and in combination with the embodiments in the previous section.

In certain embodiments, it may be desirable to provide the immunoconjugate with cysteine engineered anti-EGFR and MUC1 bispecific antibody, e.g., "thioMAbs," in which one or more residues of the bispecific antibody are substituted with cysteine residues. In particular embodiments, the substituted residues occur at accessible sites of the bispecific antibody. By substituting those residues with cysteine, reactive thiol groups are thereby positioned at accessible sites of the bispecific antibody and may be used to conjugate the bispecific antibody to other moieties, such as drug moieties or linker-drug moieties, to create an immunoconjugate, as described further herein. Cysteine engineered antibodies may be generated as described, e.g., in U.S. Patent No. 7,521,541, 8,30,930, 7,855,275, 9,000,130, or WO2016040856.

### Multispecificity of the antibody

This section describes the specificity of the anti-EGFR and MUC1 bispecific antibody in the immunoconjugate. The embodiments in this section define the bispecific antibody both independent of the embodiments in the previous section and in combination with the embodiments in the previous section.

The EGFR and MUC1 antibody in the immunoconjugate is a bispecific antibody. Multispecific antibodies are monoclonal antibodies that have binding specificities for at least two different sites, i.e., different epitopes on different antigens or different epitopes on the same antigen. In certain embodiments, the bispecific antibody has three or more binding specificities. In certain embodiments, two of the binding specificities are for EGFR and MUC1 and the other (one or more) specificity is for any other antigen. In certain embodiments, bispecific antibodies may bind to two (or more) different epitopes of EGFR and/or MUC1. Multispecific (e.g., bispecific) antibodies may also be used to localize cytotoxic agents or cells to cells which express EGFR and MUC1 as is done with the immunoconjugate of the present invention. Multispecific antibodies can be prepared as full-length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)) and "knob-in-hole" engineering (see, e.g., U.S. Patent No. 5,731,168, and Atwell et al., J. Mol. Biol. 270:26 (1997)). Multi- specific antibodies may also be made by engineering electrostatic steering effects for making antibody Fc-heterodimeric molecules (see, e.g., WO 2009/089004); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5): 1547-1553 (1992) and WO 2011/034605); using the common light chain technology for circumventing the light chain mis-pairing problem (see, e.g., WO 98/50431); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol, 152:5368 (1994)); and preparing trispecific antibodies as described, e.g., in Tutt et al. J. Immunol. 147: 60 (1991).

Engineered antibodies with three or more antigen binding sites, including for example, "Octopus antibodies," or DVD-Ig are also included herein (see, e.g. WO 2001/77342 and WO 2008/024715). Other examples of multispecific antibodies with three or more antigen binding sites can be found in WO 2010/115589, WO 2010/112193, WO 2010/136172, WO2010/145792, and WO 2013/026831. The bispecific antibody or antigen binding fragment thereof also includes a "Dual Acting FAb" or "DAF" (see, e.g., US 2008/0069820 and WO 2015/095539).

Multi- specific antibodies may also be provided in an asymmetric form with a domain crossover in one or more binding arms of the same antigen specificity, i.e. by exchanging the VH/VL domains (see e.g., WO 2009/080252 and WO 2015/150447), the CH1/CL domains (see e.g., WO 2009/080253) or the complete Fab arms (see e.g., WO 2009/080251, WO 2016/016299, also see Schaefer et al, PNAS, 108 (2011) 1187-1191, and Klein at al., MAbs 8 (2016) 1010-20). Asymmetrical Fab arms can also be engineered by introducing charged or non-charged amino acid mutations into domain interfaces to direct correct Fab pairing. See e.g., WO 2016/172485. Various further molecular formats for multispecific antibodies are known in the art and are included herein (see e.g., Spiess et al., Mol Immunol 67 (2015) 95-106).

A particular type of multispecific antibodies, also included herein, are bispecific antibodies designed to simultaneously bind to a surface antigen on a target cell, e.g., a tumor cell, and to an activating, invariant component of the T cell receptor (TCR) complex, such as CD3, for retargeting of T cells to kill target cells.

Examples of bispecific antibody formats that may be useful for this purpose include, but are not limited to, the so-called "BiTE" (bispecific T cell engager) molecules wherein two scFv molecules are fused by a flexible linker (see, e.g., WO2004/ 10638 1 , WO2005/061547, WO2007/042261 , and WO2008/ 1 19567, Nagorsen and Bauerle, Exp Cell Res 3 17, 1255-1260 (2011)); diabodies (Holliger et al., Prot Eng 9, 299-305 (1996)) and derivatives thereof, such as tandem diabodies ("TandAb"; Kipriyanov et al., J Mol Biol 293, 41-56 (1999)); "DART" (dual affinity retargeting) molecules which are based on the diabody format but feature a C-terminal disulfide bridge for additional stabilization (Johnson et al., J Mol Biol 399, 436-449 (2010)), and so-called triomabs, which are whole hybrid mouse/rat IgG molecules (reviewed in Seimetz et al., Cancer Treat Rev 36, 458-467 (2010)). Particular T cell bispecific antibody formats included herein are described in WO 2013/026833, WO2013/026839, WO 2016/020309; Bacac et al., Oncoimmunology 5(8) (2016) eI203498.

In a further aspect, the immunoconjugate may incorporate any of the features, singly or in combination, as described in the sections below on other components of the immunoconjugate in addition to the above embodiments on the bispecific antibody of the immunoconjugate.

### Growth inhibiting agent of the immunoconjugate

This section is on the growth inhibiting agent of the immunoconjugate. The embodiments disclosed herein can be combined with the embodiments disclosed in the section of the anti-EGFR and MUC1 bispecific antibody of the immunoconjugate described in the previous section.

In some embodiments, an immunoconjugate is provided comprising:
(a) a bispecific antibody that binds to EGFR and MUC1,
(b) a growth inhibiting agent, and
(c) a linker covalently linking (a) and (b).

In some embodiments, the growth inhibiting agent is a cytotoxic drug (also referred to as a cytotoxic agent). In some embodiments, the growth inhibiting agent is a radioactive moiety.

In some embodiments, the growth inhibiting agent is a cytotoxic drug selected from a group consisting of a chemotherapeutic agent, enzyme, antibiotic, toxin such as small molecule toxin or enzymatically active toxin, toxoid, vinca, taxane, maytansinoid or maytansinoid analog, tomaymycin or pyrrolobenzodiazepine derivative, cryptophycin derivative, leptomycin derivative, microtubulin inhibitor such as auristatin or dolastatin analog, prodrug, topoisomerase I inhibitor, topoisomerase II inhibitor, DNA alkylating agents, anti-tubulin agent, CC-1065 and CC-1065 analog.

In some embodiments, the growth inhibiting agent is a camptothecin derivative.

In some embodiments, the growth inhibiting agent is a topoisomerase I inhibitor such as exatecan or deruxtecan.

Exatecan and deruxtecan are camptothecin derivatives. Camptothecin derivatives are a unique class of anticancer agents that have an inhibiting effect on topoisomerase I activity as their mechanism of action. Among camptothecin derivatives that have been evaluated clinically, irinotecan and topotecan are the most widely used drugs for which antineoplastic activities have been clinically confirmed. Irinotecan in particular has been demonstrated to be superior to conventional therapies that had been widely used for colorectal cancers and now plays an indispensable role in the standard therapy.

Exatecan is a novel synthetic camptothecin derivative with a unique hexacyclic structure. The compound is water soluble and does not require metabolic activation. Similar to other camptothecin derivatives, the closed lactone ring of exatecan undergoes pH-dependent hydrolysis to yield an open carboxylate form. An intact lactone ring was found to be necessary for topoisomerase I inhibition. Studies suggest that exatecan has more potent antitumor activity and less toxicity than other camptothecin derivatives.

In some embodiments, the growth inhibiting agent is exatecan.

In some embodiments, the growth inhibiting agent is deruxtecan.

Exatecan has the chemical name (1*S*,9*S*)-1-Amino-9-ethyl-5-fluoro-1,2,3,9,12,15-hexahydro-9-hydroxy-4-methyl-10*H*,13*H-*benzo(de)pyrano(3',4':6,7)indolizino(1,2-b)quinoline-10,13-dione. Exatecan is represented by the following structural formula:

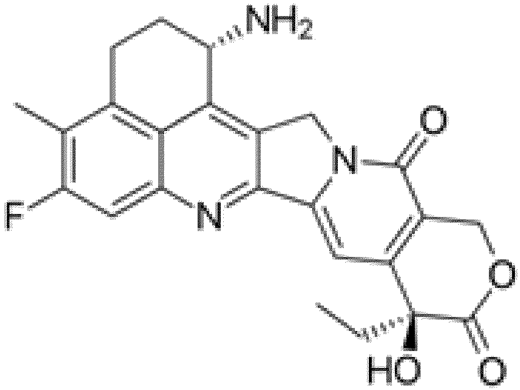

Deruxtecan has the chemical name 6-(2,5-dioxopyrrol-1-yl)-*N*-[2-[[2-[[(2*S*)-1-[[2-[[2-[[(10*S*,23*S*)-10-ethyl-18-fluoro-10-hydroxy-19-methyl-5,9-dioxo-8-oxa-4, 15-diazahexacyclo[14.7.1.0^{2,14}.0^{4,13}.0^{6,11}.0^{20,24}]tetracosa-1,6(11),12,14,16,18,20(24)-heptaen-23-yl]amino]-2-oxoethoxy]methylamino]-2-oxoethyl]amino]-1-oxo-3-phenylpropan-2-yl]amino]-2-oxoethyl]amino]-2-oxoethyl]hexanamide.

In further embodiments of the invention, other topoisomerase I inhibitors may be used. Examples of suitable topoisomerase I inhibitors include camptothecin (CPT) and analogs thereof such as topotecan, irinotecan, silatecan, cositecan, lurtotecan, gimatecan, belotecan and rubitecan. Examples of some cytotoxic drugs and of methods of conjugation are further given in the application WO2008/010101 which is incorporated by reference.

In a further aspect, the immunoconjugate may incorporate any of the features, singly or in combination, as described in the sections below on other components of the immunoconjugate in addition to the above embodiments on the growth inhibiting agent of the immunoconjugate.

### Linker and drug linker compound of the immunoconjugate

This section is on the Linker and drug linker compound of the immunoconjugate. The embodiments disclosed herein can be combined with the embodiments disclosed in the section of the anti-EGFR and MUC1 bispecific antibody of the immunoconjugate and the growth inhibitory agent of the immunoconjugate described in the previous sections.

In some embodiments, an immunoconjugate is provided comprising:
(a) a bispecific antibody that binds to EGFR and MUC1,
(b) a growth inhibiting agent, and
(c) a linker covalently linking (a) and (b).

The linkers connecting drug and antibody are designed to maximize systemic stability after parenteral application. The release of e.g., exatecan from the immunoconjugates of the invention within target cells leads to very high potency and outstanding bystander effects. A potent bystander effect may be beneficial for the treatment of patients with heterogeneous target expression.

In some embodiments, the bispecific antibody is covalently linked via a linker to the growth inhibiting agent. Linkers are well known in the art and include e.g. disulfide groups, thioether groups, acid labile groups, photolabile groups, peptidase labile groups and esterase labile groups. Conjugation of the bispecific antibody of the invention with cytotoxic drugs or other growth inhibiting agents may be performed e.g. using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1 - pyrrolidinyl ester (nitro-SPDB), 4-(Pyridin-2-yldisulfanyl)-2-sulfo-butyric acid (sulfo-SPDB), N-succinimidyl (2-pyridyldithio) propionate (SPDP), succinimidyl (N-maleimidomethyl) cyclohexane-1 - carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1 ,5-difluoro-2,4-dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to an antibody (WO 94/11026).

In some embodiments, the linker may be a "cleavable linker", which may facilitate release of the cytotoxic drug or other growth inhibiting agent inside of or in the vicinity of a cell, e.g. a tumor cell. In some embodiments, the linker is a linker cleavable in an endosome of a mammalian cell. For example, an acid-labile linker, a peptidase-sensitive linker, an esterase labile linker, a photolabile linker or a disulfide-containing linker may be used.

In some embodiments, the linker is a linker cleavable by the human enzyme glucuronidase. In some embodiments, the linker in the immunoconjugate of the invention is a β-glucuronide linker. In some embodiments, the linker is a linker cleavable by the human enzyme legumain. In some embodiments, the linker is a lysosomally cleavable maleimide-GGFG peptide linker.

In other embodiments, the linker may be a "non-cleavable linker" (for example an SMCC linker). Release of the growth inhibiting agent from the bispecific antibody can occur upon lysosomal degradation of the bispecific antibody.

In some embodiments, the linker may additionally comprise an H2N comprising moiety, such as a triple glycine moiety. In some embodiments, the linker may additionally comprise a maleimide moiety. In some embodiments, the linker may additionally comprise a triple glycine moiety.

When referring to a structural formula representing an immunoconjugate, the following nomenclature is also used herein: a growth inhibiting agent and a linker, taken together, are also referred to as a "drug linker compound" and represented by the formula: [(linker)- (growth inhibiting agent)]; for instance, an exatecan molecule and a linker, taken together, is referred to as a drug linker compound and represented by the formula: [(linker)-(exatecan)] moiety.

In some embodiments, the linker is β-glucuronide linker, and the growth inhibiting agent is exatecan resulting in a "β-glucuronide-exatecan", "drug linker compound" represented by the following **formula (I):**

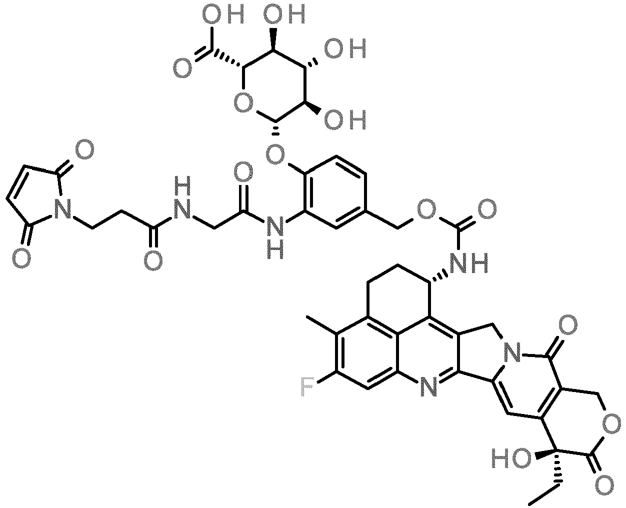

The drug linker compound shown in the **formula (I)** above comprises a maleimide moiety. In some embodiments, the drug linker compound shown in the **formula (I)** above is conjugated to interchain cysteines in the bispecific antibody. The resulting immunoconjugate can be represented by the following **formula (II):**

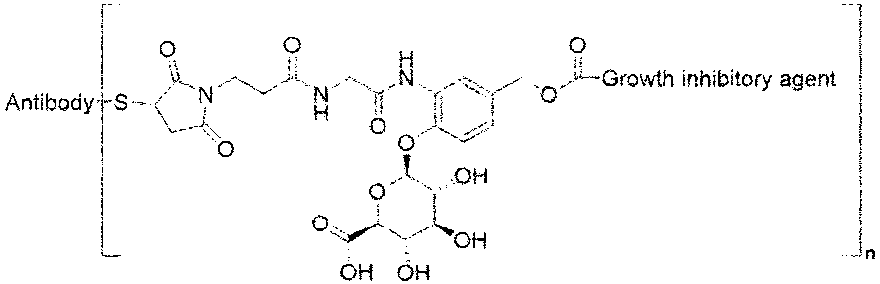

The number n is also referred to as "drug-to-antibody ratio" (or "DAR"); this number n is always to be understood as an average number for any given (preparation of an) immunoconjugate. The bispecific antibody is as described in the previous section titled "Anti-EGFR and MUC1 bispecific antibody of the immunoconjugate". In some embodiments, DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the linker is β-glucuronide linker, and the growth inhibiting agent is exatecan resulting in a "β-glucuronide-exatecan", "drug linker compound" represented by the following **formula (III):**

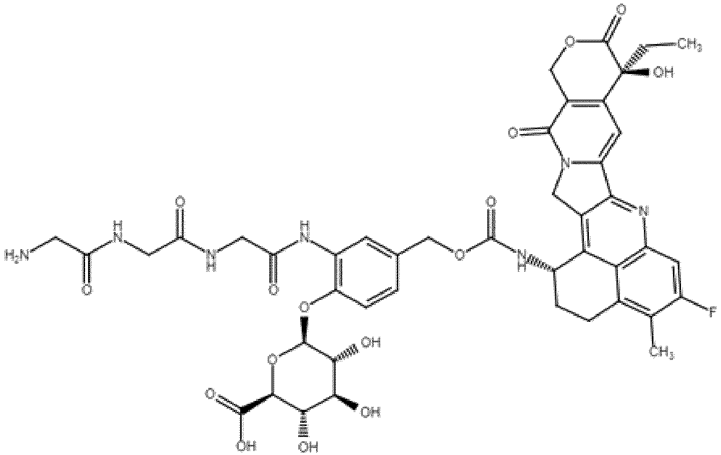

The drug linker compound shown in the **formula (III)** above comprises an H2N comprising moiety, which is a triple glycine moiety. In some embodiments, the drug linker compound shown in **formula (III)** above is conjugated to a glutamine (Q) in the bispecific antibody. The resulting immunoconjugate can be represented by the following **formula (IV):**

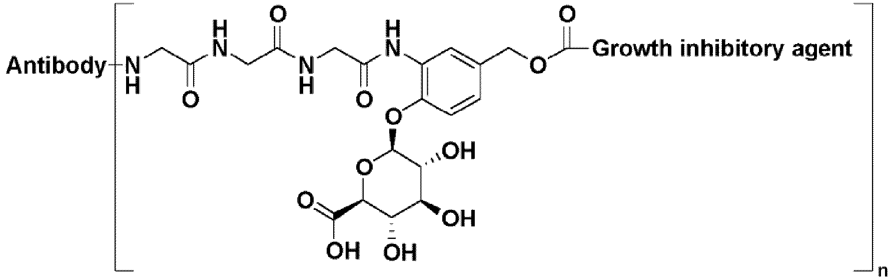

The number n is also referred to as "drug-to-antibody ratio" (or "DAR"); this number n is always to be understood as an average number for any given (preparation of an) immunoconjugate. The bispecific antibody is as described in the previous section titled "Anti-EGFR and MUC1 bispecific antibody of the immunoconjugate". In some embodiments, DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the linker is a is a linker cleavable by the human enzyme legumain, and the growth inhibiting agent is exatecan resulting in a "drug linker compound" represented by the following **formula (V):**

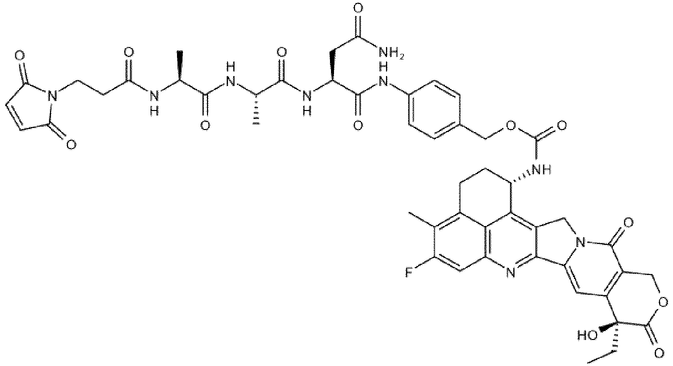

The drug linker compound shown in the **formula (V)** above comprises a maleimide moiety. In some embodiments, the drug linker compound shown in the **formula (V)** above is conjugated to interchain cysteines in the bispecific antibody. The resulting immunoconjugate can be represented by the following **formula (VI):**

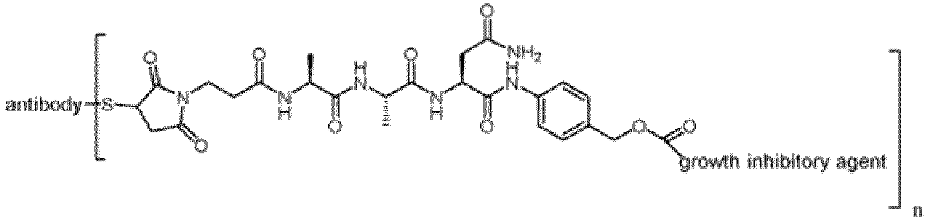

The number n is also referred to as "drug-to-antibody ratio" (or "DAR"); this number n is always to be understood as an average number for any given (preparation of an) immunoconjugate. The bispecific antibody is as described in the previous section titled "Anti-EGFR and MUC1 bispecific antibody of the immunoconjugate". In some embodiments, DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the linker is a lysosomally cleavable maleimide-GGFG peptide linker and the growth inhibiting agent is deruxtecan resulting in a maleimide-GGFG-deruxtecan "drug linker compound" represented by the following **formula (VII):**

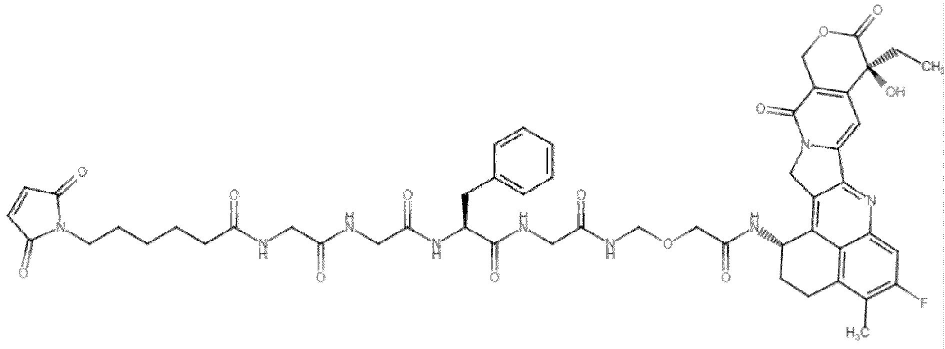

The drug linker compound shown in the **formula (VII)** above comprises a maleimide moiety. In some embodiments, the drug linker compound shown in the **formula (VII)** above is conjugated to interchain cysteines in the bispecific antibody.

In some embodiments, the linker is a lysosomally cleavable dipeptide, valine-citrulline (vc) and the growth inhibiting agent is monomethyl auristatin E (MMAE, a tubulin inhibitor) resulting in a vcMMAE "drug linker compound" represented by the following **formula (VIII):**

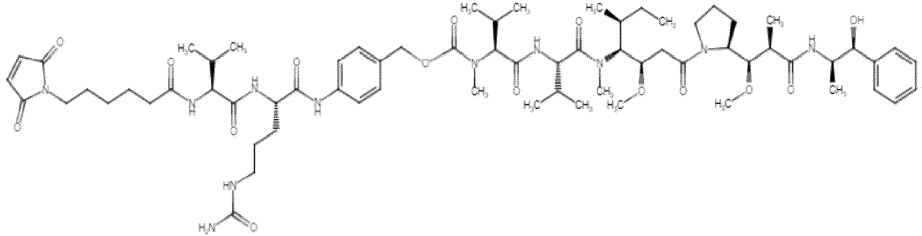

The drug linker compound shown in the **formula (VIII)** above comprises a maleimide moiety. In some embodiments, the drug linker compound shown in the **formula (VIII)** above is conjugated to interchain cysteines in the bispecific antibody. In some embodiments, DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In a further aspect, the immunoconjugate may incorporate any of the features, singly or in combination, as described in the sections below on other components of the immunoconjugate in addition to the above embodiments on the linker and drug linker compound of the immunoconjugate.

### Immunoconjugate

This section is on the immunoconjugate of the present invention. The embodiments disclosed herein can be combined with the embodiments disclosed in the section of the anti-EGFR and MUC1 bispecific antibody of the immunoconjugate, the growth inhibitory agent of the immunoconjugate and the linker and drug linker compound of the immunoconjugate described in the previous sections.

In some embodiments, an immunoconjugate is provided comprising:
(a) a bispecific antibody that binds to EGFR and MUC1,
(b) a growth inhibiting agent, and
(c) a linker covalently linking (a) and (b).

The present invention provides immunoconjugates, also referred to herein as antibody-drug conjugates (ADCs) or, more briefly, conjugates. As used herein, all these terms have the same meaning and are interchangeable.

In some embodiments, the bispecific antibody that binds to EGFR and MUC1 is as described in the section herein above titled "Anti-EGFR and MUC1 bispecific antibody of the immunoconjugate". In some embodiments, the growth inhibiting agent is as described in the section herein above titled "Growth inhibiting agent of the immunoconjugate". In some embodiments, the linker covalently linking the bispecific antibody and the growth inhibiting agent is as described in the section herein above titled "Linker and drug linker compound of the immunoconjugate".

In some embodiments, the immunoconjugate of the invention comprises a drug linker compound as described in the section "Linker and drug linker compound of the immunoconjugate". In some embodiments, the immunoconjugate of the invention comprises a drug linker compound comprising a topoisomerase inhibitor I as the growth inhibiting agent as described in the sections "Growth inhibiting agent of the immunoconjugate" and "Linker and drug linker compound of the immunoconjugate". In some embodiments, the immunoconjugate of the invention comprises a drug linker compound comprising exatecan as the growth inhibiting agent as described in the sections "Growth inhibiting agent of the immunoconjugate" and "Linker and drug linker compound of the immunoconjugate". In some embodiments, the immunoconjugate of the invention comprises a drug linker compound comprising exatecan as the growth inhibiting agent and a glucuronidase-cleavable linker as described in the sections "Growth inhibiting agent of the immunoconjugate" and "Linker and drug linker compound of the immunoconjugate". In some embodiments, the immunoconjugate of the invention comprises a drug linker compound comprising exatecan as the growth inhibiting agent and a β-glucuronide linker as described in the sections "Growth inhibiting agent of the immunoconjugate" and "Linker and drug linker compound of the immunoconjugate".

In some embodiments, an immunoconjugate is provided comprising:
(a) a bispecific antibody that binds to EGFR and MUC1 as described in the section titled "Anti-EGFR and MUC1 bispecific antibody of the immunoconjugate"
(b) a growth inhibiting agent, which is exatecan as described in the section titled "Growth inhibiting agent of the immunoconjugate" and
(c) a β-glucuronide linker covalently linking (a) and (b) as described in the section titled "Linker and drug linker compound of the immunoconjugate" above.

In some embodiments, the drug linker compound comprises a maleimide moiety and the linker is covalently attached to the bispecific antibody at a sulfur atom of a cysteine residue of the bispecific antibody.

For example, this cysteine residue of the bispecific antibody may be one of the cysteine residues capable of forming an interchain disulfide bond (also referred to herein as an interchain disulfide bridge). As there are four interchain disulfide bonds in an IgG1 antibody, involving a total of eight cysteine residues, attachment of the linker to the antibody at a sulfur atom of such cysteine residues provides that the DAR may be up to 8 and, in such cases, the DAR is typically between 7 and 8, such as between 7.5 and 8.0 (i.e. about 8), provided that the antibody is an IgG1 or has the same number of interchain disulfide bonds as an IgG1. In some embodiments, the interchain disulfide bonds have been reduced. In some embodiments, the interchain disulfide bonds have been reduced due to a C226S substitution. In some embodiments, the interchain disulfide bonds have been reduced due to a C226S substitution in both Fc domains of the bispecific antibody as described in the section of "anti-EGFR and MUC1 bispecific antibody of the immunoconjugate". In some embodiments, the interchain disulfide bonds have been reduced due to a C226S substitution in the first and the second polypeptides of the bispecific antibody as described in the section of "anti-EGFR and MUC1 bispecific antibody of the immunoconjugate".

In some embodiments, the bispecific antibody can comprise engineered cysteines e.g., "thioMAbs," in which one or more residues of the bispecific antibody are substituted with cysteine residues. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the drug linker compound comprises a maleimide moiety and the linker is covalently attached to the bispecific antibody at a sulfur atom of a cysteine residue of the bispecific antibody. In some embodiments, the drug linker compound comprises a maleimide moiety and the linker is covalently attached to the bispecific antibody at a sulfur atom of a cysteine residue of the bispecific antibody, wherein the cysteine is an interchain cysteine capable of forming a disulfide bond. In some embodiments, the drug linker compound comprises a maleimide moiety and the linker is covalently attached to the bispecific antibody at a sulfur atom of a cysteine residue of the bispecific antibody., wherein the cysteine is an interchain cysteine capable of forming a disulfide bond except the C226 cysteine. In some embodiments, the drug linker compound comprises a maleimide moiety and the linker is covalently attached to the bispecific antibody comprising a C226S at a sulfur atom of a cysteine residue of the bispecific antibody, wherein the cysteine is an interchain cysteine capable of forming a disulfide bond. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the drug linker compound comprises an H2N comprising moiety such as a triple-glycine moiety and the linker is covalently attached to the bispecific antibody at a glutamine (Q) residue of the bispecific antibody. In some embodiments, the glutamine (Q) is a substrate for a microbial transglutaminase. In some embodiments, the drug linker compound comprises exatecan as the growth inhibiting agent and a β-glucuronide linker. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

Throughout the present disclosure, a given molecule of a certain drug linker compound is only conjugated to one given residue of the antibody at a time. This means that a given molecule of a given drug linker compound cannot be conjugated simultaneously to more than one residue of the antibody. Of course, various individual molecules of the same drug linker compound can be conjugated to the antibody simultaneously at different residues of the antibody, but each individual drug linker molecule is conjugated to only one residue of the antibody at a time. Accordingly, if the present disclosure states that a drug linker compound of formula (II) is conjugated to a glutamine (Q) residue in a light chain of the antibody and to a glutamine (Q) residue in a heavy chain of the antibody, this is meant to designate that a first molecule of the drug linker compound of formula (II) is conjugated to a glutamine (Q) residue in a light chain of the antibody, and a second molecule of the drug linker compound of formula (II) is conjugated to another glutamine (Q) residue in a heavy chain of the antibody. However, the first molecule of the drug linker compound of formula (II) is not conjugated at the same time to a glutamine (Q) residue in a light chain and to a glutamine (Q) residue in a heavy chain of the antibody.

In some embodiments, the immunoconjugate comprises a drug-linker compound as described in section "Linker and drug linker compound of the immunoconjugate" that is conjugated to at least one light chain of the bispecific antibody via glutamine (Q) and at least one heavy chain of the bispecific antibody via glutamine (Q). In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to at least one light chain of the antibody via glutamine (Q) and the H2N comprising moiety of the drug linker compound and at least one heavy chain of the antibody via glutamine (Q) and the H2N comprising moiety of the drug linker compound. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to at least one light chain constant region (CL) of the bispecific antibody via glutamine (Q) and at least one heavy chain constant region (CH) of the bispecific antibody via glutamine (Q). In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to at least one light chain constant region (CL) of the antibody via glutamine (Q) and the H2N comprising moiety of the drug linker compound and at least one heavy chain constant region (CH) of the antibody via glutamine (Q) and the H2N comprising moiety of the drug linker compound. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to two light chain constant regions (CLs) of the bispecific antibody via glutamine (Q) in each CL and two heavy chain constant regions (CHs) of the bispecific antibody via glutamine (Q) in each CH. In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to two light chain constant regions (CLs) of the antibody via glutamine (Q) and the H2N comprising moiety of the drug linker compound in each CL and two heavy chain constant regions (CHs) of the antibody via glutamine (Q) and the H2N comprising moiety of the drug linker compound in each CH. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to at least one CL of the bispecific antibody via glutamine (Q) in the sequence selected from the group consisting of GGTLQSPP, LLQGA, GGLLQGPP, TLQSG, TLQSPP, and TLQSA, preferably wherein said sequence is at the C-terminus of said CL, and at least one CH of the bispecific antibody via glutamine (Q) at position 295 of said CH, wherein EU numbering is used for defining the position of said glutamine at position 295. In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to at least one CL of the antibody via glutamine (Q) in the sequence selected from the group consisting of GGTLQSPP, LLQGA, GGLLQGPP, TLQSG, TLQSPP, and TLQSA, and the H2N comprising moiety of the drug linker compound, preferably wherein said sequence is at the C-terminus of said CL, and at least one CH of the antibody via glutamine (Q) at position 295 of said CH and the H2N comprising moiety of the drug linker compound, wherein EU numbering is used for defining the position of said glutamine at position 295. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to two CLs of the bispecific antibody via glutamine (Q) in the sequence selected from the group consisting of GGTLQSPP, LLQGA, GGLLQGPP, TLQSG, TLQSPP, and TLQSA, preferably wherein said sequence is at the C-terminus of said CL, and two CHs of the bispecific antibody via glutamine (Q) at position 295 of said CH, wherein EU numbering is used for defining the position of said glutamine at position 295. In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to two CLs of the antibody via glutamine (Q) in the sequence selected from the group consisting of GGTLQSPP, LLQGA, GGLLQGPP, TLQSG, TLQSPP, and TLQSA, and the H2N comprising moiety of the drug linker compound, preferably wherein said sequence is at the C-terminus of said CLs, and two CHs of the antibody via glutamine (Q) at position 295 of said CHs and the H2N comprising moiety of the drug linker compound, wherein EU numbering is used for defining the position of said glutamine at position 295. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to at least one CL of the bispecific antibody via glutamine (Q) in the sequence GGTLQSPP, preferably wherein said sequence is at the C-terminus of said CL, and at least one CH of the bispecific antibody via glutamine (Q) at position 295 of said CH, wherein EU numbering is used for defining the position of said glutamine at position 295. In some embodiments, the glutamine (Q) is a substrate for a microbial transglutaminase. In some embodiments, the immunoconjugate comprises a drug-linker compound as described in section "Linker and drug linker compound of the immunoconjugate" that is conjugated to at least one CL of the antibody via glutamine (Q) in the sequence GGTLQSPP and the H2N comprising moiety of the drug linker compound, preferably wherein said sequence is at the C-terminus of said CL, and at least one CH of the antibody via glutamine (Q) at position 295 of said CH and the H2N comprising moiety of the drug linker compound, wherein EU numbering is used for defining the position of said glutamine at position 295. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate comprises a drug-linker compound as described in section "Linker and drug linker compound of the immunoconjugate" that is conjugated to two CLs of the bispecific antibody via glutamine (Q) in the sequence GGTLQSPP, preferably wherein said sequence is at the C-terminus of said CL, and two CHs of the bispecific antibody via glutamine (Q) at position 295 of said CH, wherein EU numbering is used for defining the position of said glutamine at position 295. In some embodiments, the glutamine (Q) is a substrate for a microbial transglutaminase. In some embodiments, the drug linker compound comprises exatecan as the growth inhibiting agent and a β-glucuronide linker preferably with an H2N comprising moiety such as a triple glycine moiety. In some embodiments, the DAR is from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In some embodiments, the immunoconjugate may be a fusion protein comprising the bispecific antibody of the invention and a cytotoxic or growth inhibiting polypeptide (as the growth inhibiting agent); such fusion proteins may be made by recombinant techniques or by peptide synthesis, i.e. methods well known in the art. A molecule of encoding DNA may comprise respective regions encoding the two portions of the conjugate (antibody and cytotoxic or growth inhibiting polypeptide, respectively) either adjacent to one another or separated by a region encoding a linker peptide.

### Methods of preparing immunoconjugates

This section is on the methods of preparing the immunoconjugate of the present invention. The embodiments disclosed herein can be combined with the embodiments disclosed in the section of the anti-EGFR and MUC1 bispecific antibody of the immunoconjugate, the growth inhibiting agent of the immunoconjugate, the linker and drug linker compound of the immunoconjugate, and the immunoconjugate described in the previous sections.

In some embodiments, the immunoconjugate of the present invention can be obtained by a process comprising the steps of:
(i) preparing a compound comprising the linker and the growth inhibiting agent also referred to herein as a "drug-linker compound" as described in section "Linker and drug linker compound of the immunoconjugate";
(ii) providing an anti-EGFR and MUC1 bispecific antibody as described in section "anti-EGFR and MUC1 bispecific antibody of the immunoconjugate";
(iii) bringing into contact the anti-EGFR and MUC1 bispecific antibody with the drug-linker compound in a suitable reaction buffer,
(iv) optionally separating the conjugate which was formed in (iii) from the unreacted bispecific antibody and/or drug-linker compound.

Suitable methods for preparing an immunoconjugate of the invention are well known in the art (see e.g. Hermanson G. T., Bioconjugate Techniques, Third Edition, 2013, Academic Press). For instance, methods of conjugating a cytotoxic drug to an antibody via a linker that attaches covalently to cysteine residues of interchain disulfide bridges of the antibody are well known. For conjugation to the cysteine residues of an antibody, the antibody is subjected to reduction (e.g. using TCEP) before step (iii). Suitable reduction conditions to reduce only the interchain disulfide bonds are known in the art.

In some embodiments, the drug-linker compound is represented by **formula (I).**

Furthermore, methods of conjugating a cytotoxic drug to an antibody via a linker that attaches covalently to glutamine residues via a microbial transglutaminase (MTG), so called "MTG conjugation" are also known e.g., as described in WO2023170239.

In some embodiments, a method of preparing an immunoconjugate of the present invention is provided comprising the following steps:
(i) preparing a compound comprising the linker and the growth inhibiting agent (e.g. cytotoxic drug), also referred to herein as a "drug-linker compound" as described in section "Linker and drug linker compound of the immunoconjugate";
(ii) providing an anti-EGFR and MUC1 bispecific antibody as described in section "anti-EGFR and MUC1 bispecific antibody of the immunoconjugate";
(iii) bringing into contact the anti-EGFR and MUC1 bispecific antibody with the drug-linker compound in a suitable reaction buffer comprising a microbial transglutaminase,
(iv) optionally separating the conjugate which was formed in (iii) from the unreacted bispecific antibody and/or drug-linker compound.

In some embodiments, the microbial transglutaminase is selected from a group consisting of SEQ ID NO: 25, 26, 27, 28, 29, 30, 31, 32, 33, 34, 52, 53, and 54. In some embodiments, the suitable reaction buffer is a reaction buffer that is between 0% and 7 % DMSO, between 20 and 30 mM HEPES (preferably 24 mM HEPES) and pH of between 6.8 and 7.4 (preferably pH 7); or a reaction buffer that comprises between 1% and 10% DMSO, between 5 and 100 mM HEPES, and comprises less than 150 mM NaCl and has a pH of between 6 and 8; or a reaction buffer that comprises between 1% and 10% DMSO, between 5 and 60 mM HEPES, and comprises less than 10 mM NaCl (preferably no NaCl) and has a pH of between 6 and 7.5; or a reaction buffer that comprises between 5 and 100 mM HEPES, and comprises less than 150 mM NaCl and has a pH of between 6 and 8; or a reaction buffer that comprises between 5 and 60 mM HEPES, and comprises less than 10 mM NaCl (preferably no NaCl) and has a pH of between 6 and 7.5.

The reaction temperature for conjugation is usually between 20 and 40°C. The reaction time can vary and is typically from 1 to 24 hours. The reaction between the bispecific antibody and the drug linker compound can be monitored by size exclusion chromatography (SEC) with a refractometric and/or UV detector. If the conjugate yield is too low, the reaction time can be extended.

A number of different chromatography methods can be used by the person skilled in the art in order to perform the separation of step (iii): the conjugate can be purified e.g. by SEC, adsorption chromatography (such as ion exchange chromatography, I EC), hydrophobic interaction chromatography (HIC), affinity chromatography, mixed-support chromatography such as hydroxyapatite chromatography, or high performance liquid chromatography (HPLC) such as reverse-phase HPLC. Purification by dialysis or filtration or diafiltration can also be used.

After step (iii) and/or (iv), the conjugate-containing solution can be subjected to an additional step (iv) of purification e.g. by chromatography, ultrafiltration and/or diafiltration. Such an additional step of purification e.g. by chromatography, ultrafiltration and/or diafiltration can also be performed with the bispecific antibody-containing solution after the reduction reaction, in cases where reduction is performed prior to conjugation.

The conjugate is recovered at the end of such a process in an aqueous solution. The drug-to-antibody ratio (DAR) is a number that can vary with the nature of the antibody and of the druglinker compound used along with the experimental conditions used for the conjugation (such as the ratio (drug-linker compound)/(antibody), the reaction time, the nature of the solvent and of the cosolvent if any). Thus, the contact between the antibody and the drug-linker compound can lead to a mixture comprising several conjugates differing from one another by different drug-to-antibody ratios. The DAR that is determined is thus an average value.

An exemplary method which can be used to determine the DAR consists of measuring spectrophotometrically the ratio of the absorbance at of a solution of purified conjugate at AD and 280 nm. 280 nm is a wavelength generally used for measuring protein concentration, such as antibody concentration. The wavelength D is selected so as to allow discriminating the drug from the antibody, i.e. as readily known to the skilled person, AD is a wavelength at which the drug has a high absorbance and AD is sufficiently remote from 280 nm to avoid substantial overlap in the absorbance peaks of the drug and antibody. For instance, AD may be selected as being 370 nm for exatecan (or for camptothecin or other camptothecin analogs), or 252 nm for maytansinoid molecules.

A method of DAR calculation may be derived e.g. from Antony S. Dimitrov (ed), LLC, 2009, Therapeutic Antibodies and Protocols, vol 525, 445, Springer Science: The absorbances for the conjugate at AD (AAD) and at 280 nm (A280) are measured either on the monomeric peak of the size exclusion chromatography (SEC) analysis (allowing to calculate the "DAR(SEC)" parameter) or using a classic spectrophotometer apparatus (allowing to calculate the "DAR(UV)" parameter). The absorbances can be expressed as follows: ${A}_{280} = \left({C}_{D} X {ε}_{D 280}\right) + \left({C}_{A} X {ε}_{A 280}\right)$ wherein :
C_{D} and C_{A} are respectively the concentrations in the solution of the drug and of the antibody ε_{DλD} and ε_{D280} are respectively the molar extinction coefficients of the drug at λ_{D} and 280 nm ε_{AλD} and ε_{A280} are respectively the molar extinction coefficients of the antibody at λ_{D} and 280 nm.

Resolution of these two equations with two unknowns leads to the following equations: ${C}_{D} = \left[\left({ε}_{A 280} X {A}_{λD}\right)-\left({ε}_{AλD} X {A}_{280}\right)\right] / \left[\left({ε}_{DλD} X {ε}_{A 280}\right)\right]$ $CA = \left[A280-\left(CD X εD280\right)\right] / εA 280$

The average DAR is then calculated from the ratio of the drug concentration to that of the antibody: DAR = CD / CA.

Exemplary methods for preparing an immunoconjugate of the invention are described in the Examples.

### Pharmaceutical compositions

The immunoconjugates of the invention described in section titled "immunoconjugate", above, may be combined with pharmaceutically acceptable carriers, diluents and/or excipients, and optionally with sustained-release matrices including but not limited to the classes of biodegradable polymers, non-biodegradable polymers, lipids or sugars, to form pharmaceutical compositions.

Thus, another aspect of the invention relates to a pharmaceutical composition comprising an effective amount of the immunoconjugate of the invention and a pharmaceutically acceptable carrier, diluent and/or excipient.

Examples of suitable carriers, diluents and/or excipients include, but are not limited to, one or more of water, amino acids, saline, phosphate buffered saline, buffer phosphate, acetate, citrate, succinate; amino acids and derivates such as histidine, arginine, glycine, proline, glycylglycine; inorganic salts such as NaCI or calcium chloride; sugars or polyalcohols such as dextrose, glycerol, ethanol, sucrose, trehalose, mannitol; surfactants such as polysorbate 80, polysorbate 20, poloxamer 188; and the like, as well as combination thereof. In many cases, it will be useful to include isotonic agents, such as sugars, polyalcohols, or sodium chloride in a pharmaceutical composition, and the formulation may also contain an antioxidant such as tryptamine and/or a stabilizing agent such as Tween 20.

The form of the pharmaceutical compositions, the route of administration, the dosage and the regimen naturally depend upon the condition to be treated, the severity of the illness, the age, weight, and gender of the patient, etc.

The pharmaceutical compositions of the invention can be formulated for a topical, oral, parenteral, intranasal, intravenous, intramuscular, subcutaneous or intraocular administration and the like.

In an embodiment, the pharmaceutical compositions contain vehicles which are pharmaceutically acceptable for a formulation for injection. These may be isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical composition can be administrated through drug combination devices.

The doses used for the administration can be adapted as a function of various parameters, and for instance as a function of the mode of administration used, of the relevant pathology, or alternatively of the desired duration of treatment.

To prepare pharmaceutical compositions, the bispecific antibody or immunoconjugate of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions; in all such cases, the form must be sterile and injectable with the appropriate device or system for delivery without degradation, and it must be stable under the conditions of manufacture and storage and must be preserved against the contaminating action of microorganisms, such as bacteria and fungi.

Solutions of active compounds as free base or pharmacologically acceptable salts can be prepared in water suitably mixed with a surfactant. Dispersions can also be prepared in glycerol, liquid polyethylene glycols, and mixtures thereof and in oils. Under ordinary conditions of storage and use, these preparations may contain a preservative to prevent the growth of microorganisms.

An immunoconjugate of the invention can be formulated into a pharmaceutical composition in a neutral or salt form. Pharmaceutically acceptable salts include the acid addition salts (formed with the free amino groups of the protein) which are formed with inorganic acids such as, for example, hydrochloric or phosphoric acids, or such organic acids as acetic, oxalic, tartaric, or mandelic acid, and the like. Salts formed with the free carboxyl groups can also be derived from inorganic bases such as, for example, sodium, potassium, ammonium, calcium, or ferric hydroxides, and such organic bases as isopropylamine, trimethylamine, glycine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In some cases, it may be desirable to include isotonic agents, for example, sugars or sodium chloride.

Prolonged absorption of the injectable compositions can be brought about by the use in the compositions of agents delaying absorption, for example, aluminum monostearate and gelatin.

Sterile injectable solutions can be prepared by incorporating the active compounds in the required amount in the appropriate solvent with any of the other ingredients enumerated above, as required, followed by filtered sterilization. Generally, dispersions can be prepared by incorporating the various sterilized active ingredients into a sterile vehicle which contains the basic dispersion medium and the required other ingredients from those enumerated above. In the case of sterile powders for the preparation of sterile injectable solutions, methods of preparation include vacuum-drying and freeze-drying techniques which yield a powder of the active ingredient plus any additional desired ingredient from a previously sterile-filtered solution thereof.

The preparation of more concentrated, or highly concentrated solutions for direct injection is also contemplated, where the use of DMSO as solvent is envisioned to result in extremely rapid penetration, delivering high concentrations of the active agents to a small tumor area.

Upon formulation, solutions can be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution can be suitably buffered if necessary and the liquid diluent first rendered isotonic with sufficient saline or glucose. These aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCI solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The immunoconjugate of the invention may be formulated within a therapeutic mixture to comprise e.g. about 0.01 to 100 milligrams per dose or so.

In addition to the immunoconjugate formulated for parenteral administration, such as intravenous or intramuscular injection, other pharmaceutically acceptable forms include e.g. tablets or other solids for oral administration, time release capsules, and any other form currently used.

In some embodiments, the use of liposomes and/or nanoparticles is contemplated for the introduction of polypeptides into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 pm) are generally designed using polymers able to be degraded in vivo. Biodegradable polyal kyl-cyanoacrylate nanoparticles, or biodegradable polylactide or polylactide coglycolide nanoparticles that meet these requirements are contemplated for use in the present invention, and such particles may be easily made by those of skill in the art.

Liposomes can be formed from phospholipids that are dispersed in an aqueous medium and spontaneously form multilamellar concentric bilayer vesicles (also termed multilamellar vesicles (MLVs)). MLVs generally have diameters of from 25 nm to 4 pm. Sonication of MLVs results in the formation of small unilamellar vesicles (SLIVs) with diameters in the range of 200 to 500 A, containing an aqueous solution in the core. The physical characteristics of liposomes depend on pH, ionic strength and the presence of divalent cations.

Besides the above-mentioned examples, further pharmaceutical forms such as nanoparticles, microparticles and -capsules, implants (e.g. lipid implants), or self-solidifying or - emulsifying systems, are also contemplated.

### Medical use and methods of treatment of the immunoconjugate

This section is on the medical use and methods of treatment of the immunoconjugate of the present invention. The embodiments disclosed herein can be combined with the embodiments disclosed in the section of the anti-EGFR and MUC1 bispecific antibody of the immunoconjugate, the growth inhibiting agent of the immunoconjugate, the linker and drug linker compound of the immunoconjugate, and the immunoconjugate described in the previous sections.

The inventors have found that the immunoconjugates of the invention induces a marked anti-tumour activity in vivo e.g. in murine xenograft models derived from a patient (PDX) or cell-line (CDX). The immunoconjugate and pharmaceutical compositions of the invention may thus be useful for treating cancers expressing EGFR and/or MUC1. The inventors have surprisingly found that the immunoconjugate of the present invention induces marked anti-tumour activity while minimizing toxicities. The inventors have surprisingly found that the immunoconjugate of the present invention induces marked anti-tumour activity while minimizing toxicities such as dermatological toxicities. This is even more surprising because the immunoconjugate of the invention comprises an anti-EGFR and MUC1 bispecific antibody in which the EGFR binding portion comprising the VH and VL is not attenuated, i.e., the EGFR binding portion binds to e.g., human EGFR with a Kd of as low as less than 10 nM. This is even more surprising because the immunoconjugate of the invention comprises an anti-EGFR and MUC1 bispecific antibody in which the EGFR binding portion binds to e.g., human EGFR with a Kd of as low as less than 5 nM. This is even more surprising because the immunoconjugate of the invention comprises an anti-EGFR and MUC1 bispecific antibody in which the EGFR binding portion binds to e.g., human EGFR with a Kd of as low as from 1.5 to 2 nM.

Accordingly, the present invention provides the immunoconjugate or pharmaceutical composition of the invention for use as a medicament. For instance, the invention provides the immunoconjugate or pharmaceutical composition of the invention for use in the treatment of cancer. The invention further provides a method of treating cancer, comprising administering the immunoconjugate or pharmaceutical composition of the invention to a subject in need thereof.

The cancer to be treated with immunoconjugates, or pharmaceutical compositions of the invention is preferably a cancer expressing EGFR and/or MUC1. The cancer to be treated with immunoconjugates, or pharmaceutical compositions of the invention is preferably a cancer expressing EGFR. The cancer to be treated with immunoconjugates, or pharmaceutical compositions of the invention is preferably a cancer expressing MUC1. The cancer to be treated with immunoconjugates, or pharmaceutical compositions of the invention is preferably a cancer expressing EGFR and MUC1. Expression of EGFR and/or MUC1 by cells may be readily assayed for instance by using an antibody against EGFR and/or MUC1.

In some embodiments, the cancer to be treated with immunoconjugates, or pharmaceutical compositions of the invention is selected from the group consisting of biliary tract cancer, oesophageal squamous cell carcinoma (ESCC), ovarian cancer such as epithelial ovarian cancer, non-small cell lung cancer (NSCLC), bladder cancer, and SCCHN (squamous cell carcinoma of head and neck (SCCHN).

In some embodiments, the cancer is non-small cell lung cancer.

In some embodiments, the cancer is ovarian cancer such as epithelial ovarian cancer.

In some embodiments, the cancer is squamous cell carcinoma of head and neck.

In the immunoconjugate, the anti-EGFR and MUC1 bispecific antibody as described in the section "anti-EGFR and MUC1 bispecific antibody of the immunoconjugate" is conjugated (linked) to a growth inhibiting agent as described in the section "growth inhibiting agent of the immunoconjugate". The bispecific antibody within the immunoconjugate of the invention may thus be useful for targeting said growth inhibiting agent to cancerous cells expressing EGFR and MUC1 on their surface.

In some embodiments, the immunoconjugate of the invention comprises an anti-EGFR and MUC1 bispecific antibody as described in the section "anti-EGFR and MUC1 bispecific antibody of the immunoconjugate", a growth inhibiting agent as described in the section "growth inhibiting agent of the immunoconjugate" and a linker as described in the section "linkers and drug linker compounds of the immunoconjugate" linking the EGFR and MUC1 bispecific antibody and the growth inhibiting agent. In some embodiments, the growth inhibiting agent is a topoisomerase I inhibitor, exatecan. In some embodiments, the linker is a linker cleavable by a human enzyme glucuronidase such as a β-glucuronide linker. In some embodiments, the immunoconjugate of the invention comprises a drug linker compound comprising exatecan as the growth inhibiting agent and a β-glucuronide linker as described in the section "Growth inhibiting agent of the immunoconjugate". In some embodiments, the drug linker compound is conjugated to the bispecific antibody at a DAR from 1 to 10. In some embodiments, DAR is from 2 to 8. In some embodiments DAR is below 8. In some embodiments, DAR is below 5. In some embodiments, DAR is from about 2 to about 4. In this context "about 2" refers to a value 2±0.5, and therefore, 1.5 to 2.5. In this context "about 4" refers to a value 4±0.5, and therefore, 3.5 to 4.5. In some embodiments, DAR is about 4. In some embodiments, DAR is from 3.2 to 4.8. In some embodiments, DAR is from 3.5 to 4.5. In some embodiments, DAR is from 3.5 to 4.2.

In preferred embodiments, the subject is human.

### Use of the immunoconjugate for the manufacture of a medicament

In some embodiments, use of the immunoconjugate for the manufacture of a medicament for the treatment of cancer in a subject is provided.

In some embodiments, use of the immunoconjugate for the manufacture of a medicament for the treatment of cancer in a subject is provided comprising administering the immunoconjugate or a pharmaceutical composition of the immunoconjugate to the subject.

In some embodiments, use of the immunoconjugate for the manufacture of a medicament for reducing tumour volume in a subject having cancer is provided. In some embodiments, use of the immunoconjugate for the manufacture of a medicament for reducing tumour volume in a subject having cancer is provided comprising administering the immunoconjugate or a pharmaceutical composition of the immunoconjugate to the subject.

In some embodiments, use of the immunoconjugate for the manufacture of a medicament for delaying tumour progression in a subject having cancer is provided. In some embodiments, use of the immunoconjugate for the manufacture of a medicament for delaying tumour progression in a subject having cancer is provided comprising administering the immunoconjugate or a pharmaceutical composition of the immunoconjugate to the subject.

In some embodiments, use of the immunoconjugate for the manufacture of a medicament for lowering the risk of toxicities when treating cancer is provided. In some embodiments, use of the immunoconjugate for the manufacture of a medicament for lowering the risk of toxicities when treating cancer is provided comprising administering the immunoconjugate or a pharmaceutical composition of the immunoconjugate to the subject.

### Method of treating cancer

In some embodiments, a method of treating cancer in a subject is provided comprising administering an effective amount of the immunoconjugate or a pharmaceutical composition comprising an effective amount of the immunoconjugate to the subject.

In some embodiments, a method of reducing tumour volume in a subject having cancer is provided comprising administering an effective amount of the immunoconjugate or a pharmaceutical composition comprising an effective amount of the immunoconjugate to the subject.

In some embodiments, a method of delaying tumour progression in a subject having cancer is provided comprising administering an effective amount of the immunoconjugate or a pharmaceutical composition comprising an effective amount of the immunoconjugate to the subject to the subject.

In some embodiments, a method of lowering the risk of toxicities when treating cancer in a subject is provided comprising administering an effective amount of the immunoconjugate or a pharmaceutical composition comprising an effective amount of the immunoconjugate to the subject.

### Immunoconjugate for use as a medicament or for use in treating cancer

In some embodiments, the immunoconjugate is for use as a medicament.

In some embodiments, the immunoconjugate is for use in the treatment of cancer in a subject. In some embodiments, the immunoconjugate is for use in the treatment of cancer in a subject comprising administering the immunoconjugate or a pharmaceutical composition of the immunoconjugate to the subject.

In some embodiments, the immunoconjugate is for use in reducing tumour volume in a subject having cancer. In some embodiments, the immunoconjugate is for use in reducing tumour volume in a subject having cancer comprising administering the immunoconjugate or a pharmaceutical composition comprising the immunoconjugate to the subject.

In some embodiments, the immunoconjugate is for use in delaying tumour progression in a subject having cancer. In some embodiments, the immunoconjugate is for use in delaying tumour progression in a subject having cancer comprising administering the immunoconjugate or a pharmaceutical composition comprising the immunoconjugate to the subject.

In some embodiments, the immunoconjugate is for use in lowering the risk of toxicities when treating cancer in a subject. In some embodiments, the immunoconjugate is for use in lowering the risk of toxicities when treating cancer in a subject comprising administering the immunoconjugate or a pharmaceutical composition comprising the immunoconjugate to the subject.

### Selected advantages of the medical use and methods of treatment

In this section, selected advantages of the immunoconjugate are provided that are achieved in the use of the immunoconjugate in the manufacture of a medicament, the immunoconjugate for use in treating cancer, or the methods of treating cancer.

In some embodiments, the tumour volume is reduced as compared to the tumour volume in a subject that is administered the bispecific antibody alone.

In some embodiments, the tumour volume is reduced as compared to the tumour volume in a subject that is administered a comparative immunoconjugate wherein the comparative immunoconjugate differs from the immunoconjugate only in that the bispecific antibody in the comparative immunoconjugate binds to an antigen that is not expressed in the cancer.

In some embodiments, the delay in tumour progression is longer as compared to the delay in tumour progression in a subject that is administered the bispecific antibody alone.

In some embodiments, the delay in tumour progression is longer as compared to the delay in tumour progression in a subject that is administered a comparative immunoconjugate wherein the comparative immunoconjugate differs from the immunoconjugate only in that the bispecific antibody in the comparative immunoconjugate binds to an antigen that is not expressed in the cancer.

In some embodiments, the delay in tumour progression is longer as compared to the delay in tumour progression in a subject that is administered a comparative immunoconjugate wherein the comparative immunoconjugate differs from the immunoconjugate only in that the growth inhibiting agent in the comparative immunoconjugate is from the class of tubulin inhibitors such as auristatin.

In some embodiments, the immunoconjugate is for use in lowering the risk of toxicity when treating cancer.

This is a remarkable finding of the present invention because one of the concerns in the treatment of cancer is toxic effects of the drugs used. The inventors have surprisingly found that the immunoconjugate of the present invention reduces toxicities and at the same time provides high efficacy in a variety of cancers.

In some embodiments, the risk of toxicity is lower as compared to the risk of toxicity in a subject that is administered a comparative immunoconjugate wherein the comparative immunoconjugate differs from the immunoconjugate only in that the drug linker compound in the comparative immunoconjugate is conjugated to the bispecific antibody at a higher DAR such as two times higher.

In some embodiments, the risk of toxicities such as dermatological toxicities in a subject is lower as compared to the risk of toxicities such as dermatological toxicities in a subject that is administered a comparative immunoconjugate wherein the comparative immunoconjugate differs from the immunoconjugate in that the growth inhibiting agent in the comparative immunoconjugate is hemiasterlin.

In some embodiments, the risk of toxicities such as dermatological toxicities in a subject is lower as compared to the risk of toxicities such as dermatological toxicities in a subject that is administered a comparative immunoconjugate wherein the comparative immunoconjugate substantially differs from the immunoconjugate only in that the growth inhibiting agent in the comparative immunoconjugate is hemiasterlin.

### Dosing regimen

This section is on the dosing regimen of the immunoconjugate of the present invention. The embodiments disclosed herein can be combined with the embodiments disclosed in the section of the anti-EGFR and MUC1 bispecific antibody of the immunoconjugate, the growth inhibiting agent of the immunoconjugate, the linker and drug linker compound of the immunoconjugate, the immunoconjugate, and the medical use and methods of treatment of the immunoconjugate described in the previous sections.

Due to the above surprising finding of lower off-target toxicities such as lower risk of dermatological toxicities, the inventors also surprisingly found that the novel immunoconjugate of the present invention can be safely administered at high doses.

In some embodiments, the subject is administered at least 1 mg/kg of the immunoconjugate. In some embodiments, the subject is administered at least 1.2 mg/kg of the immunoconjugate. In some embodiments, the subject is administered at least 1.5 mg/kg of the immunoconjugate. In some embodiments, the subject is administered at least 1.2 to 10 mg/kg of the immunoconjugate. In some embodiments, the subject is administered at least 1.2 to 8 mg/kg of the immunoconjugate. In some embodiments, the subject is administered at least 1.2 to 7 mg/kg of the immunoconjugate. In some embodiments, the subject is administered at least 1.2 to 6 mg/kg of the immunoconjugate. In some embodiments, the subject is administered at least 1.2 mg/kg, 1.5 mg/kg, at least 3 mg/kg, at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is administered at least 1.5 mg/kg of the immunoconjugate. In some embodiments, the subject is administered at least 1.5 to 10 mg/kg of the immunoconjugate. In some embodiments, the subject is administered at least 1.5 to 8 mg/kg of the immunoconjugate. In some embodiments, the subject is administered at least 1.5 to 7 mg/kg of the immunoconjugate. In some embodiments, the subject is administered at least 1.5 to 6 mg/kg of the immunoconjugate. In some embodiments, the subject is administered at least 1.5 mg/kg, at least 3 mg/kg, at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is administered at least 2 mg/kg of the immunoconjugate. In some embodiments, the subject is administered at least 2 to 10 mg/kg of the immunoconjugate. In some embodiments, the subject is administered at least 2 to 8 mg/kg of the immunoconjugate. In some embodiments, the subject is administered at least 2 to 7 mg/kg of the immunoconjugate. In some embodiments, the subject is administered at least 2 to 6 mg/kg of the immunoconjugate. In some embodiments, the subject is administered at least 2 mg/kg, 3 mg/kg, at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is administered at least 3 mg/kg of the immunoconjugate. In some embodiments, the subject is administered at least 3 to 10 mg/kg of the immunoconjugate. In some embodiments, the subject is administered at least 3 to 8 mg/kg of the immunoconjugate. In some embodiments, the subject is administered at least 3 to 7 mg/kg of the immunoconjugate. In some embodiments, the subject is administered at least 3 to 6 mg/kg of the immunoconjugate. In some embodiments, the subject is administered at least 3 mg/kg, at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is administered at least 3.5 mg/kg of the immunoconjugate. In some embodiments, the subject is administered at least 3.5 to 10 mg/kg of the immunoconjugate. In some embodiments, the subject is administered at least 3.5 to 8 mg/kg of the immunoconjugate. In some embodiments, the subject is administered at least 3.5 to 7 mg/kg of the immunoconjugate. In some embodiments, the subject is administered at least 3.5 to 6 mg/kg of the immunoconjugate. In some embodiments, the subject is administered at least 3.5 mg/kg, at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is administered at least 4 mg/kg of the immunoconjugate. In some embodiments, the subject is administered at least 4 to 10 mg/kg of the immunoconjugate. In some embodiments, the subject is administered at least 4 to 8 mg/kg of the immunoconjugate. In some embodiments, the subject is administered at least 4 to 7 mg/kg of the immunoconjugate. In some embodiments, the subject is administered at least 4 to 6 mg/kg of the immunoconjugate. In some embodiments, the subject is administered at least 4 mg/kg, at least 5 mg/kg, at least 6 mg/kg, at least 7 mg/kg, at least 8 mg/kg, at least 9 mg/kg, or at least 10 mg/kg of the immunoconjugate. In some embodiments, the subject is administered the immunoconjugate or the pharmaceutical composition comprising the immunoconjugate qw, q2w, q3w, or q4w. In some embodiments, the subject is administered the immunoconjugate or the pharmaceutical composition comprising the immunoconjugate q3w.

In some embodiments, the subject is human. In some embodiments, the human subject is administered an effective amount of the immunoconjugate and the effective amount of the immunoconjugate is calculated based on the effective amount of the immunoconjugate being least 4.5 mg/kg to at least 9 mg/kg in a mouse.

In some embodiments, the subject is human. In some embodiments, the human subject is administered an effective amount of the immunoconjugate and the effective amount of the immunoconjugate is calculated based on the effective amount of the immunoconjugate being least 8 mg/kg in a non-human primate.

In some embodiments, the subject is human. In some embodiments, the human subject is administered an effective amount of the immunoconjugate and the effective amount of the immunoconjugate is calculated based on the effective amount of the immunoconjugate being least 16 mg/kg in a non-human primate.

In some embodiments, the subject is human. In some embodiments, the human subject is administered an effective amount of the immunoconjugate and the effective amount of the immunoconjugate is calculated based on the effective amount of the immunoconjugate being least 32 mg/kg in a non-human primate.

In some embodiments, the subject is administered at least 1.0 to 6 mg/kg of the immunoconjugate q3w. In some embodiments, the subject is administered at least 1.2 to 6 mg/kg of the immunoconjugate q3w. In some embodiments, the subject is administered at least 1.5 to 6 mg/kg of the immunoconjugate q3w. In some embodiments, the subject is administered at least 2 to 6 mg/kg of the immunoconjugate q3w.

In some embodiments, the subject is administered at least 1.2 to 5 mg/kg of the immunoconjugate q3w. In some embodiments, the subject is administered at least 1.2 to 4.8 mg/kg of the immunoconjugate q3w.

In some embodiments, the subject is administered the immunoconjugate or the pharmaceutical composition comprising the immunoconjugate as a single dose.

In some embodiments, the subject is administered the immunoconjugate or the pharmaceutical composition comprising the immunoconjugate qw, q2w, q3w, or q4w.

In some embodiments, the subject is administered the immunoconjugate or the pharmaceutical composition comprising the immunoconjugate qw.

In some embodiments, the subject is administered the immunoconjugate or the pharmaceutical composition comprising the immunoconjugate q2w.

In some embodiments, the subject is administered the immunoconjugate or the pharmaceutical composition comprising the immunoconjugate q3w.

In some embodiments, the subject is administered the immunoconjugate or the pharmaceutical composition comprising the immunoconjugate q4w.

In some embodiments, the administration is intravenous injection or intravenous infusion.

### Embodiments of the invention

### First List of embodiments of the invention

**Embodiment: 1** An immunoconjugate comprising:
   (a) a bispecific antibody that binds to EGFR and MUC1,
   (b) a growth inhibiting agent, and
   (c) a linker covalently linking (a) and (b).
**Embodiment: 2** The immunoconjugate of embodiment 1, wherein the bispecific antibody comprises
   - an EGFR binding portion comprising
      ∘ a heavy chain variable region (VH) comprising
         a heavy chain complementary determining region (HCDR) 1 of SEQ ID NO: 1,
         a HCDR 2 of SEQ ID NO: 2, and
         a HCDR 3 of SEQ ID NO: 3, and
      ∘ a light chain variable region (VL) comprising
         a light chain complementarity determining region (LCDR) 1 of SEQ ID NO: 4,
         a LCDR 2 of SEQ ID NO: 5, and
         a LCDR 3 of SEQ ID NO: 6, and
   - a MUC1 binding portion comprising
      ∘ a heavy chain variable region (VH) comprising
         a heavy chain complementary determining region (HCDR) 1 of SEQ ID NO: 7,
         a HCDR 2 of SEQ ID NO: 8, and
         a HCDR 3 of SEQ ID NO: 9, and
      ∘ a light chain variable region (VL) comprising
         a light chain complementarity determining region (LCDR) 1 of SEQ ID NO: 10,
         a LCDR 2 of SEQ ID NO: 11, and
         a LCDR 3 of SEQ ID NO: 12.
**Embodiment:** 3 The immunoconjugate of embodiment 1 or 2, wherein the bispecific antibody comprises
   - an EGFR binding portion comprising
      ∘ a heavy chain variable region (VH) comprising an amino acid sequence that is at least 95%, 96%, 97%, 98%, 99% or 100% identical to the amino acid sequence of SEQ ID NO: 13, and
      ∘ a light chain variable region (VL) comprising an amino acid sequence that is at least 95%, 96%, 97%, 98%, 99% or 100% identical to the amino acid sequence of SEQ ID NO: 14, and
   - a MUC1 binding portion comprising
      ∘ a heavy chain variable region (VH) comprising
         an amino acid sequence that is at least 95%, 96%, 97%, 98%, 99% or 100% identical to the amino acid sequence of SEQ ID NO: 15, and
      ∘ a light chain variable region (VL) comprising
         an amino acid sequence that is at least 95%, 96%, 97%, 98%, 99% or 100% identical to the amino acid sequence of SEQ ID NO: 16.
**Embodiment: 4** The immunoconjugate of any one of embodiments 1 to 3, wherein the bispecific antibody binds to EGFR such as human EGFR with a binding affinity (K_{D}), preferably wherein the K_{D} is less than 10 nM such as less than 5 nM .
**Embodiment: 5** The immunoconjugate of any one of embodiments 2 to 4, wherein the bispecific antibody binds to EGFR such as human EGFR with a binding affinity (K_{D}) via the EGFR binding portion, preferably wherein the K_{D} is less than 10 nM such as less than 5 nM,.
**Embodiment: 6** The immunoconjugate of any one of embodiments 1 to 5, wherein the bispecific antibody binds to EGFR such as human EGFR with a binding affinity (K_{D}) of less than 5 nM.
**Embodiment: 7** The immunoconjugate of any one of embodiments 1 to 6, wherein the bispecific antibody comprises an Fc region comprising two Fc domains, wherein one of the Fc domains comprises at least one knob substitution, and the other Fc domain comprises at least one hole substitution.
**Embodiment: 8** The immunoconjugate of any one of embodiments 1 to 7, wherein the bispecific antibody comprises an Fc region comprising two Fc domains, wherein one of the Fc domains comprises a T366W substitution, and the other Fc domain comprises **at least one** substitution selected from the group consisting of T366S, L368A, and Y407V, wherein EU numbering is used for said amino acid substitutions.
**Embodiment: 9** The immunoconjugate of any one of embodiments 1 to 8, wherein the bispecific antibody comprises an Fc region comprising two Fc domains, wherein one of the Fc domains comprises a T366W substitution, and the other Fc domain comprises T366S, L368A, and Y407V substitutions, wherein EU numbering is used for said amino acid substitutions.
**Embodiment: 10** The immunoconjugate of any one of embodiments 1 to 9, wherein the bispecific antibody comprises an Fc region comprising two Fc domains, wherein one of the Fc domains comprises a S354C substitution, and the other Fc domain comprises a Y349C substitution, wherein EU numbering is used for said amino acid substitutions.
**Embodiment: 11** The immunoconjugate of any one of embodiments 1 to 10, wherein the bispecific antibody comprises an Fc region comprising two Fc domains, wherein one of the Fc domains comprises T366W and S354C substitutions, and the other Fc domain comprises T366S, L368A, Y407V, and Y349C substitutions, wherein EU numbering is used for said amino acid substitutions.
**Embodiment: 12** The immunoconjugate of any one of embodiments 1 to 11, wherein the bispecific antibody comprises an Fc region comprising two Fc domains and at least one, and preferably both Fc domains comprise a C226S substitution, wherein EU numbering is used for said amino acid substitution.
**Embodiment: 13** The immunoconjugate of any one of embodiments 1 to 12, wherein the bispecific antibody comprises an Fc region comprising two Fc domains and at least one, and preferably both Fc domains comprise a L234A and L235A (LALA) substitution, wherein EU numbering is used for said amino acid substitutions.
**Embodiment: 14** The immunoconjugate of any one of embodiments 1 to 13, wherein the bispecific antibody comprises an Fc region comprising two Fc domains and at least one, and preferably both Fc domains comprise a D356E and L358M substitution, wherein EU numbering is used for said amino acid substitutions.
**Embodiment: 15** The immunoconjugate of any one of embodiments 1 to 14, wherein the bispecific antibody comprises an Fc region comprising two Fc domains and at least one, and preferably both Fc domains comprise **at least one** of the following substitutions
   - a C226S substitution,
   - a L234A, and L235A substitution (LALA substitution), and
   - D356E and L358M substitution,
   wherein EU numbering is used for said amino acid substitutions.
**Embodiment: 16** The immunoconjugate of any one of embodiments 1 to 15 wherein the bispecific antibody comprises an Fc region comprising two Fc domains and at least one, and preferably both Fc domains comprise the following substitutions
   - a C226S substitution,
   - a L234A, and L235A substitution (LALA substitution), and
   - D356E and L358M substitution,
   wherein EU numbering is used for said amino acid substitutions.
**Embodiment: 17** The immunoconjugate of any one of embodiments 2 to 6, wherein the bispecific antibody comprises:
   (i) a first polypeptide comprising a first Fc domain and a single-chain Fv fragment (scFv), wherein the scFv comprises the VH and VL of the MUC1 binding portion,
   (ii) a second polypeptide comprising a second Fc domain and a heavy chain of a Fab fragment comprising the VH of the EGFR binding portion, and
   (iii) a third polypeptide comprising a light chain of the Fab fragment comprising the VL of the EGFR binding portion.
**Embodiment: 18** The immunoconjugate of embodiment 17, wherein
   - the Fc domain of the first polypeptide comprises at least one knob substitution, and the Fc domain of the second polypeptide comprises at least one hole substitution, or
   - the Fc domain of the second polypeptide comprises at least one knob substitution, and the Fc domain of the first polypeptide comprises at least one hole substitution.
**Embodiment: 19** The immunoconjugate of embodiment 17 or 18, wherein
   - the first polypeptide comprises a T366W substitution, and the second polypeptide comprises **at least one** substitution selected from the group consisting of T366S, L368A, and Y407V, or
   - the second polypeptide comprises a T366W substitution, and the first polypeptide comprises at least one substitution selected from the group consisting of T366S, L368A, and Y407V,
   wherein EU numbering is used for said amino acid substitutions.
**Embodiment: 20** The immunoconjugate of any one of embodiments 17 to 19, wherein
   - the first polypeptide comprises a T366W substitution, and the second polypeptide comprises T366S, L368A, and Y407V substitutions, or
   - the second polypeptide comprises a T366W substitution, and the first polypeptide comprises T366S, L368A, and Y407V substitutions,
   wherein EU numbering is used for said amino acid substitutions.
**Embodiment: 21** The immunoconjugate of any one of embodiments 17 to 20, wherein
   - the first polypeptide comprises a S354C substitution, and the second polypeptide comprises a Y349C substitution, or
   - the second polypeptide comprises a S354C substitution, and the first polypeptide comprises a Y349C substitution,
   wherein EU numbering is used for said amino acid substitutions.
**Embodiment: 22** The immunoconjugate of any one of embodiments 17 to 21, wherein
   - the first polypeptide comprises T366W and S354C substitutions, and the second polypeptide comprises T366S, L368A, Y407V, and Y349C substitutions, or
   - the second polypeptide comprises a T366W and S354C substitutions, and the first polypeptide comprises T366S, L368A, Y407V, and Y349C substitutions,
   wherein EU numbering is used for said amino acid substitutions.
**Embodiment: 23** The immunoconjugate of any one of embodiments 17 to 22, wherein the Fc domain of the first polypeptide comprises at least one knob substitution, and the Fc domain of the second polypeptide comprises at least one hole substitution.
**Embodiment: 24** The immunoconjugate of any one of embodiments 17 to 23, wherein the first polypeptide comprises a T366W substitution, and the second polypeptide comprises **at least one** substitution selected from the group consisting of T366S, L368A, and Y407V, wherein EU numbering is used for said amino acid substitutions.
**Embodiment: 25** The immunoconjugate of any one of embodiments 17 to 24, wherein the first polypeptide comprises a T366W substitution, and the second polypeptide comprises T366S, L368A, and Y407V substitutions, wherein EU numbering is used for said amino acid substitutions.
**Embodiment: 26** The immunoconjugate of any one of embodiments 17 to 25, wherein the first polypeptide comprises a S354C substitution, and the second polypeptide comprises a Y349C substitution, wherein EU numbering is used for said amino acid substitutions.
**Embodiment: 27** The immunoconjugate of any one of embodiments 17 to 26, wherein the first polypeptide comprises T366W and S354C substitutions, and the second polypeptide comprises T366S, L368A, Y407V and Y349C substitutions, wherein EU numbering is used for said amino acid substitutions.
**Embodiment: 28** The immunoconjugate of any one of embodiments 17 to 27, wherein the first and/or the second polypeptide comprises a C226S substitution, wherein EU numbering is used for said amino acid substitution.
**Embodiment: 29** The immunoconjugate of any one of embodiments 17 to 28, wherein the first and/or the second polypeptide each comprises a L234A and L235A (LALA) substitution, wherein EU numbering is used for said amino acid substitutions.
**Embodiment: 30** The immunoconjugate of any one of embodiments 17 to 29, wherein the first and/or the second polypeptide each comprises a D356E and L358M substitution, wherein EU numbering is used for said amino acid substitutions.
**Embodiment: 31** The immunoconjugate of any one of embodiments 17 to 30, wherein the first polypeptide comprises **at least one** of the following substitutions:
   - a C226S substitution,
   - a L234A, and L235A substitution (LALA substitution), and
   - D356E and L358M substitution,
   wherein EU numbering is used for said amino acid substitutions.
**Embodiment: 32** The immunoconjugate of any one of embodiments 17 to 31, wherein the first polypeptide comprises the following substitutions:
   - a C226S substitution,
   - a L234A, and L235A substitution (LALA substitution), and
   - D356E and L358M substitution,
   wherein EU numbering is used for said amino acid substitutions.
**Embodiment: 33** The immunoconjugate of any one of embodiments 17 to 32, wherein the second polypeptide comprises **at least one** of the following substitutions:
   - a C226S substitution,
   - a L234A, and L235A substitution (LALA substitution), and
   - D356E and L358M substitution,
   wherein EU numbering is used for said amino acid substitutions.
**Embodiment: 34** The immunoconjugate of any one of embodiments 17 to 33, wherein the second polypeptide comprises the following substitutions:
   - a C226S substitution,
   - a L234A, and L235A substitution (LALA substitution), and
   - D356E and L358M substitution,
   wherein EU numbering is used for said amino acid substitutions.
**Embodiment: 35** The immunoconjugate of any one of embodiments 17 to 34, wherein the first and the second polypeptide comprises the following substitutions:
   - a C226S substitution,
   - a L234A, and L235A substitution (LALA substitution), and
   - D356E and L358M substitution,
   wherein EU numbering is used for said amino acid substitutions.
**Embodiment: 36** The immunoconjugate of any one of embodiments 17 to 35, wherein
   - the first polypeptide comprises
      an amino acid sequence that is at least 90%, 95%, 96%, 97%, 98%, 99% or 100% identical to the amino acid sequence of SEQ ID NO: 17,
   - the second polypeptide comprises
      an amino acid sequence that is at least 90%, 95%, 96%, 97%, 98%, 99% or 100% identical to the amino acid sequence of SEQ ID NO: 18, and
   - the third polypeptide comprises
      an amino acid sequence that is at least 90%, 95%, 96%, 97%, 98%, 99% or 100% identical to the amino acid sequence of SEQ ID NO: 19.
**Embodiment: 37** The immunoconjugate of any one of embodiments 1 to 36, wherein the bispecific antibody is glycosylated.
**Embodiment: 38** The immunoconjugate of any one of embodiments 1 to 37, wherein the growth inhibitory agent is a cytotoxic drug or a radioactive moiety.
**Embodiment: 39** The immunoconjugate of any one of embodiments 1 to 38, wherein the growth inhibitory agent is selected from a group consisting of a chemotherapeutic agent, enzyme, antibiotic, a toxin such as a small molecule toxin or enzymatically active toxin, toxoid, vinca, taxane, maytansinoid or maytansinoid analog, tomaymycin or pyrrolobenzodiazepine derivative, cryptophycin derivative, leptomycin derivative, microtubulin inhibitor such as auristatin or dolastatin analog, prodrug, topoisomerase I inhibitor, topoisomerase II inhibitor, DNA alkylating agent, anti-tubulin agent, CC-1065 and CC-1065 analog.
**Embodiment: 40** The immunoconjugate of any one of embodiments 1 to 39, wherein the growth inhibitory agent is exatecan or deruxtecan.
**Embodiment: 41** The immunoconjugate of any one of embodiments 1 to 40, wherein the growth inhibitory agent is exatecan.
**Embodiment: 42** The immunoconjugate of any one of embodiments 1 to 41, wherein the linker is a cleavable linker.
**Embodiment: 43** The immunoconjugate of any one of embodiments 1 to 42, wherein the linker is a linker cleavable in an endosome of a mammalian cell.
**Embodiment: 44** The immunoconjugate of any one of embodiments 1 to 43, wherein the linker is a linker cleavable by a human enzyme selected from glucuronidase and legumain.
**Embodiment: 45** The immunoconjugate of any one of embodiments 1 to 44, wherein the linker is a linker cleavable by a human glucuronidase enzyme.
**Embodiment: 46** The immunoconjugate of any one of embodiments 1 to 45, wherein the linker is a β-glucuronide linker.
**Embodiment: 47** The immunoconjugate of any one of embodiments 1 to 46, wherein the linker and the growth inhibitory agent in combination represents a drug-linker compound, preferably wherein the drug-linker compound is conjugated to the bispecific antibody at an average drug-to-antibody ratio (DAR) from 1 to 10 such as 2 to 8 or about 4 such as 3.5 to 4.5 or 3.8 to 4.2.
**Embodiment: 48** The immunoconjugate of embodiment 47, wherein the drug-linker compound has the following formula: 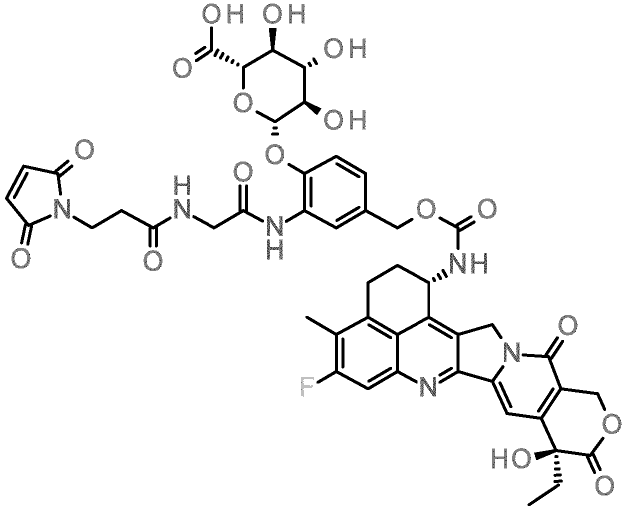
**Embodiment: 49** The immunoconjugate of any one of embodiments 1 to 48, wherein the immunoconjugate has the following formula: 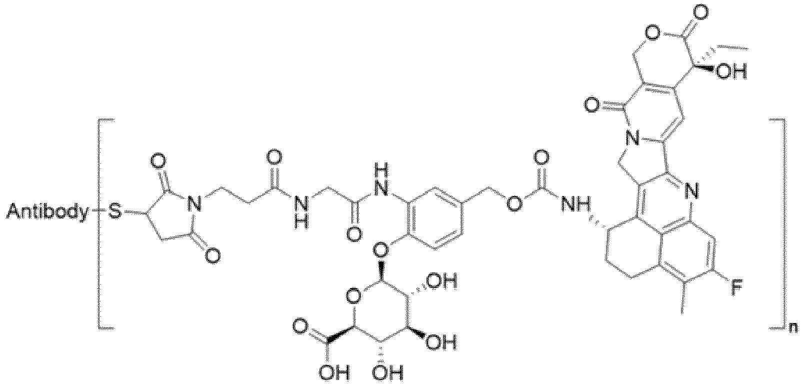
**Embodiment: 50** The immunoconjugate of embodiment 49, wherein the S is a sulfur atom of a cysteine of the bispecific antibody.
**Embodiment: 51** The immunoconjugate of embodiment 50, wherein the cysteine of the bispecific antibody is one of the cysteines capable of forming an interchain disulfide bond.
**Embodiment: 52** The immunoconjugate of any one of embodiments 49 to 51, wherein n represents an average drug-to-antibody ratio (DAR).
**Embodiment: 53** The immunoconjugate of any one of embodiments 49 to 52, wherein n is from 1 to 10, from 2 to 6, or from 1 to 5.
**Embodiment: 54** The immunoconjugate of any one of embodiments 49 to 53, wherein n is below 8.
**Embodiment: 55** The immunoconjugate of any of embodiments 48 to 54, wherein n is below 5.
**Embodiment: 56** The immunoconjugate of any one of embodiments 49 to 55, wherein n is from about 2 to about 4.
**Embodiment: 57** The immunoconjugate of any one of embodiments 49 to 56, wherein n is about 4 such as 3.5 to 4.5, or 3.8 to 4.2.
**Embodiment: 58** A pharmaceutical composition comprising the immunoconjugate of any one of embodiments 1 to 57, and a pharmaceutically acceptable excipient.
**Embodiment: 59** The immunoconjugate of any one of embodiments 1 to 57 or the pharmaceutical composition of embodiment 58, for use as a medicament.
**Embodiment: 60** The immunoconjugate of any one of embodiments 1 to 57, or the pharmaceutical composition of embodiment 58 for use in the treatment of cancer in a subject.
**Embodiment: 61** The immunoconjugate of any one of embodiments 1 to 57, or the pharmaceutical composition of embodiment 58 for use in reducing tumour volume in a subject having cancer.
**Embodiment: 62** The immunoconjugate of any one of embodiments 1 to 57, or the pharmaceutical composition of embodiment 58 for use in delaying tumour progression in a subject having cancer.
**Embodiment: 63** The immunoconjugate of any one of embodiments 1 to 57, or the pharmaceutical composition of embodiment 58 for use in reducing the risk of toxicities when treating cancer in a subject.
**Embodiment: 64** The immunoconjugate of any one of embodiments 1 to 57, or the pharmaceutical composition of embodiment 58 for use in reducing the risk of dermatological toxicities when treating cancer in a subject.
**Embodiment: 65** Use of the immunoconjugate of any one of embodiments 1 to 57, or the pharmaceutical composition of embodiment 58 for the manufacture of a medicament for the treatment of cancer in a subject.
**Embodiment: 66** Use of the immunoconjugate of any one of embodiments 1 to 57, or the pharmaceutical composition of embodiment 58 for the manufacture of a medicament for reducing tumour volume in a subject having cancer.
**Embodiment: 67** Use of the immunoconjugate of any one of embodiments 1 to 57, or the pharmaceutical composition of embodiment 58 for the manufacture of a medicament for delaying tumour progression in a subject having cancer.
**Embodiment: 68** Use of the immunoconjugate of any one of embodiments 1 to 57, or the pharmaceutical composition of embodiment 58 for the manufacture of a medicament for reducing the risk of toxicities when treating cancer in a subject.
**Embodiment: 69** Use of the immunoconjugate of any one of embodiments 1 to 57, or the pharmaceutical composition of embodiment 58 for the manufacture of a medicament for reducing the risk of dermatological toxicities when treating cancer in a subject.
**Embodiment: 70** The immunoconjugate or the pharmaceutical composition for use of any one of embodiments 59 to 64, or the use of any one of embodiments 55 to 59, wherein the subject is administered the pharmaceutical composition or the immunoconjugate.
**Embodiment: 71** A method of treating cancer in a subject comprising administering an effective amount of the immunoconjugate of any one of embodiments 1 to 58 to the subject.
**Embodiment: 72** The immunoconjugate or the pharmaceutical composition for use of any one of embodiments 59 to 64, or 70, or the use of any one of embodiments 65 to 70, or the method of embodiment 71, wherein the cancer is a MUC1 expressing cancer and preferably EGFR expressing cancer.
**Embodiment: 73** The immunoconjugate or the pharmaceutical composition for use of any one of embodiments 59 to 64, 70, or 72, or the use of any one of embodiments 65 to 70, or 72, or the method of embodiment 71 or 72, wherein the cancer is selected from the group consisting of biliary tract cancer, esophageal squamous cell carcinoma (ESCC), non-small cell lung cancer (NSCLC) ovarian cancer such as epithelial ovarian cancer, bladder cancer, and squamous cell carcinoma of head and neck (SCCHN).
**Embodiment: 74** The immunoconjugate or the pharmaceutical composition for use of any one of embodiments 58 to 64, 70, 72 or 73, or the use of any one of embodiments 65 to 70, 72 or 73, or the method of any one of embodiments 71 to 73, wherein the subject is human.
**Embodiment: 75** The immunoconjugate or the pharmaceutical composition for use of any one of embodiments 70 to 74, or the use of any one of embodiments 70 to 74, or the method of any one of embodiments 71 to 74, wherein the subject is administered at least 1 mg/kg of the immunoconjugate or from 1 mg/kg to 10 mg/kg of the immunoconjugate.
**Embodiment: 76** The immunoconjugate or the pharmaceutical composition for use of any one of embodiments 70 to 75, or the use of any one of embodiments 70 to 75, or the method of any one of embodiments 71 to 75, wherein the subject is administered the pharmaceutical composition or an effective amount of the immunoconjugate qw, q2w, or q3w.

### Second list of embodiments of the invention

**Embodiment: 2** An immunoconjugate comprising:
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion,
      - a second polypeptide comprising a second Fc domain and a heavy chain of a Fab fragment comprising a VH of the EGFR binding portion, and
      - a third polypeptide comprising a light chain of the Fab fragment comprising a VL of the EGFR binding portion,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises at least one knob substitution, and the Fc domain of the second polypeptide comprises at least one hole substitution, or the second Fc domain of the second polypeptide comprises at least one knob substitution, and the Fc domain of the first polypeptide comprises at least one hole substitution,
   wherein the growth inhibiting agent is a topoisomerase I inhibitor such as exatecan, and wherein the linker is a cleavable linker, preferably a β-glucuronide linker preferably comprising a maleimide moiety.
**Embodiment: 3** An immunoconjugate comprising:
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion,
      - a second polypeptide comprising a second Fc domain and a heavy chain of a Fab fragment comprising a VH of the EGFR binding portion, and
      - a third polypeptide comprising a light chain of the Fab fragment comprising a VL of the EGFR binding portion,

      ∘ wherein the VH of the EGFR binding portion comprises an amino acid sequence that is at least 95% identical to the amino acid sequence of SEQ ID NO: 13, and comprises ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises at least one knob substitution, and the Fc domain of the second polypeptide comprises at least one hole substitution, or the second Fc domain of the second polypeptide comprises at least one knob substitution, and the Fc domain of the first polypeptide comprises at least one hole substitution,
   wherein the growth inhibiting agent is exatecan, and the linker is a β-glucuronide linker comprising a maleimide moiety, wherein said linker and said growth inhibiting agent in combination represents a drug-linker compound that has the following formula **(I):** 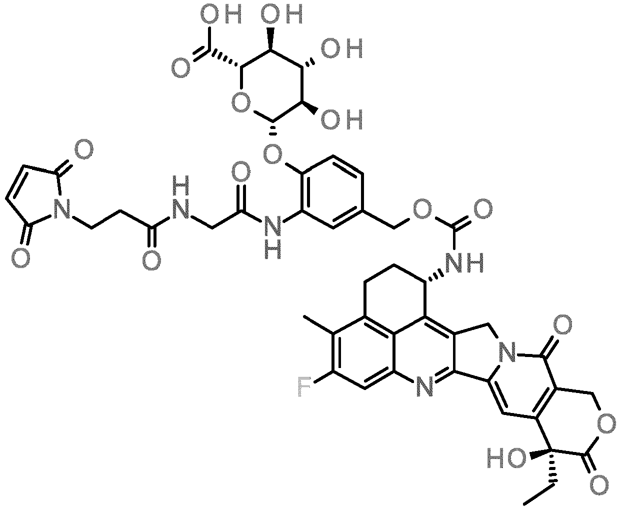
   and wherein the drug-linker compound is conjugated to the antibody at an average drug-to-antibody ratio (DAR) of below 8 or about 4 such as 3.5 to 4.5 or 3.8 to 4.2.
**Embodiment: 4** An immunoconjugate comprising:
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion,
      - a second polypeptide comprising a second Fc domain and a heavy chain of a Fab fragment comprising a VH of the EGFR binding portion, and
      - a third polypeptide comprising a light chain of the Fab fragment comprising a VL of the EGFR binding portion,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises at least one knob substitution, and the Fc domain of the second polypeptide comprises at least one hole substitution, or the second Fc domain of the second polypeptide comprises at least one knob substitution, and the Fc domain of the first polypeptide comprises at least one hole substitution,
   wherein the growth inhibiting agent is exatecan, and the linker is a β-glucuronide linker comprising a maleimide moiety, wherein the immunoconjugate has the following formula **(II):** 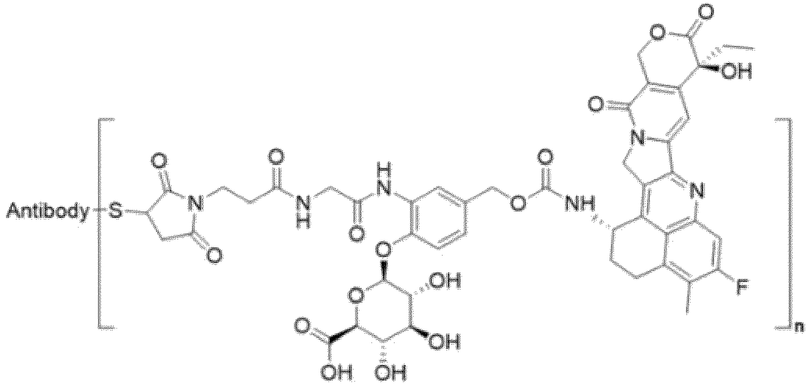
   wherein "n" represents an average drug-to-antibody ratio (DAR), wherein the drug-linker compound is conjugated to the antibody at a DAR of below 8 or about 4 such as 3.5 to 4.5 or 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 5** An immunoconjugate of any one of embodiments 1 to 3, wherein the first polypeptide comprises a T366W and S354C substitution, and the second polypeptide comprises T366S, L368A, Y407V, and Y349C substitutions, and wherein the first and the second polypeptide each comprises the following substitutions:
   - a C226S substitution,
   - a L234A, and L235A substitution (LALA substitution), and
   - D356E and L358M substitution.
**Embodiment: 6** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 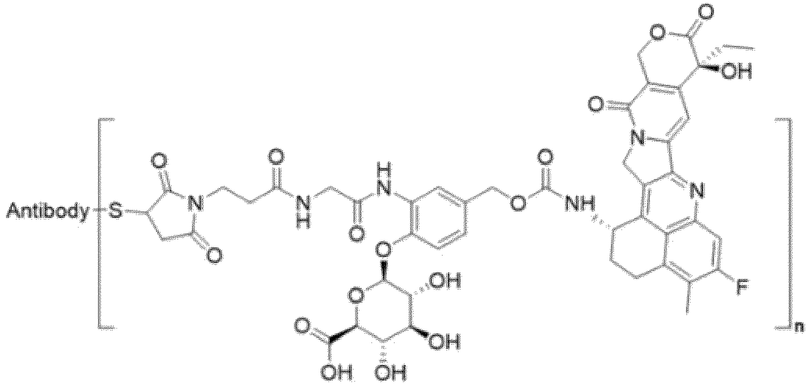
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 7** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 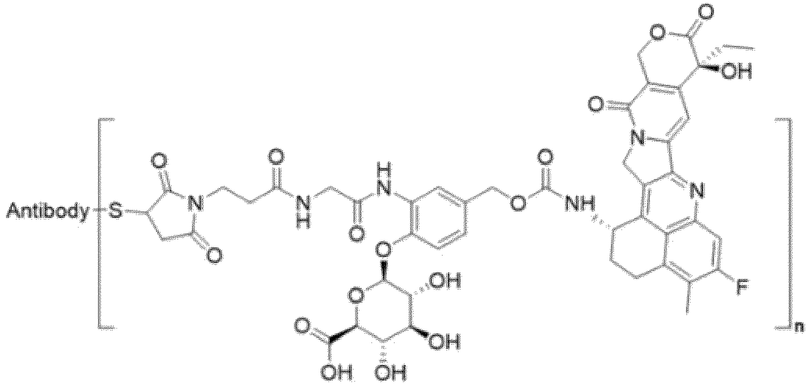
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 8** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 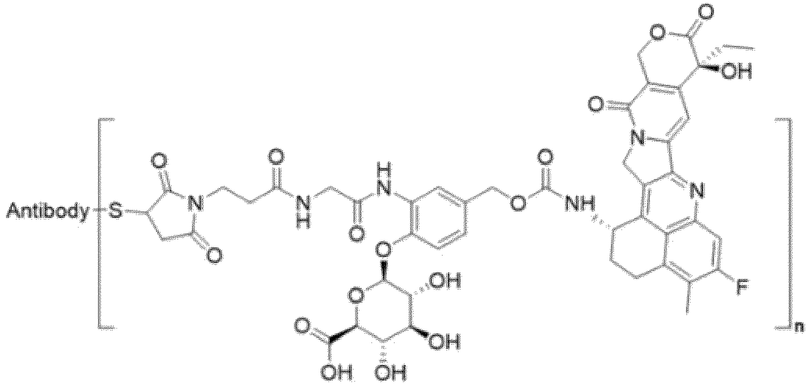
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 9** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      o HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      o LCDR 1 of SEQ ID NO: 4,
      o LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      o HCDR 1 of SEQ ID NO: 7,
      o HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      o LCDR 1 of SEQ ID NO: 10,
      o LCDR2 of SEQ ID NO: 11, and
      o LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 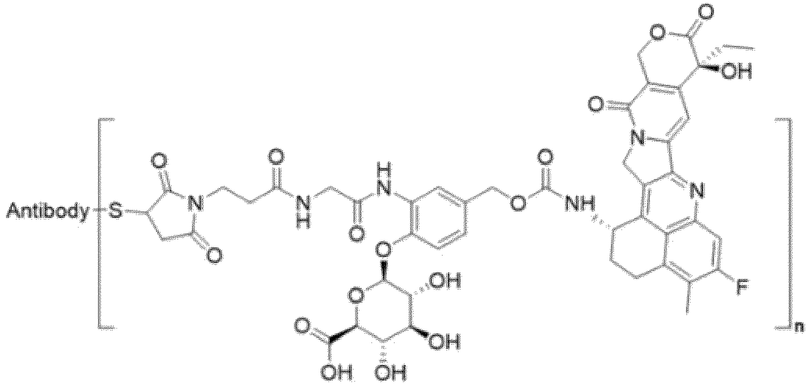
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 10** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 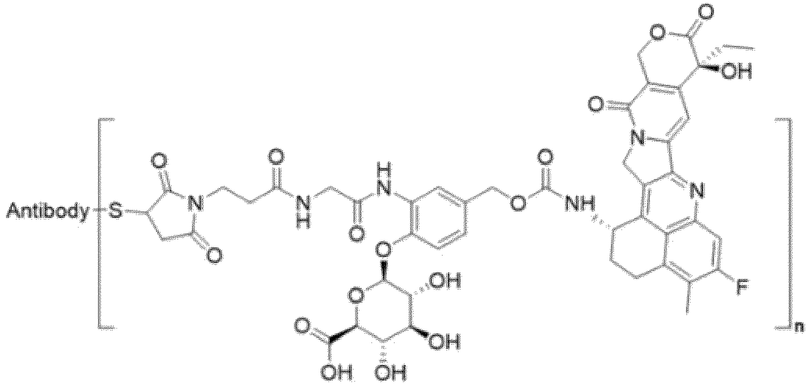
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 11** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 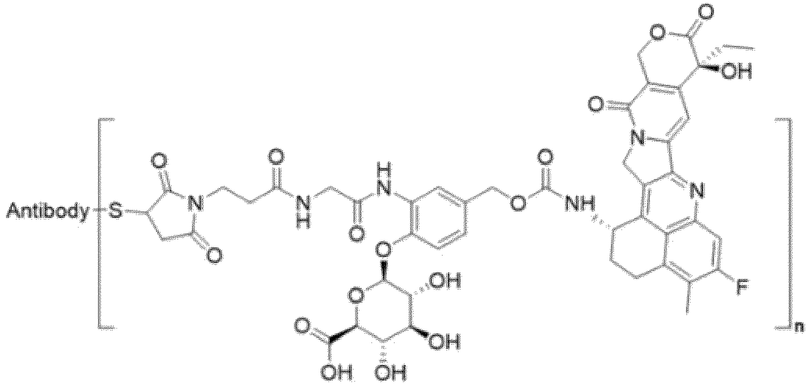
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 12** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
   wherein the VH of the EGFR binding portion comprises the amino acid sequence of SEQ ID NO: 13, the VL of the EGFR binding portion comprises the amino acid sequence of SEQ ID NO: 14,
   wherein the VH of the MUC1 binding portion comprises the amino acid sequence of SEQ ID NO: 15, wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 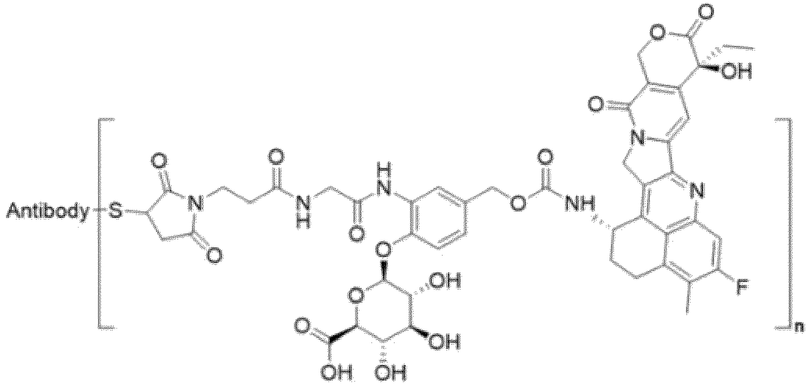
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 13** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 95% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 95% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 95% identical to the amino acid sequence of SEQ ID NO: 19,
      - wherein the VH of the EGFR binding portion comprises the amino acid sequence of SEQ ID NO: 13,
      - the VL of the EGFR binding portion comprises the amino acid sequence of SEQ ID NO: 14,
      - wherein the VH of the MUC1 binding portion comprises the amino acid sequence of SEQ ID NO: 15,
      - wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 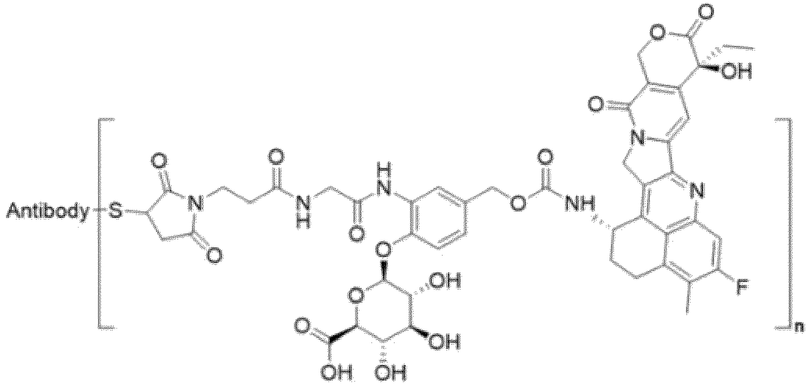
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 14** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 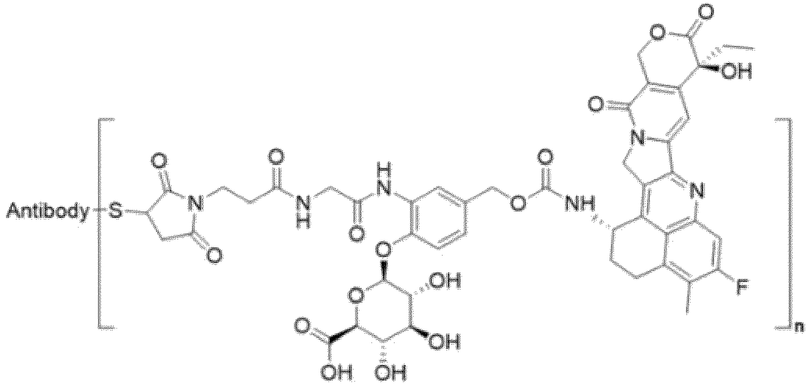
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 15** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 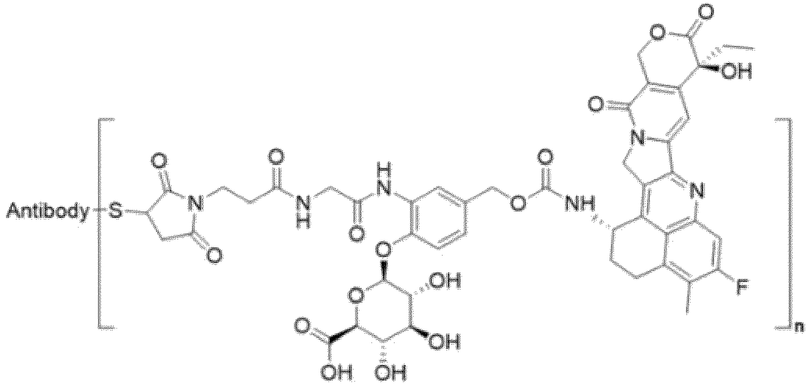
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 16** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 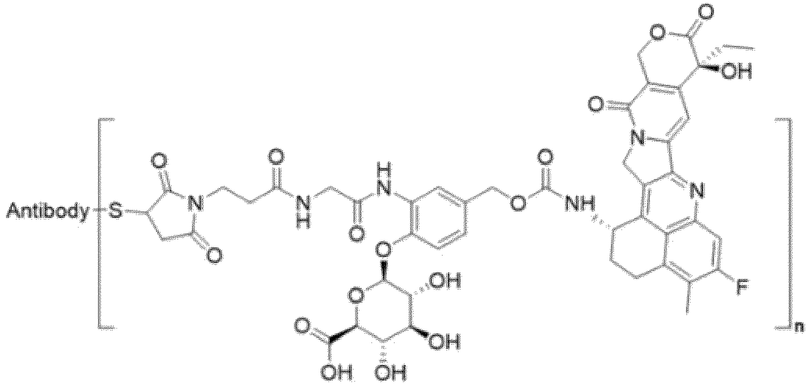
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 17** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 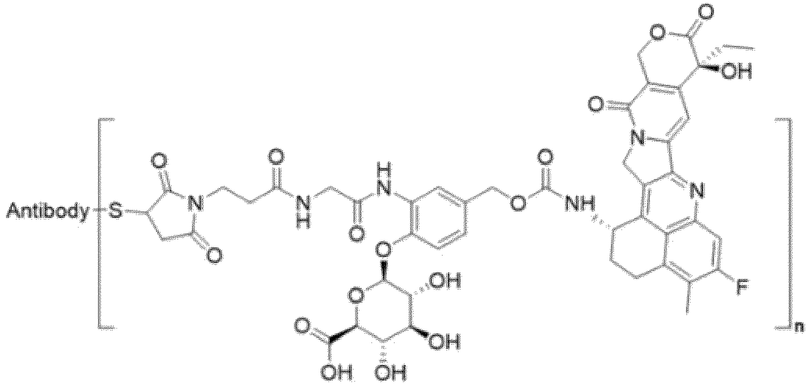
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 18** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at
   position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 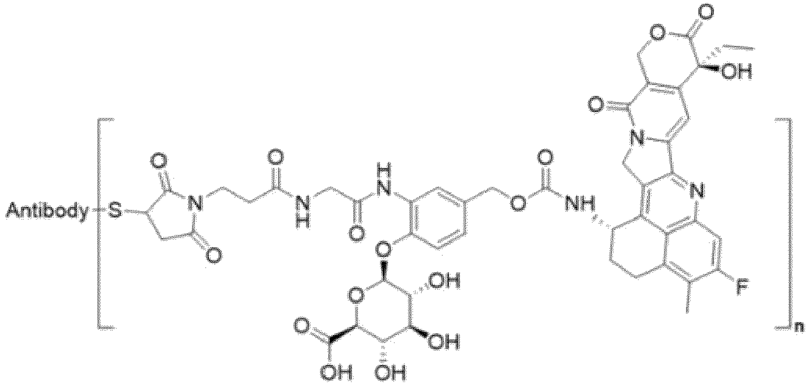
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 19** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 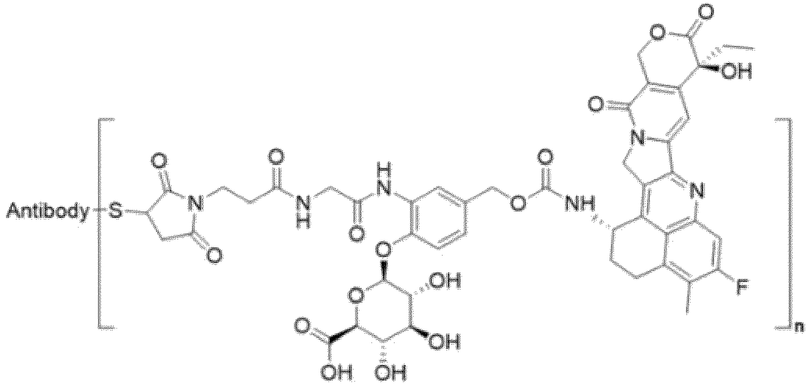
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 20** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
   wherein the VH of the EGFR binding portion comprises the amino acid sequence of SEQ ID NO: 13, the VL of the EGFR binding portion comprises the amino acid sequence of SEQ ID NO: 14,
   wherein the VH of the MUC1 binding portion comprises the amino acid sequence of SEQ ID NO: 15, wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 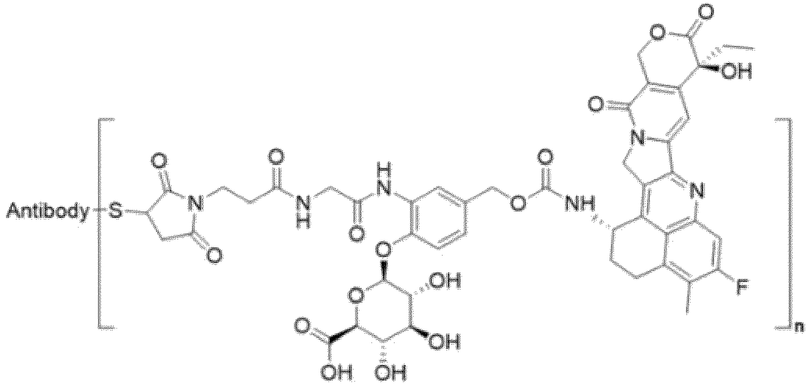
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 21** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 98% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 98% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 98% identical to the amino acid sequence of SEQ ID NO: 19,
      - wherein the VH of the EGFR binding portion comprises the amino acid sequence of SEQ ID NO: 13,
      - the VL of the EGFR binding portion comprises the amino acid sequence of SEQ ID NO: 14,
      - wherein the VH of the MUC1 binding portion comprises the amino acid sequence of SEQ ID NO: 15,
      - wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 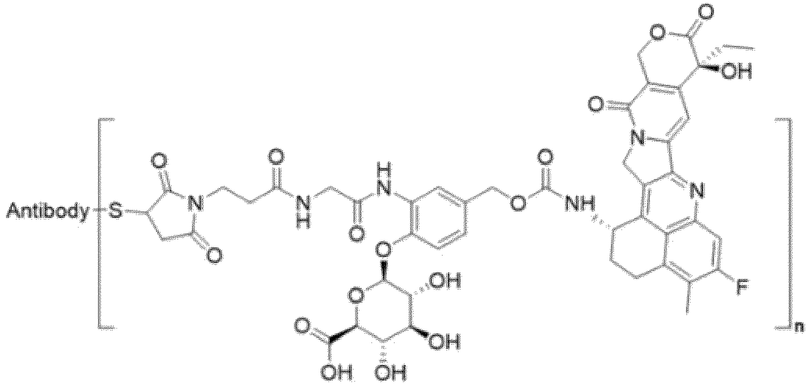
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 22** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 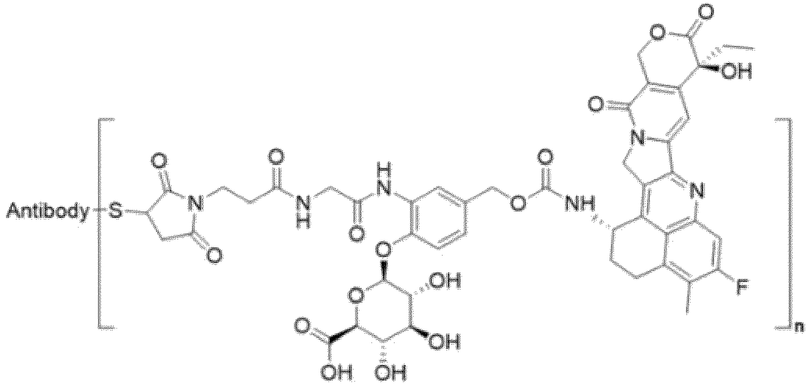
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 23** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 95% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 95% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 95% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 95% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 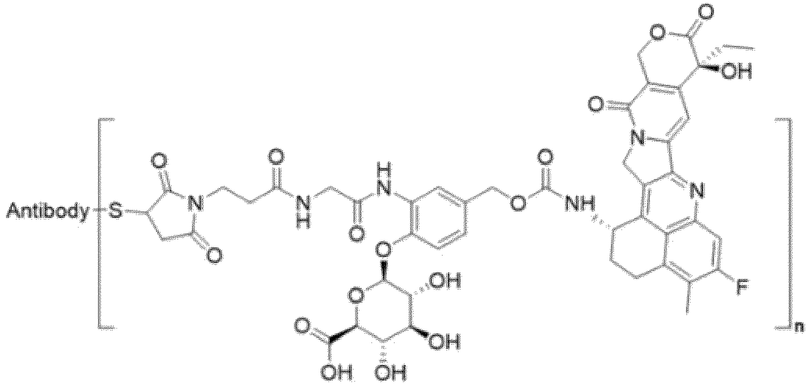
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 24** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 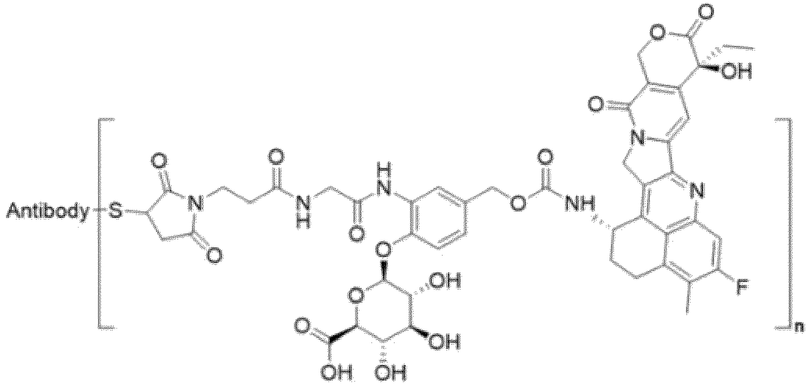
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 25** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 98% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 98% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 98% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 98% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 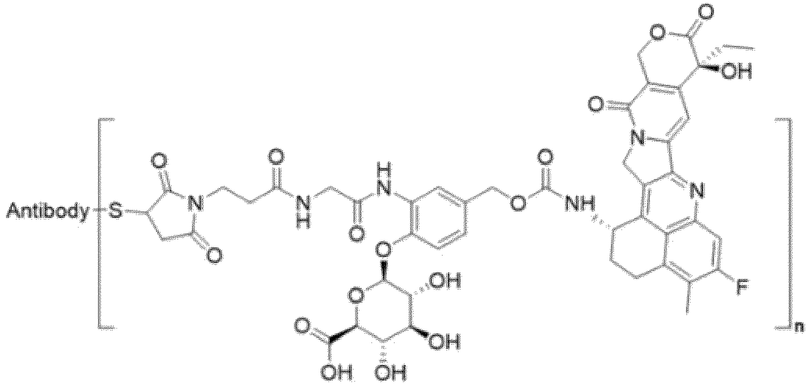
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 26** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 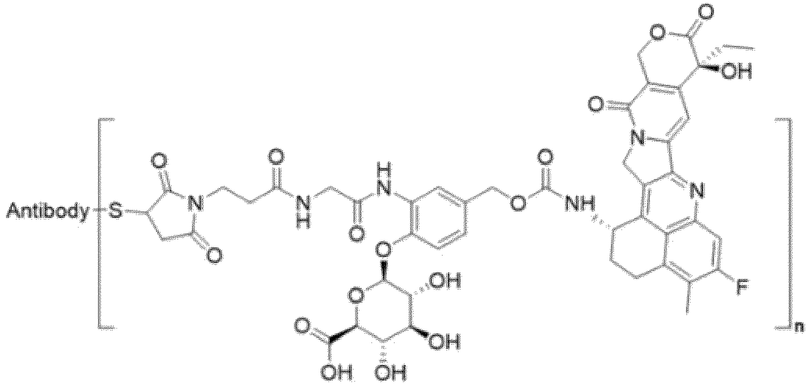
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 27** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises an amino acid sequence that is at least 99% identical to the amino acid sequence of SEQ ID NO: 13, and comprises
      ∘ HCDR 1 of SEQ ID NO: 1,
      ∘ HCDR2 of SEQ ID NO: 2, and
      ∘ HCDR 3 of SEQ ID NO: 3, and
   the VL of the EGFR binding portion comprises an amino acid sequence that is at least 99% identical to the amino acid sequence of SEQ ID NO: 14, and comprises
      ∘ LCDR 1 of SEQ ID NO: 4,
      ∘ LCDR2 of SEQ ID NO: 5, and
      ∘ LCDR 3 of SEQ ID NO: 6, and
   wherein the VH of the MUC1 binding portion comprises an amino acid sequence that is at least 99% identical to the amino acid sequence of SEQ ID NO: 15, and comprises
      ∘ HCDR 1 of SEQ ID NO: 7,
      ∘ HCDR2 of SEQ ID NO: 8, and
      ∘ HCDR 3 of SEQ ID NO: 9, and
   wherein the VL of the EGFR binding portion comprises an amino acid sequence that is at least 99% identical to the amino acid sequence of SEQ ID NO: 16, and comprises
      ∘ LCDR 1 of SEQ ID NO: 10,
      ∘ LCDR2 of SEQ ID NO: 11, and
      ∘ LCDR 3 of SEQ ID NO: 12, and
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 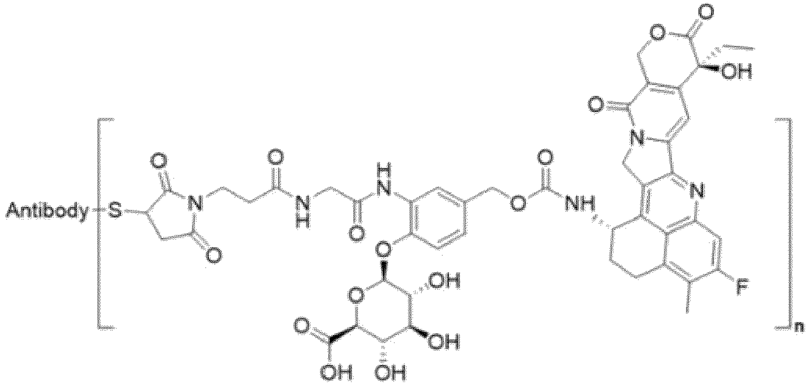
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 28** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
   wherein the VH of the EGFR binding portion comprises the amino acid sequence of SEQ ID NO: 13, the VL of the EGFR binding portion comprises the amino acid sequence of SEQ ID NO: 14,
   wherein the VH of the MUC1 binding portion comprises the amino acid sequence of SEQ ID NO: 15,
   wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 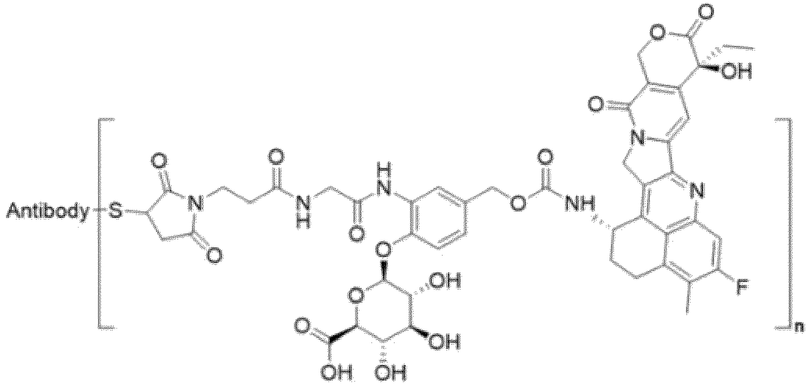
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 29** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises an amino acid sequence that is at least 99% identical to the amino acid sequence of SEQ ID NO: 17,
      - a second polypeptide comprising a second Fc domain and a heavy chain of a Fab fragment comprising a VH of the EGFR binding portion, wherein said second polypeptide comprises an amino acid sequence that is at least 99% identical to the amino acid sequence of SEQ ID NO: 18, and
      - a third polypeptide comprising a light chain of the Fab fragment comprising a VL of the EGFR binding portion, wherein said third polypeptide comprises an amino acid sequence that is at least 99% identical to the amino acid sequence of SEQ ID NO: 19,
      - wherein the VH of the EGFR binding portion comprises the amino acid sequence of SEQ ID NO: 13,
      - the VL of the EGFR binding portion comprises the amino acid sequence of SEQ ID NO: 14,
      - wherein the VH of the MUC1 binding portion comprises the amino acid sequence of SEQ ID NO: 15,
      - wherein the VL of the EGFR binding portion comprises the amino acid sequence of SEQ ID NO: 16,
   wherein the first Fc domain of the first polypeptide comprises a tryptophan at position 366, a cysteine at position 354, a serine at position 226, an alanine at position 234, an alanine at position 235, a glutamic acid at position 356 and a methionine at position 358, and
   the second Fc domain of the second polypeptide comprises a serine at position 366, an alanine at position 368, a valine at position 407, a cysteine at position 349, a serine at position 226, an alanine at position 234, an alanine at position 235, a glutamic acid at position 356 and a methionine at position 358,
   wherein amino acid positions are defined by EU numbering;
   wherein the growth inhibiting agent is exatecan, and the linker is a β-glucuronide linker comprising a maleimide moiety, wherein the immunoconjugate has the following formula **(II):** 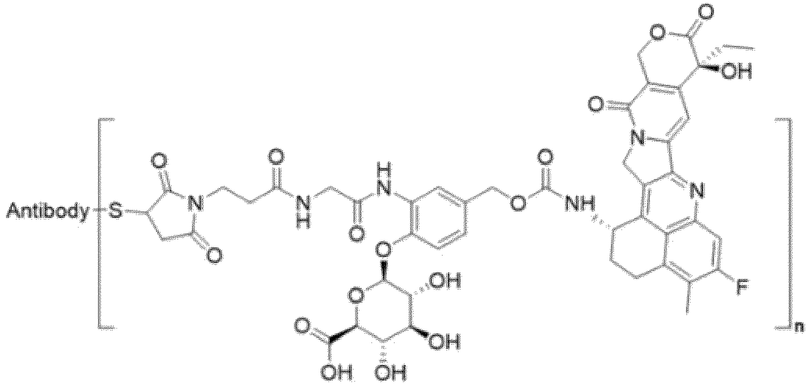
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 30** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 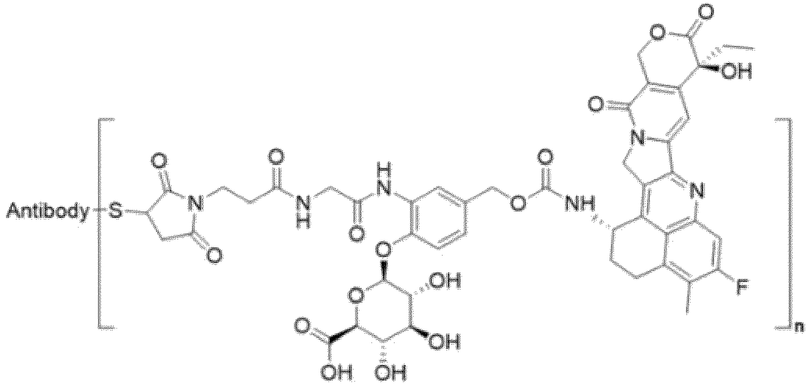
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 31** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 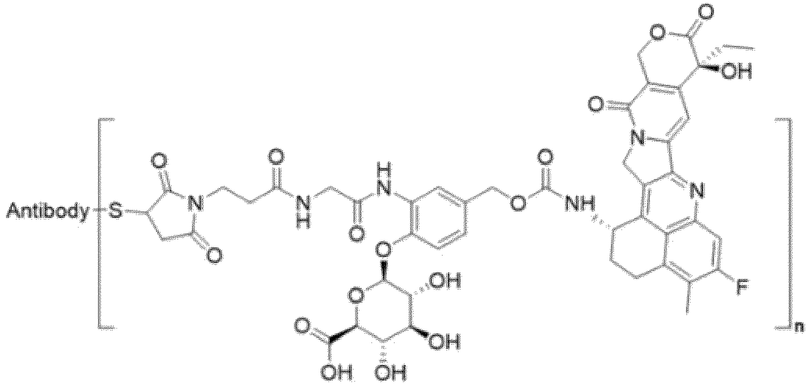
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 32** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 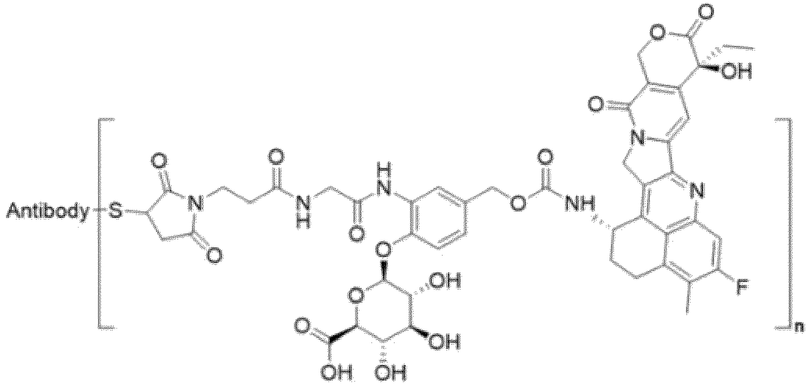
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 33** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 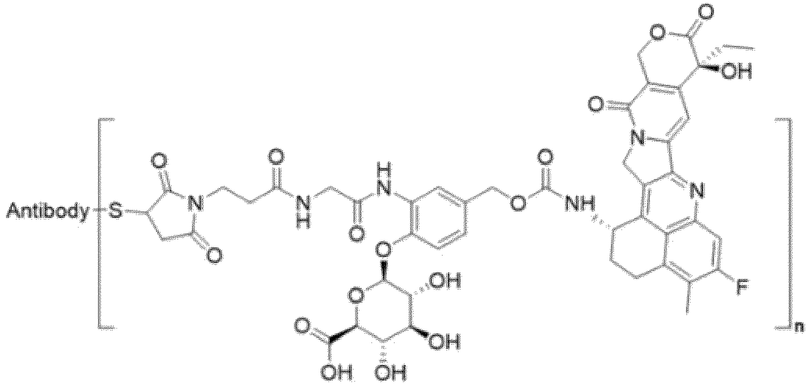
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 34** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 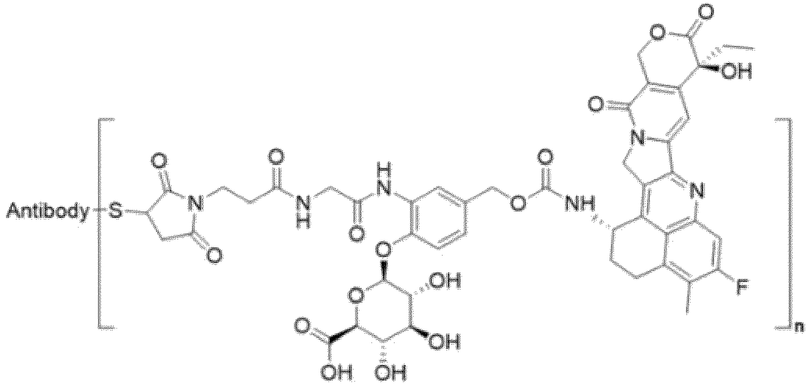
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 35** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 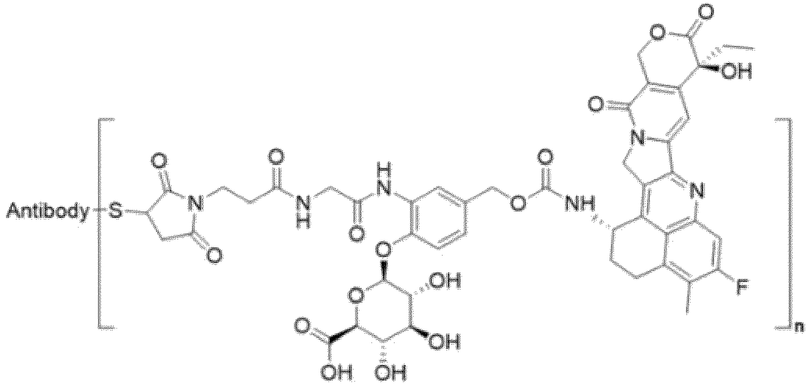
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.
**Embodiment: 36** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 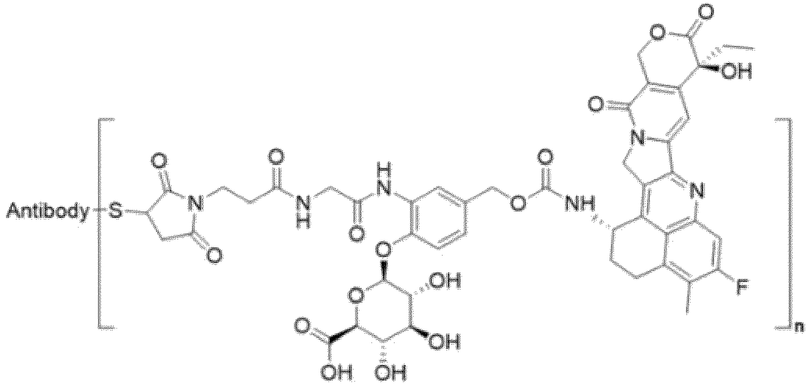
   wherein "n" represents an average drug-to-antibody ratio (DAR), wherein n is 3.5 to 4.5,
   wherein the "S" is a sulfur atom of a cysteine of the bispecific antibody, wherein the cysteine of the bispecific antibody is one of the cysteines capable of forming an interchain disulfide bond.
**Embodiment: 37** An immunoconjugate comprising
   (a) a bispecific antibody that binds to EGFR and MUC1 comprising an EGFR binding portion and a MUC1 binding portion,
   (b) a growth inhibiting agent, and
   (c) a linker covalently linking (a) and (b),

   wherein the bispecific antibody comprises
      - a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
      - a second polypeptide comprising a second Fc domain and a heavy chain of a Fab fragment comprising a VH of the EGFR binding portion, wherein said second polypeptide comprises the amino acid sequence of SEQ ID NO: 18, and
      - a third polypeptide comprising a light chain of the Fab fragment comprising a VL of the EGFR binding portion, wherein said third polypeptide comprises the amino acid sequence of SEQ ID NO: 19,
   wherein the growth inhibiting agent is exatecan, and the linker is a β-glucuronide linker comprising a maleimide moiety, wherein the immunoconjugate has the following formula **(II):** 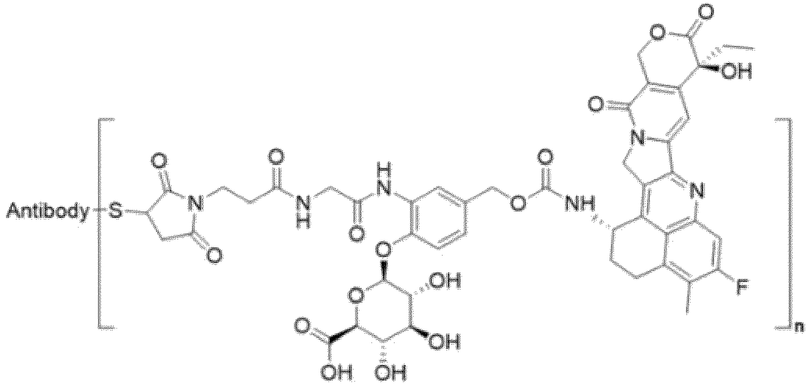
   wherein "n" represents an average drug-to-antibody ratio (DAR), wherein n is 3.8 to 4.2,
   wherein the "S" is a sulfur atom of a cysteine of the bispecific antibody, wherein the cysteine of the bispecific antibody is one of the cysteines capable of forming an interchain disulfide bond.

The skilled person would understand that combinations of the listed embodiments with other features described in the disclosure are of course, also included herein.

### Examples

### Summary of molecules used in the Examples

**Table 1** below provides a summary of abbreviations used in the examples to denote antibodies and immunoconjugates.

**Table 1 - summary of abbreviations used in the examples**

| **Antibody (mAb), Drug Linker Compound (DL), or Immunoconjugate (ADC)** | **Description** |
|---|---|
| **EGFR/MUC-MAB1** | **EGFR/MUC-MAB1** is an EGFR and MUC1 bispecific antibody as described in WO_2021247798_A1 (sequences numbers 3, 11 and 12). The EGFR is a Fab fragment and the MUC1 is an scFV. |
| | It comprises a first polypeptide comprising a first Fc domain and the single-chain Fv fragment (scFv) comprising the MUC1 VH and VL. |
| | It comprises a second polypeptide comprising a second Fc domain and a heavy chain of the Fab fragment comprising the EGFR VH. |
| | It comprises a third polypeptide comprising a light chain of the Fab fragment comprising the EGFR VL |
| | The bispecific antibody is based on a strand- exchange engineered domains **(SEED)-based** CH3 heterodimer platform as described in WO_2021247798_A1 and WO2022/128716 - the so-called "seed backbone". |
| | Produced in cell-free expression system. |
| **EGFR/MUC-MAB2** | Same as **EGFR/MUC-MAB1** and further: |
| | • The unnatural amino acids were replaced according to the canonical IgG1 amino acids, K216 in the heavy chain by the canonical E and the N-terminal methionines removed. |
| | • In order to reduce immune effector functions, the three mutations L234A/L235A/P329G (-> PGLALA) were included resulting in the sequence of **EGFR/MUC-MAB2.** |
| | Produced in CHO cells. |
| **EGFR/MUC-MAB3** | Same as **EGFR/MUC-MAB2** and further: |
| | • C226S mutation |
| | Produced in CHO cells. |
| **EGFR/MUC-MAB4** | EGFR/MUC-MAB4 is an EGFR and MUC1 bispecific antibody. |
| | It comprises an EGFR (VH) of SEQ ID NO: 13, and an EGFR (VL) of SEQ ID NO: 14. It comprises a MUC1 (VH) of SEQ ID NO: 15, and a MUC1 (VL) of SEQ ID NO: 16. |
| | The EGFR is a Fab fragment and the MUC1 is an scFV. |
| | It comprises a first polypeptide comprising a first Fc domain and the single-chain Fv fragment (scFv) comprising the MUC1 VH and VL. |
| | It comprises a second polypeptide comprising a second Fc domain and a heavy chain of the Fab fragment comprising the EGFR VH. |
| | It comprises a third polypeptide comprising a light chain of the Fab fragment comprising the EGFR VL |
| | The Fc domains are engineered to comprise **knob into hole substitutions (KiH).** The first polypeptide comprises T366W knob substitution. The second polypeptide comprises T366S, L368A, and Y407V hole substitutions. |
| | The Fc domains further comprise |
| | • a C226S substitution, |
| | • a L234A/L235A/P329G -> PGLALA Mutation. |
| | Produced in CHO cells. |
| **EGFR/MUC-MAB5** | Same as **EGFR/MUC-MAB4** and further: |
| | • The first polypeptide comprises a further S354C knob substitution. |
| | • The second polypeptide comprises a further Y349C hole substitution. |
| | Produced in CHO cells. |
| **EGFR/MUC-MAB6** | Same as **EGFR/MUC-MAB5** and further: |
| | • PGLALA was changed to LALA mutation |
| | • D356E and L358M substitution was introduced. |
| | Details: |
| | EGFR/MUC-MAB6 is an EGFR and MUC1 bispecific antibody. |
| | It comprises an EGFR (VH) of SEQ ID NO: 13, and an EGFR (VL) of SEQ ID NO: 14. It comprises a MUC1 (VH) of SEQ ID NO: 15, and a MUC1 (VL) of SEQ ID NO: 16. |
| | The EGFR is a Fab fragment and the MUC1 is an scFV. |
| | It comprises a first polypeptide comprising a first Fc domain and the single-chain Fv fragment (scFv) comprising the MUC1 VH and VL (SEQ ID NO: 17) |
| | It comprises a second polypeptide comprising a second Fc domain and a heavy chain of the Fab fragment comprising the EGFR VH (SEQ ID NO:18) |
| | It comprises a third polypeptide comprising a light chain of the Fab fragment comprising the EGFR VL (SEQ ID NO: 19) |
| | The Fc domains are engineered to comprise **knob into hole substitutions (KiH).** The first polypeptide comprises T366W and S354C substitutions knob substitutions. The second polypeptide comprises T366S, L368A, Y407V and Y349C hole substitutions. |
| | The Fc domains further comprise |
| | • a C226S substitution, |
| | • a L234A, and L235A substitution (LALA substitution), and |
| | • D356E and L358M substitution. |
| | Produced in CHO cells. |
| **Drug Linker Compound (DL)** | Maleimide- β-glucuronide -exatecan |
| | 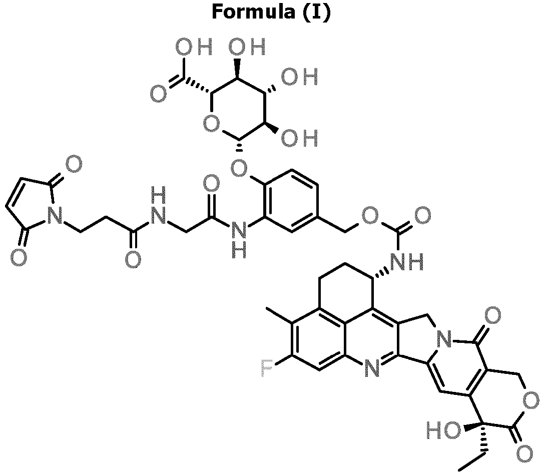 |
| **EGFR/MUC-ADC1** | EGFR/MUC-MAB1 is conjugated to vc-hemiasterlin with a DAR of 4 (for details see e.g. WO 2022/128716 A1, WO 2021/247798A1 or US 2023/0310629 A1) |
| **EGFR/MUC-ADC3** | EGFR/MUC-MAB3 conjugated to Drug Linker Compound (Formula (I) - Maleimide- β-glucuronide -exatecan) |
| **EGFR/MUC-ADC4** | EGFR/MUC-MAB4 conjugated to Drug Linker Compound (Formula (I) - Maleimide- β-glucuronide -exatecan) |
| **EGFR/MUC-ADC5** | EGFR/MUC-MAB5 conjugated to Drug Linker Compound (Formula (I) - Maleimide- β-glucuronide -exatecan) |
| **EGFR/MUC-ADC6** | EGFR/MUC-MAB6 conjugated to Drug Linker Compound (Formula (I) - Maleimide- β-glucuronide -exatecan) |

### Example 1 - Drug Linker compound Formula (I) synthesis

Synthesis of a drug-linker compound with glucuronide-based linker of formula (I) of Table 1: Drug-linker compound 9 in this example:

### Protocol of chemical preparation

### Step 1: Compound 1

### Step 1: Compound 1

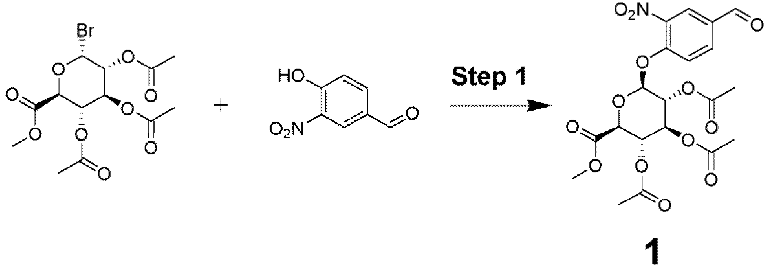

To a stirred solution of (2S,3S,4S,5R,6R)-3,4,5-Triacetoxy-6-bromo-tetrahydropyran-2-carboxylic acid methyl ester (8.30 g; 20.90 mmol; 1.00 eq.) and 4-Hydroxy-3-nitro-benzaldehyde (5.24 g; 31.35 mmol; 1.50 eq.) in Acetonitrile (83.00 ml; 10.00 V) was added Silver(I) oxide (9.69 g; 41.80 mmol; 2.00 eq.). The reaction mixture was stirred at RT for 16 h.

The reaction mixture was filtered through celite. The filtrate was concentrated under vacuum to get solid. The solid was dissolved in EtOAc and washed with 10% aqueous solution of NaHCCh to remove excess 4-Hydroxy-3-nitro-benzaldehyde. The organic layer was concentrated under vacuum to get compound 1 as sand colour solid.
- Yield: 9.0 g
- Percentage Yield: 89.1%
- Analytical data:
NMR: ¹H-NMR (400 MHz, DMSO-d6): 9.98 (s,1 H), 8.46 (s, 1 H), 8.25-8.21 (m, 1 H),7.64 (d, J = 11.60Hz, 1 H), 5.94 (d, J= 10.00 Hz, 1 H), 5.51-5.44 (m, 1 H), 5.20-5.09 (m, 2H),4.80 (d, J = 13.20 Hz, 1 H), 3.64 (s,3H), 2.09 (s, 9H).

### Step 2: Compound 2

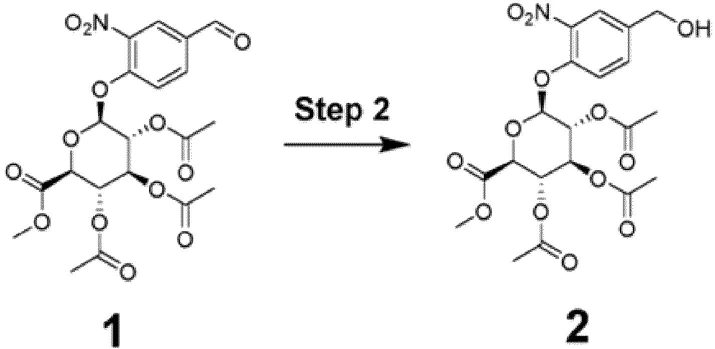

To a stirred solution of compound 1 (9.00 g; 18.62 mmol; 1.00 eq.) in Propan-2-ol (33.00 ml; 3.67 V) and CHC (167.00 ml; 18.56 V) were added silica gel 60-120 (3.60 g; 112.09 mmol; 6.02 eq.) followed by sodium borohydride (1.80 g; 46.55 mmol; 2.50 eq.). The reaction mixture was stirred for 1 h at RT. After completion, the reaction mixture was quenched with cooled H2O and filtered through celite. The filtrate was extracted with Dichloromethane and dried over Na2SC>4. The solvent was concentrated to get compound 2 as off-white powder.
- Yield: 8.70 g
- Percentage Yield: 92.4%
- Analytical data

LCMS: Column: ATLANTIS dC18 (50x4.6mm) 5 pm; Mobile phase A: 0.1 % HCOOH in H2O: ACN (95:5); B: ACN
RT (min): 2.05; M+H: 503.2, Purity: 96.6%

### Step 3: Compound 3

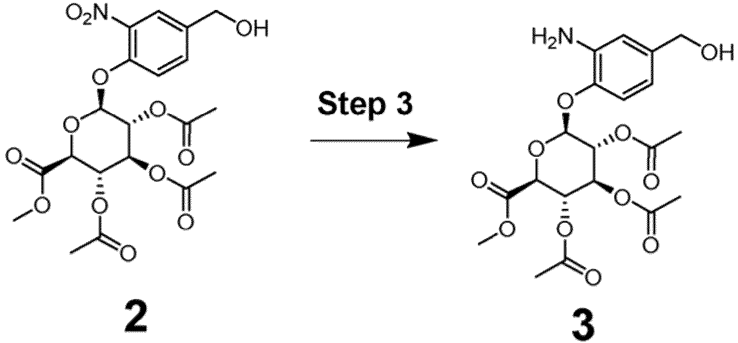

To a stirred solution of compound 2 (8.70 g; 17.21 mmol; 1.00 eq.) in ethyl acetate (100.00 ml; 11.49 V) and THF (100.00 ml; 11.49 V) was added Palladium on carbon (10% w/w) (2.50 g; 2.35 mmol; 0.14 eq.). The reaction mixture stirred for 3 h at RT under hydrogen atmosphere. After completion, the reaction mixture was filtered off through celite. The solvent was concentrated under vacuum to get compound 3 as off-white solid.
- Yield: 8.5 g
- Percentage Yield: 100%
- Analytical data:

LCMS: Column: ATLANTIS dC18 (50x4.6mm) 5 pm; Mobile phase A: 0.1 % HCOOH in H2O: ACN (95:5); B: ACN
RT (min): 1.73; M+H: 456.10, Purity: 95.1%

### Step 4: Compound 4

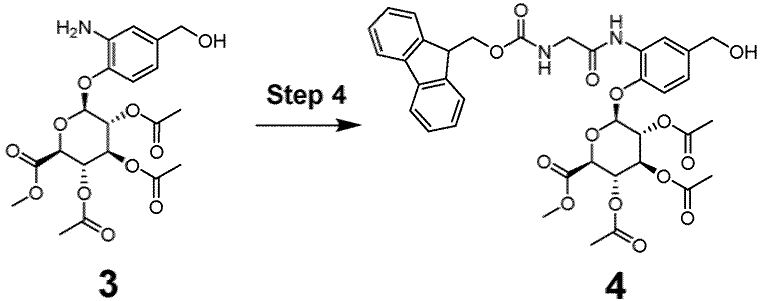

To a stirred solution of compound 3 (10.00 g; 20.89 mmol; 1.00 eq.) and (9H-Fluoren-9-ylmethoxycarbonylamino)-acetic acid (7.60 g; 25.06 mmol; 1.20 eq.) in DCM (250.00 ml; 25.00 V) was added 2-Ethoxy-2H-quinoline-1-carboxylic acid ethyl ester (15.65 g; 62.66 mmol; 3.00 eq.) at 0 °C. The reaction mixture was stirred for 16h at RT. After completion, solvent was removed under reduced pressure to get a crude product. The crude product was purified by column chromatography (56% EtOAc: petroleum ether) to get compound with purity 80%. The compound was purified further by washings with30% EtOAc and pet ether to get compound 4 as white solid.
- Yield: 8.5 g
- Percentage Yield: 50.7%
- Analytical data:

LCMS: Column: ATLANTIS dC18 (50x4.6mm) 5 pm; Mobile phase A: 0.1 % HCOOH in H₂O: ACN (95:5); B: ACN
RT (min): 3.03; M+H: 735.2, Purity: 81.9 %

### Step 5: compound 5

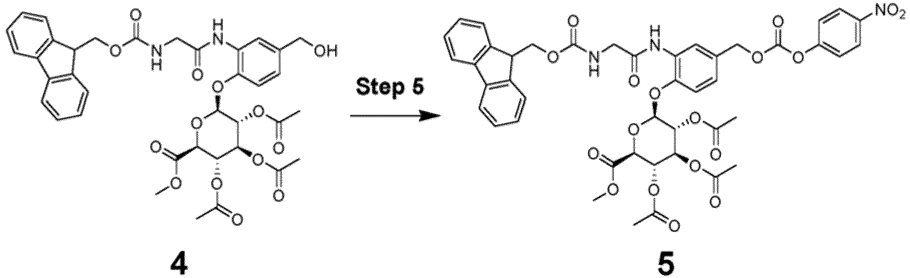

To a stirred solution of compound 4 (2.00 g; 2.49 mmol; 1 .00 eq.) in THF (40.00 ml; 20.00 V) at 0 °C, were added Carbonic acid bis-(4-nitro-phenyl) ester (3.06 g; 9.97 mmol; 4.00 eq.) and DI PEA (4.40 ml; 24.92 mmol; 10.00 eq.). The reaction mixture was stirred at RT for 12 h. After completion of the reaction, reaction mixture was concentrated under vacuum. The crude product was purified by column chromatography using silica gel (230-400) and pet ether I ethyl acetate as an eluent to afford compound 5 as pale yellow solid.
- Yield: 2.0 g
- Percentage Yield: 84.6%
- Analytical data:
LCMS: Column: X-Bridge C8(50X4.6) mm, 3.5pm; Mobile phase: A: 0.1% TFA in MilliQ water; B: ACN RT (min): 3.24; M+H: 900.20, Purity: 94.9%

### Step 6: Compound 6

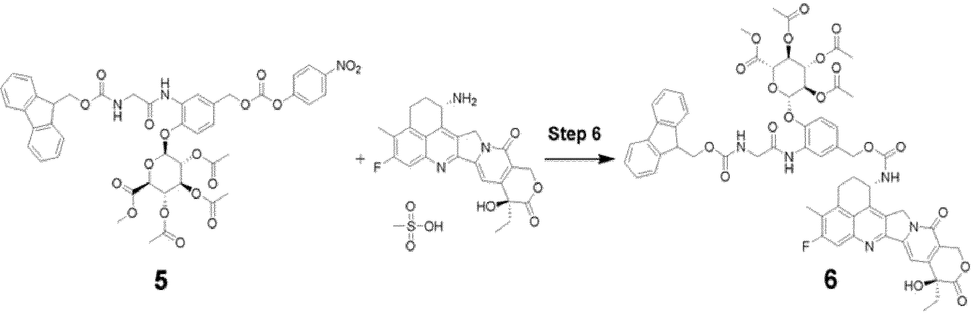

Compound 5 (1 ,369 g; 1 ,00 eq.) was dissolved in N,N-dimethylformamide (15,00 ml), Exatecan mesylate (679,7 mg; 1 ,00 eq.), 4-methylmorpholine for synthesis (0,422 ml; 3,00 eq.) and 1-Hydroxybenzotriazol (172,8 mg; 1 ,00 eq.) were added. The reaction mixture was stirred at room temperature for overnight. After the stirring time the reaction suspension was changed to a brown solution. The reaction was monitored by LC-MS, which showed a complete conversion of the starting material. The reaction mixture was purified via RP flash chromatography. The product containing fractions were combined, concentrated in vacuo and lyophilized overnight to afford compound 6 as an yellow solid.
- Yield: 1.59 g
- Percentage Yield: 87.5%
- Analytical data:

LCMS: Column: Chromolith HR RP-18e (50-4,6 mm); Mobile phase A: 0.05% HCOOH in H2O; B: 0.04% HCOOH and 1% H₂O in ACN; T: 40 °C; Flow: 3,3 ml/min; MS: 100-2000, amu positive; 1 % - >100% B: 0 ->2,0min; 100% B: 2,0 ->2,5 min
RT (min): 1.95; M+H: 1196.40, Purity: 84.4%.

### Step 7: Compound 7

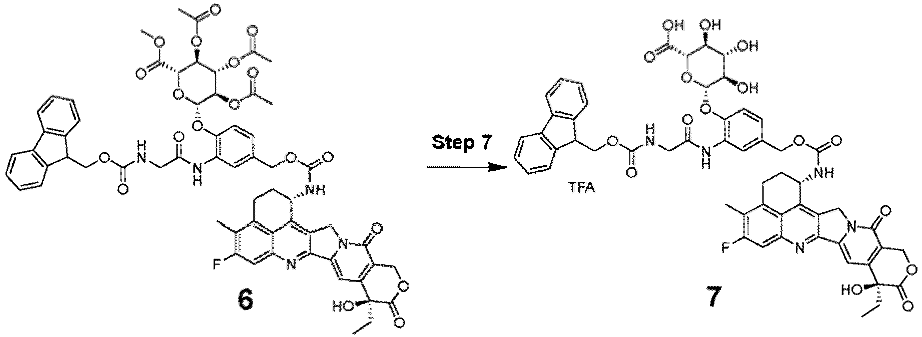

Compound 6 (1 ,586 g; 1 ,00 eq.) was dissolved in tetrahydrofuran (50,00 ml) and a solution (0.1M) of LiOH (contains Lithium hydroxide hydrate (281 ,77 mg; 6,00 eq.) in water (67,100 ml)) was added dropwise at 0°C. The pH value was checked during the addition. The pH should not exceed 10. The addition of the solution of LiOH was completed after 1.5 hours. The reaction was monitored by LC-MS, which showed a complete conversion of the starting material. The reaction was quenched with citric acid solution, pH adjusted to 5. The reaction mixture was concentrated under reduced pressure. The crude was purified by prep. HPLC.
The product containing fractions were combined and lyophilized to afford Compound 7 as a dark yellow solid.
- Yield: 728 mg
- Percentage Yield: 54.8%
- Analytical data:

LCMS: Column: Chromolith HR RP-18e (50-4,6 mm); Mobile phase A: 0.05% HCOOH in H2O; B: 0.04% HCOOH and 1% H₂O in ACN; T: 40 °C; Flow: 3,3 ml/min; MS: 100-2000, amu positive; 1 % - >100% B: 0 ->2,0min; 100% B: 2,0 ->2,5 min
RT (min): 1.68; M+H: 1056.30, Purity: 98.5%.

### Step 8: Compound 8

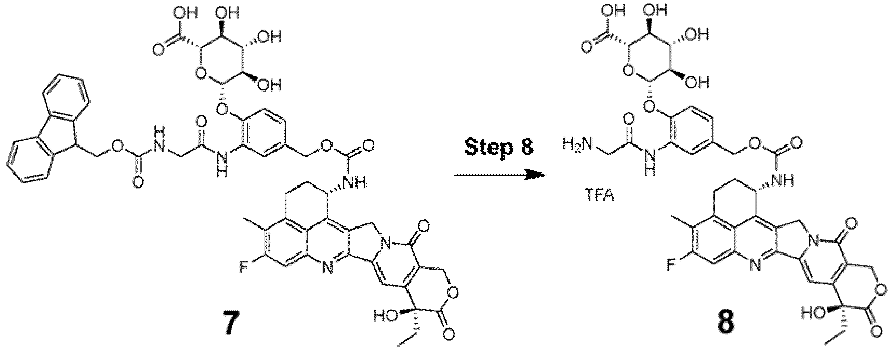

Compound 7 (728,000 mg; 1 ,00 eq.) was dissolved in N,N-dimethylformamide (20,00 ml). Piperidine (136,513 pl; 2,00 eq.) was added and the solution was stirred at RT for totally 4 hours. The reaction was monitored by LC-MS, which showed a complete conversion of the starting material. The reaction mixture was concentrated under reduced pressure and the crude product was purified by RP flash chromatography. The product containing fractions were combined, the solvent was removed partially and it was lyophilized overnight to afford compound 8 as an yellow solid.
- Yield: 706 mg
- Percentage Yield: 100%
- Analytical data:

LCMS: Column: Chromolith HR RP-18e (50-4,6 mm); Mobile phase A: 0.05% HCOOH in H₂O; B: 0.04% HCOOH and 1% H₂O in ACN; T: 40 °C; Flow: 3,3 ml/min; MS: 100-2000, amu positive; 1 % ->100% B: 0 ->2,0min; 100% B: 2,0 ->2,5 min
RT (min): 1.22; M+H: 834.30, Purity: 97.6%.

### Step 9: Compound 9

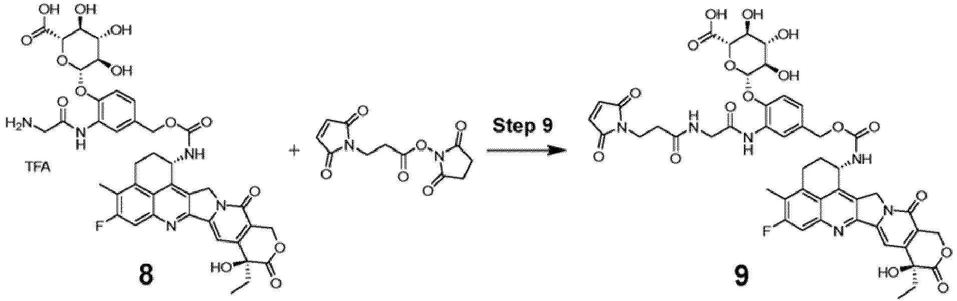

To a solution of compound 8 (854 mg; 1 ,00 eq.) in dimethylformamid (30,00 ml) were added N-ethyldiisopropylamine (149,234 pl; 1,00 eq.) and 3-(2,5-Dioxo-2,5-dihydro-pyrrol-1-yl)-propionic acid 2,5-dioxo-pyrrolidin-1-yl ester (233,61 mg; 1 ,00 eq.). The reaction mixture was stirred at RT for 3 hours. The reaction was monitored by LC-MS, which showed a complete conversion of the starting material. The reaction mixture was concentrated under reduced pressure and the crude product was by RP flash chromatography. The product containing fractions were combined, concentrated and lyophilized to give the desired produce with a purity of 91%. This material was again purified by RP chromatography to give compound 9 as an yellow solid.
- Yield: 580 mg
- Percentage Yield: 60.1%
- Analytical data:

LCMS: Column: Chromolith HR RP-18e (50-4,6 mm); Mobile phase A: 0.05% HCOOH in H2O; B: 0.04% HCOOH and 1% H₂O in ACN; T: 40 °C; Flow: 3,3 ml/min; MS: 100-2000, amu positive; 1 % ->100% B: 0 ->2,0min; 100% B: 2,0 ->2,5 min
RT (min): 1.38; M+H: 985.30, Purity: 90% (the other 10% of isomer can be removed by HPLC)
¹H NMR (500 MHz, DMSO-cfe) 6 13.10 - 12.44 (m, 1 H), 9.08 (s, 1 H), 8.32 (t, J = 5.8 Hz, 1 H), 8.16 (s, 1 H), 8.02 (d, J = 8.8 Hz, 1 H), 7.76 (d, J = 10.9 Hz, 1 H), 7.31 (s, 1 H), 7.15 - 7.09 (m, 2H), 6.98 (s, 2H), 5.48 - 5.38 (m, 2H), 5.32 - 5.22 (m, 3H), 5.11 - 5.01 (m, 2H), 4.87 (d, J = 7.6 Hz, 1 H), 3.92 - 3.88 (m, 1 H), 3.89 - 3.84 (m, 2H), 3.65 - 3.61 (m, 2H), 3.46 - 3.41 (m, 1 H), 3.42 - 3.37 (m, 1 H), 3.38 - 3.31 (m, 1 H), 3.28 - 3.20 (m, 1 H), 3.15 - 3.07 (m, 1 H), 2.48 - 2.44 (m, 2H), 2.38 (s, 3H), 2.24 - 2.13 (m, 2H), 1.94 - 1.80 (m, 2H), 0.88 (t, J = 7.3 Hz, 3H).

### Example 2 - Production of EGFR/MUC-MAB2 and EGFR/MUC-MAB3

EGFR/MUC-MAB2 and EGFR/MUC-MAB3 (see **Table 1,** above for summary) were produced via transient expression in ExpiCHO cells. The sequences for the three chains of the antibody were synthesized and cloned onto pTT5 plasmids. The plasmids were used in a ratio of 1:1:2 and 1:1:1 (heavy chain 1, heavy chain 2, light chain) for transfection, respectively. ExpiCHO cells were cultured in ExpiCHO Expression medium and transfected using ExpiFectamine^{™} reagent according to the manufacturer's instructions. For transfection, cells were seeded at a density of around 2.2 x 10^6 viable cells/ml approximately 24 hours prior to transfection. A total of 600 µg/l of DNA was used for transfection for 1000 ml of final culture volume. After 20 hours of incubation at 36.5 °C and 5% CO2, the culture was supplemented with ExpiCHO Enhancer and feed. The cells were harvested five days post-transfection, and the supernatant was collected by centrifugation at 4300 x g for 20 minutes at 4 °C.

The purification was performed using an ÄKTAxpress system chromatography system (Cytiva). Approximately 1 L of cell-free supernatant from ExpiCHO cells was loaded onto a HiTrap MabSelect SuRe 5 ml column equilibrated in PBS at pH 6.8. After washing, elution was conducted with 3 CV of 50 mM acetic acid at pH 3.0, followed by immediate neutralization to pH 6.8 using 1 M Tris at pH 8. The eluted fractions were then subjected to desalting and further purified using a HiLoad Superdex 200 pg column and PBS pH 6.8 as running buffer. Protein identity was confirmed by mass spectrometry and purity determined by analytical size exclusion chromatography (SE-HPLC) revealing 100% purity.

For EGFR/MUC-MAB3, cell-free supernatant from ExpiCHO cells was loaded onto a MabSelect SuRe column. Elution was conducted with 50 mM acetic acid at pH 3.2, followed by neutralization to pH 5.5 with 1 M Tris at pH 8. The neutralized pool was subsequently loaded onto a CIEX Eshmuno CPX column, with elution achieved through a linear gradient of NaCl. The concentrated pool was then loaded onto a HiLoad Superdex 200 pg column, eluting in 50 mM Histidine, 100 mM NaCl, pH 6.5. A final purification step on a Toyopearl Butyl-Sepharose column was performed, utilizing a gradient from 1.5 M to 0 M ammonium sulfate in 50 mM sodium phosphate pH 7.0 for elution. Finally, the antibody was formulated in 50 mM Histidine, 100 mM NaCl, pH 6.5 using a HiPrep 26/10 Desalting column.

The target protein fractions were pooled, filtered through a 0.2 µm membrane, snap frozen and stored at -80 °C. Final samples were analyzed using SE-HPLC, RP-HPLC, SDS-PAGE and mass spectrometry to confirm identity and purity revealing 100% purity for according to SE-HPLC for both antibodies.

EGFR/MUC-MAB1 is produced using the XpressCF+^{™} cell-free expression system which results in a lack of the typical antibody glycan structure, often associated with reduced antibody stability, but allows to introduce unnatural amino acids (UAA) into the antibody sequence. These UAAs serve as reactive attachments sites for the drug-linkers. EGFR/MUC-MAB1 has been described in WO 2022/128716 A1, WO 2021/247798A1 or US 2023/0310629 A1).

### Example 3- EGFR/MUC-MAB3 conjugation to yield EGFR/MUC ADC3

EGFR/MUC-MAB3 is based on the SEED platform for bispecific antibody generation as EGFR/MUC-MAB1 and EGFR/MUC-MAB2 (see **Table 1,** above).

To produce an EGFR/MUC-ADC3 of EGFR/MUC-MAB3 via conventional, established production methods, EGFR/MUC-MAB3 was expressed in mammalian cells. As EGFR/MUC-MAB1 of EGFR/MUC-ADC1 and EGFR/MUC-MAB2, it was based on the SEED (Strand Exchange Engineered Domain - Davis et al. 2010, Protein Engineering, Design and Selection, Volume 23, Issue 4, April 2010, Pages 195-202 https://doi.org/10.1093/protein/gzp094) backbone to generate bispecific antibodies. Mutations to reduce effector functions (L234A/L235A/P329G -> PGLALA) were included resulting in EGFR/MUC-MAB2 (see also **Table 1,** above for summary).

The selected conjugation strategy relied on reduction of the antibody's native interchain disulfide bonds followed by maleimide-driven conjugation to the resulting thiols. The targeted drug-to-antibody ratio (DAR) was 4 whereas EGFR/MUC-MAB2 had three disulfide bonds. Full reduction and conjugation would have led to DAR 6 ADCs, whereas partial reduction and conjugation would have resulted in a heterogenous mixture of different DAR species.

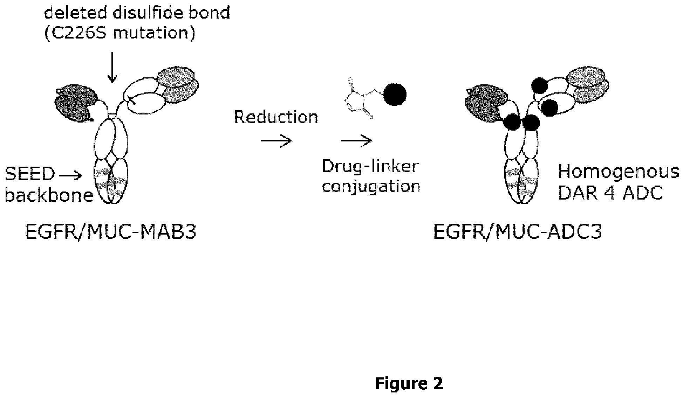

Therefore, the upper hinge disulfide bond was removed by mutating it to serine residues (C226S mutation in the heavy chain, according to EU numbering) resulting in EGFR/MUC-MAB3 (see **Figure 2**).

This strategy allowed to generate a site-specifically conjugated, homogenous DAR 4 ADC (**Figure 2**). The maleimide-glucuronide-exatecan drug-linker compound formula (I) was conjugated to the antibody resulting in EGFR/MUC-ADC3.

EGFR/MUC-MAB3 was initially subjected to mild reduction with 10 molar equivalents of tris(2-carboxyethyl)phosphine (TCEP) at a final antibody concentration of 5 mg/ml for 2 hours at 37 °C. Following this, 16 molar equivalents of drug-linker compound of formula (I) in Table 1, above, from a 20 mM stock solution in DMSO were added, and the conjugation mixture was incubated for 1 hour at 25 °C. The reaction was quenched by adding 25 equivalents of N-acetyl-L-cysteine.The crude mixture was then purified using a Superdex 200 16/60 (Cytiva) size-exclusion chromatography column with a running buffer of 50 mM Histidine, 100 mM NaCl, pH 6.5. Fractions corresponding to the ADC peak were pooled, concentrated using Amicon centrifugal devices (50 kDa cut-off), and buffer exchanged to the formulation buffer containing 10 mM Histidine, 40 mM NaCl, 6% Trehalose, 0.05% TWEEN 20, pH 5.5 using a HiPrep 26/10 Desalting Column. The final ADC, EGFR/MUC-ADC3, was sterile filtered (0.2 µm), snap-frozen, and stored at -80 °C.

Purity of ADCs was determined using analytical size-exclusion chromatography (SE-HPLC) using a Tosoh, TSKgel SuperSW3000, 4.6 x 300 mm column with isocratic elution using 50 mM NaP, 0.4 M Na_Perchlorate, pH 6.3 as solvent and 0.35 ml/min flow rate after injection of 5 µl sample. Absorption signals were recorded at 214 nm wavelength.

For DAR determination, LC-MS analysis was conducted using an Exion HPLC system, employing two buffers: Buffer A consisting of 0.1% formic acid in water and Buffer B consisting of 0.1% formic acid in acetonitrile. For reduced samples, ADCs were diluted to 0.05 mg/ml and reduced by adding 1 µl of a 500 mM TCEP solution to 5 µg of protein followed by 3 min incubation at room temperature. Samples were injected onto a bioZEN 3.6 µm Intact C4 column (2.1 x 50 mm, Phenomenex) with subsequent elution using a linear gradient from 15% to 95% Buffer B over a duration of 3 minutes, at a flow rate of 0.25 mL/min. Data acquisition was performed in positive ionization mode, covering a mass range from 400 to 4000 m/z. For MS analysis the Sciex 6600+ Triple-TOF was used with optimized instrument settings as follows: source voltage was set to 5.5 kV, declustering potential to 180 V, and accumulation time to 1 second. The source temperature was maintained at 350 °C, with gas1 and gas2 set to 50 L/h and 25 L/h, respectively, and curtain gas at 10 L/h. The collision energy (CE) was adjusted to 11. Calibration of the mass spectrometer was achieved using ESI positive calibration solution for the SCIEX X500B.Data acquired from the analysis were processed using Genedata Expressionist version 17.5. Spectra underwent smoothing via the moving average algorithm, followed by baseline subtraction using the quantile method. Deconvolution of the spectra was performed utilizing the maximum entropy method with 20 iterations at high quality. For protein mapping, a tolerance of 100 ppm was established, and variable modifications including glutamine to pyroglutamate conversion and C-terminal lysine loss were applied.

Concentration of ADCs was determined by UV-absorption spectroscopy measuring the absorbance at 280 nm and 360 nm wavelength. Concentration of the ADC was corrected for absorption of the drug-linker using formulas described by Chen et al (Chen Y. (2013) Drug-to-Antibody Ratio (DAR) by UV/Vis Spectroscopy. In: Ducry L. (eds) Antibody-Drug Conjugates. Methods in Molecular Biology (Methods and Protocols), vol 1045. Humana Press, Totowa, NJ) considering the theoretical molar extinction coefficient of the antibody at 280 nm and the experimental molar extinction coeffecient of the drug linker at both wavelengths.

ADCs were further characterized by RP-HPLC, SDS-PAGE and Endosafe PTS as described in WO 2023/170239 A1.

**Table 2: Key quality parameters of EGFR/MUC-ADC3**

| **EGFR/MUC-ADC3** | |
|---|---|
| **Concentration[mg/ml]** | 6.72 |
| **Drug-to-Antibody Ratio** | 4.07 |
| **Purity SEC [%]** | 97.62 |
| **Free drug content [%]** | 0.01 |
| **Endotoxin content [EU/mg]** | 0.63 |

### Example 4 - Pharmacokinetics of SEED-based antibodies and ADCs

The unconjugated antibody EGFR/MUC-MAB2 was tested in transgenic human FcRn TG276 mice and compared to the exatecan-carrying EGFR/MUC-ADC3.

EGFR/MUC-MAB1 and EGFR/MUC-ADC1 from cell free expression system and conjugation to unnatural amino acids as described in WO 2021/247798 A1were included as control.

The Pharmacokinetic studies in FcRn transgenic (276 model) mice were performed following single intravenous administration of 3 mg/Kg of the four molecules. Samples were taken 1, 6, 24, 48, 72, 96, 168, 240 and 336 hours after dosing for 6 animals per group.

Total antibody was measured as analyte using a generic anti human IgG MSD (Meso Scale Discovery) method. Data were pooled for PK analysis. Pharmacokinetic analyses were performed using Phoenix WinNonlin^{®} software (Pharsight Corporation, USA). A non-compartmental (model independent) approach was applied.

EGFR/MUC-MAB2 showed longer half-life compared to EGFR/MUC-MAB1. However, EGFR/MUC-ADC3 conjugated to interchain cysteines revealed a surprisingly poor pharmacokinetic behaviour indicated by high clearance and short half-life. This effect was not seen for EGFR/MUC-ADC1 compared to its parental antibody, EGFR/MUC-MAB1. The conjugation seemed to have a strong negative impact on the PK of EGFR/MUC-ADC3.

Table 3 below summarizes these pharmacokinetics results.

**Table 3: Pharmacokinetics of SEED-based antibodies and ADCs in transgenic human FcRn TG276 mice.**

| | **Drug-linker** | **Production** | **Bispecific format** | **DAR** | **ADC clearance (total mAb huFcRn Tg276 mice) [mL/h/kg]** | **t ½ [h]** |
|---|---|---|---|---|---|---|
| EGFR/MUC-MAB1 | - | Cell-free expression system | SEED | - | 4.07 | 27.0 |
| EGFR/MUC-ADC1 | DBCO-hemiasterlin (formula I WO 2021/247798 A1 | Conjugation to unnatural amino acids | SEED | 4 | 1.27 | 101 |
| EGFR/MUC-MAB2 | - | Recombinant CHO-cell expression | SEED | - | 3.04 | 67.9 |
| EGFR/MUC-ADC3 | Exatecan drug linker compound of formula (I) in Table 1 | Cysteine conjugation | SEED | 4.1 | 6.11 | 9.74 |

### Example 5 - KiH (knob-into-hole) design of EGFR/MUC-MAB4, EGFR/MUC-MAB5 - production and conjugation to yield EGFR/MUC-ADC4 and EGFR/MUC-ADC5

The conjugation strategy used leads to a complete reduction of disulfide bonds that connect the antibody chains. In monospecific IgG1 ADCs, the noncovalent interactions between the antibody CH3 domains are typically strong enough to hold the chains together, as fully reduced and conjugated antibodies are already established drugs for cancer treatment (e.g., trastuzumab deruxtecan). However, in bispecific formats, the interaction between the CH3 domains has been altered to allow for heterodimeric chain pairing.

The interaction between the SEED CH3 domains may not be strong enough for ADCs without covalent chain linkage, resulting in poor pharmacokinetic behaviour. As a result, an alternative format for bispecific antibodies was considered.

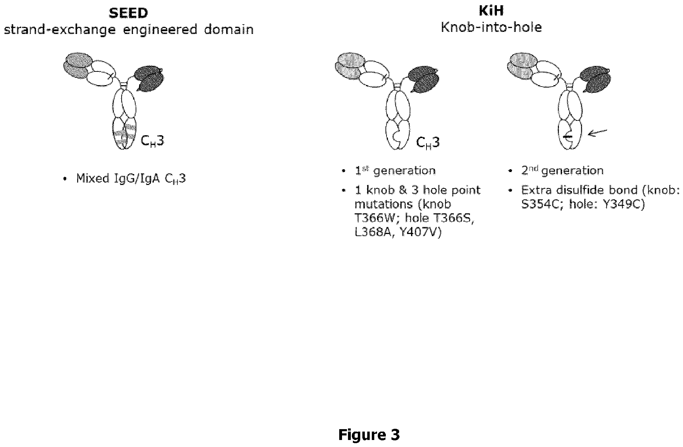

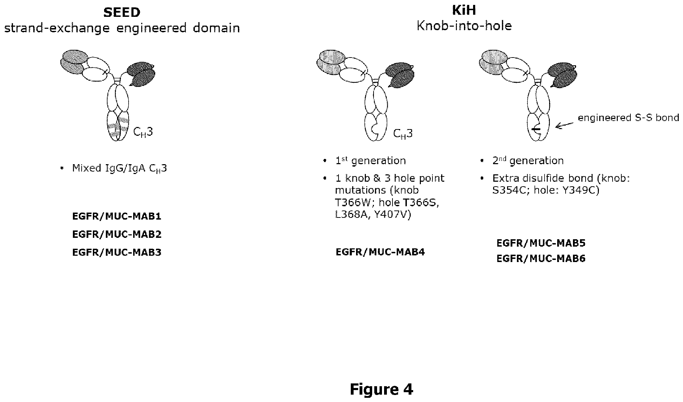

Therefore, two versions of the knob-into-hole platform were considered as alternatives. The second generation of this platform (KIH 2nd gen) includes an engineered disulfide bond in the CH3 part of the antibody. The original purpose of this bond was to increase the anticipated CH3 heterodimer. EGFRxMUC bispecific antibodies were designed based on EGFR/MUC-MAB3 including the deleted hinge cysteine but based on the two different knob-into-hole versions resulting in EGFR/MUC-MAB4 (first KIH generation) and EGFR/MUC-MAB5 (second generation) (see also **Table 1,** above for summary and **Figures 3** **and** **4**).

EGFR/MUC-MAB4 and EGFR/MUC-MAB5 were produced via transient expression in ExpiCHO cells. The sequences for the three chains of the antibody were synthesized and cloned onto pTT5 plasmids. The plasmids were used in a ratio of 1:1:2 and 3:1:1 (heavy chain knob, heavy chain hole, light chain) for transfection, respectively. ExpiCHO cells were cultured in ExpiCHO Expression medium and transfected using ExpiFectamine^{™} reagent according to the manufacturer's instructions. For transfection, cells were seeded at a density of around 2.2 x 10^6 viable cells/ml approximately 24 hours prior to transfection. A total of 600-800 µg of DNA was used for transfection for 1000 ml final culture volume. After 20 hours of incubation at 36.5 °C and 5% CO2, the culture was supplemented with ExpiCHO Enhancer and feed. The cells were harvested six days post-transfection, and the supernatant was collected by centrifugation at 4300 x g for 20 minutes at 4 °C.

EGFR/MUC-MAB4 was purified using an ÄKTAxpress system chromatography system (Cytiva). Cell-free supernatant was loaded onto a HiTrap MabSelect SuRe 5 ml column equilibrated in PBS at pH 6.8. After washing, elution was conducted with 50 mM acetic acid at pH 3.2, the pooled main peak immediately buffer exchanged to PBS pH 6.8 using a HiPrep Desalting 2.6/10 column and further purified using a HiLoad Superdex 200 pg column with PBS pH 6.8 as running buffer.

For EGFR/MUC-MAB5, cell-free supernatant was loaded onto a MabSelect SuRe column. Elution was conducted with 50 mM acetic acid at pH 3.2, followed by neutralization to pH 5.5 with 1 M Tris at pH 8. The neutralized pool was subsequently loaded onto CIEX Eshmuno CPX column after conductivity was adjusted to 3.7 mS/cm with water. Elution was achieved through a linear gradient from 0-0.5 M NaCl followed by 3 CV 0.5 M NaCl and 3 CV 1 M NaCl. The concentrated pool was then loaded onto a HiLoad Superdex 200 pg column using 50 mM Histidine, 100 mM NaCl, pH 6.5 as running buffer. For both antibodies, fractions were pooled. concentrated, 0.2 µm filtrated and aliquots flash frozen in liquid nitrogen and stored at -80°C.

EGFR/MUC-MAB4 and EGFR/MUC-MAB5 were conjugated with drug linker compound of formula (I) in Table 1 to generate EGFR/MUC-ADC4 and EGFR/MUC-ADC5, respectively, following the same procedure as for EGFR/MUC-ADC3.

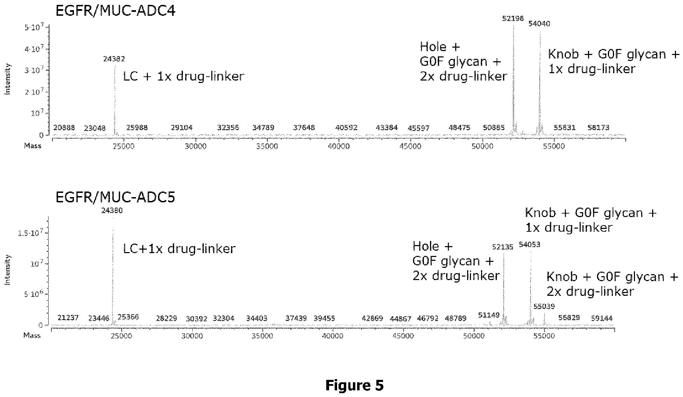

For LC-MS analysis, samples underwent an additional reduction step by adding 1 µl of a 500 mM TCEP solution to 5 µg of protein. An Exion HPLC system, utilizing buffer A (0.1% formic acid in water) and buffer B (0.1% formic acid in acetonitrile), was coupled to a Sciex 6600+ mass spectrometer equipped with a Turbo V ESI source. The protein solution was loaded onto a bioZEN 3.6 µm Intact C4, 2.1 x 50 mm column (Phenomenex) and eluted using a linear gradient from 15% to 95% buffer B over 3 minutes at a flow rate of 0.25 mL/min. The drug linker compound of formula (I) in Table 1 was conjugated to the individual chains based on the number of hinge and Fab-HC cysteines: once to the light chain, twice to the full heavy chain, and once to the knob heavy chain. A slight over conjugation was observed at the knob chain of EGFR/MUC-ADC5, potentially due to conjugation with a cysteine of engineered disulfide bond of KiH 2nd. Surprisingly, over conjugation was limited to this single species. **Figure 5** shows LC-MS analysis of reduced EGFR/MUC-ADC4 and EGFR/MUC-ADC5. +1, +2 refers to the number of attached to drug linker compound of formula (I) in Table 1.

**Table 4: Key quality parameters of EGFR/MUC-ADC4 and EGFR/MUC-ADC5**

| | EGFR/MUC-ADC4 | EGFR/MUC-ADC5 |
|---|---|---|
| Concentration[mg/ml] | 5.67 | 7.22 |
| Drug-to-Antibody Ratio | 3.96 | 4.09 |
| Purity SEC [%] | 94.9 | 97.4 |
| Free drug content [%] | 0.03 | 0.01 |
| Endotoxin content [EU/mg] | 0.41 | 0.38 |

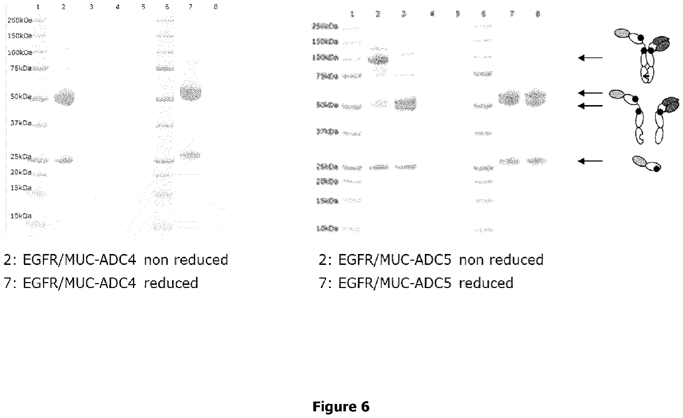

ADCs were analyzed using SDS-PAGE using the NuPAGE System (Invitrogen) according to the manufacturer's instructions (4-12% Bis-Tris Gels). Samples were applied after treatment at 70 °C for 10 minutes without and with addition of NuPage reducing agent. While the individual chains of EGFR/MUC-ADC4 separated under both conditions, the non-reduced EGFR/MUC-ADC5 showed a strong band at approximately 100 kDa, corresponding to the expected size of the covalently linked knob and hole heavy chains. **Figure 6** shows SDS PAGE analysis of EGFR/MUC-ADC4 and EGFR/MUC-ADC5.

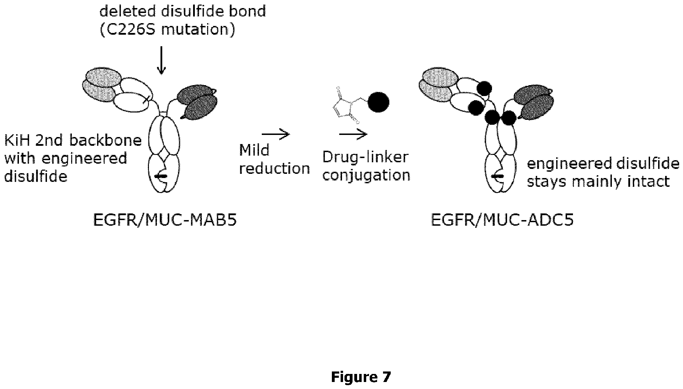

It was concluded that the engineered disulfide bond is surprisingly stable against mild reduction by TCEP under the conditions used for conjugation, thereby effectively covalently linking the two heavy chains in the final ADC (see **Figure 7**). Samples for LC-MS analysis were reduced at much higher TCEP concentrations, which ultimately led to the reduction of the engineered disulfide bond.

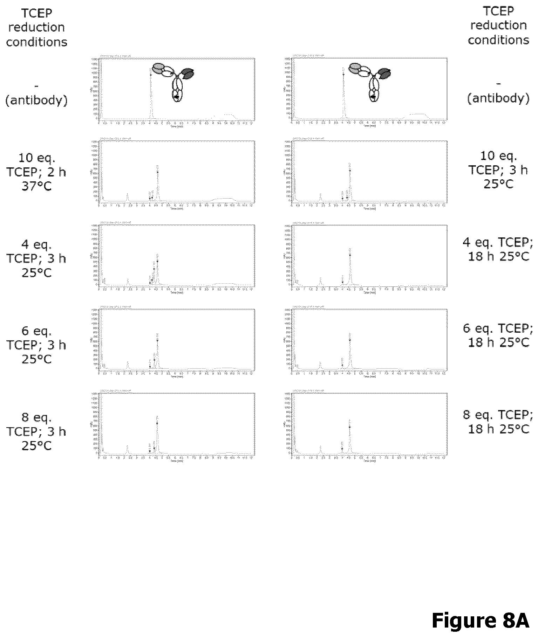

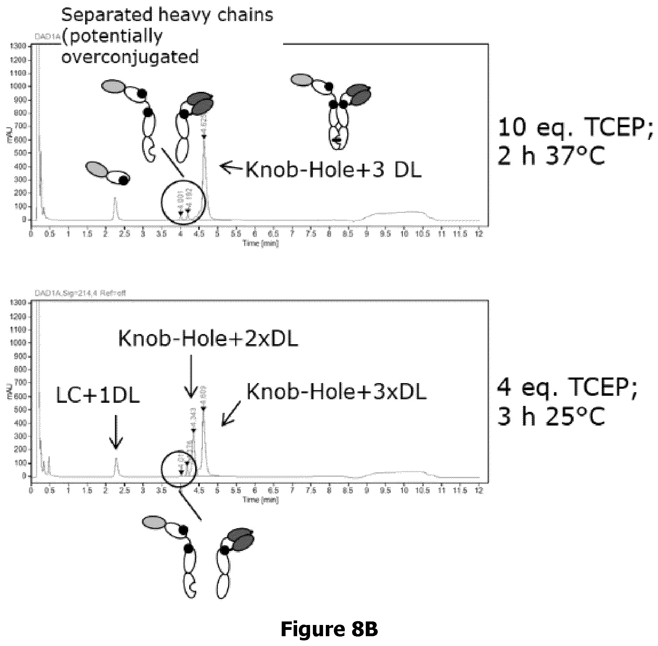

To further reduce the abundance of reduced, non-covalently connected heavy chains, the reduction conditions were optimized. EGFR/MUC-MAB5 was reduced at a concentration of 5 mg/ml under varying reaction conditions, including 4-10 molar equivalents of TCEP, at temperatures of 25 or 37 °C, for durations of 2 to 18 hours. Subsequently, the antibody was conjugated with 16 molar equivalents of a drug-linker and quenched with 25 molar equivalents of N-acetyl cysteine. The conjugated samples were then directly analyzed by reversed-phase HPLC using an Agilent PLRPS 2.1 x 50 mm, 5 µm column, employing a gradient where solvent A was Lichrosolv H2O + 0.1% TFA and solvent B was 100% acetonitrile + 0.1% TFA, with a gradient from 30% to 45% within 7.5 minutes at 65 °C and a flow rate of 1 ml/min. Increased concentrations of TCEP, reduction at 37 °C, and extended reduction times led to the formation of peaks corresponding to the separated knob and hole heavy chains, resulting from the reduction of the engineered disulfide. Conversely, insufficient reduction resulted in incomplete conjugation. Optimal conditions for achieving complete conjugation while minimizing the presence of separated heavy chains were identified as 6 to 8 equivalents of TCEP for 3 hours at 25 °C (**Figures 8A** **and** **8B**).

While both EGFR/MUC-ADC4 and EGFR/MUC-ADC5 can be successfully conjugated to the targeted DAR, EGFR/MUC-ADC5 benefits from the engineered disulfide, which stabilizes the ADC by maintaining the covalent linkage of the heavy chains. In addition to potential functional and structural advantages, this property significantly facilitates the analysis of EGFR/MUC-ADC5 in terms of assessing its heterodimeric structure, which is mandatory for the bispecific binding mode of the ADC. Several methods (e.g., SDS-PAGE, RP-HPLC, LC-MS) suitable to evaluate the chain composition run under conditions that disrupt non-covalent interactions. These methods result in the detection of individual chains only for EGFR/MUC-ADC4, whereas the correct assembly of EGFR/MUC-ADC5 can be analyzed much more easily. Furthermore, KIH2nd has been reported to form heterodimers more efficiently.

### Example 6 - In vivo studies to evaluate pharmacokinetics properties of EGFR/MUC-ADC4 and EGFR/MUC-ADC5

EGFR/MUC-ADC4 and EGFR/MUC-ADC5 were tested in transgenic humanized FcRn TG276 following single intravenous administration of 3 mg/Kg of the molecules. Samples were taken 1, 6, 24, 48, 72, 168, 240 and 336 hours after dosing for 6 animals per group (Study 21IV-0066).

Total antibody was measured as analyte using a generic anti human IgG MSD (Meso Scale Discovery) method. Data were pooled for PK analysis. Pharmacokinetic analyses were performed using Phoenix WinNonlin^{®} software (Pharsight Corporation, USA). A non-compartmental (model independent) approach was applied.

EGFR/MUC-ADC4 and EGFR/MUC-ADC5 demonstrated strongly improved PK behavior (longer T1/2; lower clearance values) compared the SEED based antibody EGFR/MUC-MAB2 and even more compared to the SEED-based EGFR/MUC-ADC3:

**Table 5: Pharmacokinetics of EGFR/MUC-ADC4 and EGFR/MUC-ADC5 in transgenic human FcRn TG276 mice.**

| | **Bispecific format** | **Type** | **DAR** | **ADC clearance (total mAb huFcRn Tg276 mice) [mL/h/kg]** | **t ½ [h]** |
|---|---|---|---|---|---|
| **EGFR/MUC-ADC4** | KiH 1st | ADC | 4.0 | 1.42 | 46.1 |
| **EGFR/MUC-ADC5** | KiH 2nd | ADC | 4.1 | 1.61 | 58.2 |

### Example 7 - Design and production and upscaling of knob-into-hole based exatecan EGFR/MUC-ADC6

Based on EGFR/MUC-MAB5, another KiH2nd based molecule by introducing mutations to switch the IgG allotype to G1m3 (D356E, L358M) and focus on the L234A, L235A mutations for immune effector silencing by changing G329 to proline. The resulting antibody has termed EGFR/MUC-MAB6.

Nucleotide sequences encoding for the individual chains were synthesized and cloned onto pGL3-based vectors for CHO-K1SV stable cell line generation. Cells were transfected and stable mini pools selected based on production titer, antibody heterodimer content and purity as well as clone stability.

The selected mini pool expressing EGFR/MUC-MAB6 was cultured under optimized conditions on a CHO fed-batch platform. Initially, a vial was thawed and cultured in MS-CHO-EM301 media supplemented with 5 µg/mL puromycin, maintaining a temperature of 36.5°C with 5% CO2 and 80% humidity. Seed expansion involved passaging every 2 to 3 days, diluting to a cell density of 0.3 × 10^6 cells/mL over a 14-day period, with cell viability consistently above 90%. The cells were transferred to a 50 liter production bioreactor, inoculated at a viable cell density of 0.20 × 10^6 cells/mL. The bioreactor was operated under controlled conditions, with pH regulated using CO₂ and Na₂CO₃, a medium volume of 35 liter, and an agitation rate of 100 rpm. Feeding strategies based on glucose concentration were implemented to optimize growth. After 13 days, the bioreactor contents were harvested and clarified through centrifugation at 4000 rpm for 15 minutes, followed by sterile filtration using a Millipore Express SHC Opticap XL10 filter processing. The final titer was 1397 mg/L and endotoxin levels below 0.01 EU/mg.

The clarified harvest was loaded onto Amsphere A3 resin after equilibration with TAP buffer (pH 7.0). The column was washed with TAP buffer at pH 7.0 and afterwards elution was performed using 1M acetic acid low pH buffer. Next, anion exchange purification was conducted using Eshmuno Q resin. The column was equilibrated with TAP buffer (20 mM Tris, 20 mM acetate, pH 8.5), the product loaded and eluted using a gradient of 0 to 2M NaCl in TAP buffer (20 mM Tris, 20 mM acetate, pH 8.5). Afterwards, cation exchange purification was performed using Fractogel COO- resin. Pre-equilibration was conducted with TAP buffer (50 mM Tris, 100 mM NaCl, pH 7.0), followed by equilibration at pH 5.5 using TAP buffer (10 mM Tris, 20 mM phosphate, 20 mM acetate, pH 5.5). An elution gradient was applied from 0% to 50% buffer B (1M NaCl in TAP buffer, pH 5.5) over 30 column volumes. The final product exhibited a titer of 22.1 g/L with acceptable impurity levels, the overall yield of the purification process was 69%.

EGFR/MUC-ADC6 was manufactured by conjugating exatecan drug linker compound of formula (I) in Table 1 to EGFR/MUC-MAB6. Reaction conditions were optimized in pre-experiments. Ultimately, 12 g antibody were diluted to 5 mg/mL in 50 mM Histidine and 100 mM NaCl, pH 6.5, and equilibrated to 25°C. TCEP was added at 7 molar equivalents and the reaction was incubated for 60 minutes at 25°C. 7 molar equivalents of drug linker compound of formula (I) was added from a 10 mM DMSO stock solution and the conjugation reaction was allowed to proceed for an additional 120 minutes at 25°C. The reaction was quenched by adding N-acetyl cysteine (NAC) at 7 molar equivalents.

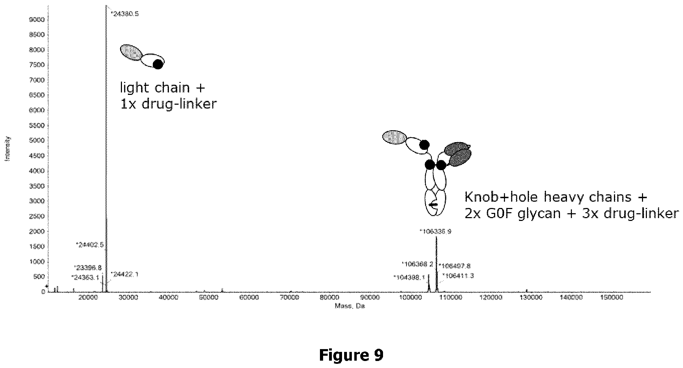

Purification of the ADC was performed using tangential flow filtration to remove excess toxin and low molecular weight impurities using the following parameters: 10DV with 80mM His, pH 5.5, concentration to ~20mg/mL, 10mM His pH 5.5; 0.11m2 Pellicon XL Ultracel 30kD TFF cassette, 110g/m² loading, 1.0 bar TMP, 5 LMM. The ADC was diluted with a second conditioning buffer to achieve a final formulation of 10mM His, 40mM NaCL, 6% Trehalose, 0.05% PS20, pH 5.5, sterile filtered (0.2 µm), snap frozen at stored at -80 °C. Analytical methods employed, i.a., SE-HPLC, RP-HPLC, and LC-MS. Concentration was determined using UV-absorption spectroscopy measuring the absorption at 280 nm and multiplied by a correction factor of 0.91 to correct by the absorption of the drug-linker. Non-reducing LC-MS analysis demonstrated that the knob and hole heavy and light chains were covalently linked, confirming selective disulfide reduction and integrity of the engineered disulfide bond (see **Figure 9**).

**Table 6: Key quality parameters of EGFR/MUC-ADC6**

| | **EGFR/MUC-ADC6** |
|---|---|
| | Analysis Before Freeze Thaw |
| DAR | 3.9 |
| SEC %monomer | 93.9 |
| Endotoxin (EU/mg) | <0.320 |
| Free Toxin % | 0.9 |
| Concentration (mg/ml) | 13.7 |

| | Analysis After Freeze Thaw |
|---|---|
| SEC %monomer | 94.1 |

### Example 8 - In vivo studies to compare pharmacokinetic properties of EGFR/MUC-ADC6 and EGFR/MUC-ADC1

EGFR/MUC-ADC6 was characterized in transgenic humanized FcRn TG32 mice PK profile.

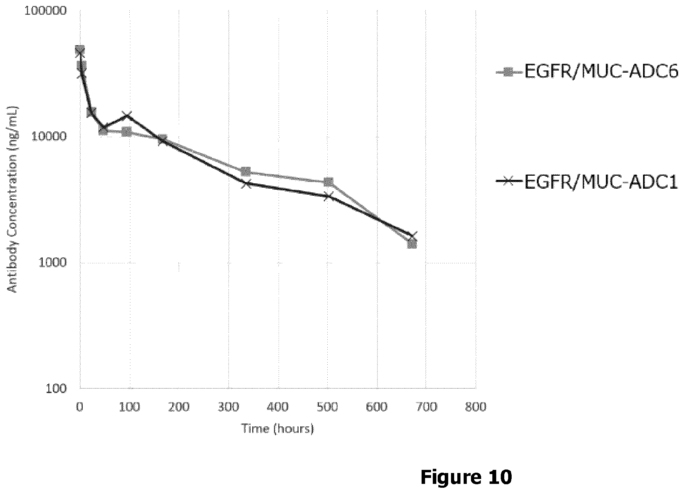

The PK study was performed following single intravenous administration of 3 mg/Kg of the molecules. Samples were taken 0.167, 4, 24, 48, 96, 168, 336, 504 and 672 h after dosing for 9 animals per group. Total antibody was measured as analyte using a generic anti human IgG MSD (Meso Scale Discovery) method. Data were pooled for PK analysis. Pharmacokinetic analyses were performed using Phoenix WinNonlin^{®} software (Pharsight Corporation, USA). A non-compartmental (model independent) approach was applied. EGFR/MUC-ADC6 revealed a similar PK profile compared to EGFR/MUC-ADC1 (see **Figure 10** and **Table 7).**

**Table 7: Pharmacokinetics of EGFR/MUC-ADC6 and EGFR/MUC-ADC1 in transgenic human FcRn TG32 mice.**

| MBE | t1/2 (h) | *Cl (mL/h/kg) |
|---|---|---|
| **EGFR/MUC-ADC6** | 217 | 0.575 |
| **EGFR/MUC-ADC1** | 215 | 0.599 |

### Example 9 - Target binding of EGFRxMUC1 bispecific ADCs

Binding kinetics of EGFR/MUC-ADC6 and EGFR/MUC-ADC1 to recombinant extracellular domain of human and cynomolgus monkey (*Macaca fascicularis*) EGFR were determined by biolayer interferometry.

Dissociation constants (KD) were determined as 1.67 nM for EGFR/MUC-ADC6 and 1.4 nM for EGFR/MUC-ADC1 against the human target and 3.87 and 2.07, respectively against the monkey target.

Binding kinetics of EGFR/MUC-ADC6 to human MUC1 were determined by SPR (Biacore) using a fusion protein of a human MUC1 VNTR domain fused to a camelid VHH domain (expressed in E. coli, not glycosylated). EGFR/MUC-ADC6 bound human MUC1 VNTR peptide, fitted with a 2 to 1 fitting model, with dissociation constants (KD) of 45.8 nM and 821.6 nM.

**Table 8: Binding kinetics of EGFR/MUC-ADC6 and EGFR/MUC-ADC1 to recombinant extracellular domain of human EGFR**

| **Ligand** | **analyte** | **KD (M)** | **KD (nM)** | | **kon(1/Ms)** | **kdis(1/s)** |
|---|---|---|---|---|---|---|
| Human EGFR-His SEQ ID NO: 55 | EGFR/MUC-ADC6 | 1.67E-09 | 1.67 | | 6.15E+05 | 1.03E-03 |
| Cyno EGFR-His SEQ ID NO: 56 | EGFR/MUC-ADC6 | 3.87E-09 | 3.87 | | 4.50E+05 | 1.74E-03 |
| Human EGFR-His SEQ ID NO: 55 | EGFR/MUC-ADC1 | 1.39E-09 | 1.39 | | 5.75E+05 | 7.99E-04 |
| Cyno EGFR-His SEQ ID NO: 56 | EGFR/MUC-ADC1 | 2.07E-09 | 2.07 | | 5.26E+05 | 1.09E-03 |

**Table 9: Binding kinetics of EGFR/MUC-ADC6 to recombinant human MUC1 VNTR domain fused to a camelid VHH domain**

| **Analyte** | **Ligand** | **Mean KD1 (nM)** | **StD** | **Mean KD2 (nM)** | **StD** |
|---|---|---|---|---|---|
| EGFR/MUC-ADC6 | 14MUC_GGGS_VHH SEQ ID NO: 57 | 45.8 | 3.3 | 821.6 | 126.7 |

### Example 10 - Generation of mono-specific comparative molecules of different valency

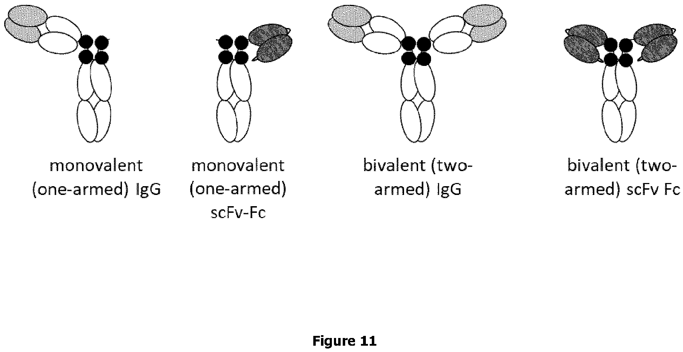

Monovalent (one-armed) and bivalent (two-armed) MUC1- and EGFR-targeting antibodies as well as isotype control antibodies were designed. Canonical interchain disulfide-forming cysteines were reduced to a total number of four where needed by mutating Fab cysteines to serine to allow site-specific conjugation of four cysteine-reactive compounds. Antibodies were recombinantly produced and conjugated with exatecan drug linker compound of formula (I) in Table 1 or with fluorescent dye maleimide-alexafluor 488 (Jena Bioscience Biochemicals) after TCEP reduction and purified by size-exclusion chromatography. See **Figure 11** and **Table 10** for a summary of comparative molecules.

### Example 11 - Cell binding of anti EGFRxMUC1 molecules

**Method:** Binding of maleimide-alexafluor488 conjugated molecules to cancer cell lines was assessed by flow cytometry. Cells were washed once, detached from culturing plates using Accutase (Sigma #A6964) and resuspended in culture medium. After centrifugation, the cell pellet was resuspended in ice-cold buffer (Gibco#14190-094)+ 3% FBS (Biochrom #S0615) + 1mM EDTA (0.5M, pH8, Invitrogen # AM9260G) + 0.05% sodium azide (Merck # 1.06688.0250, 10% -10g/100ml MilliQ. Cell suspensions were transferred to 96-well plates, pelleted by centrifugation and fluorescently labeled molecules were added and incubated for 30 min on ice in the dark. After washing PI solution (Invitrogen #P3566, end concentration: 5 µg/ml in ice-cold buffer) was added and fluorescence of cells was measured on a FACS Canto II.

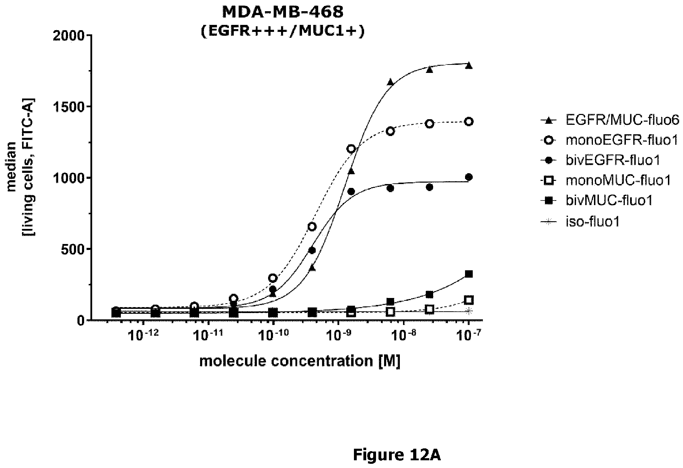

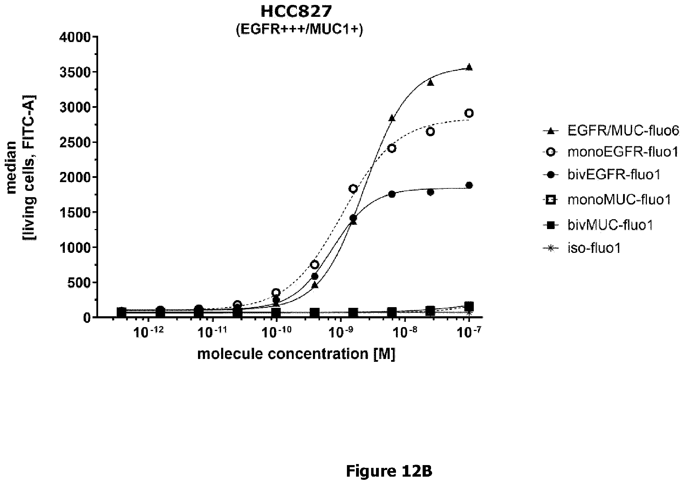

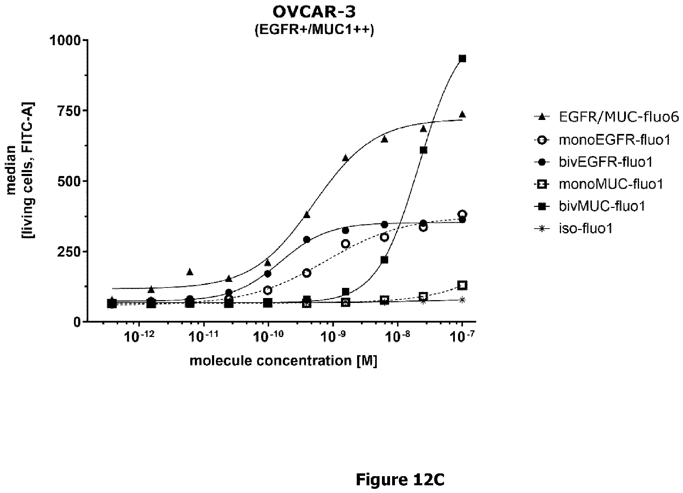

Binding to different cancer cell lines was assessed in flow cytometry experiments using fluorescently labelled molecules. Increased binding capacities were observed forof the bispecific, EGFR/MUC-fluo6 compared to the respective one-armed and two-armed MUC1 or EGFR-targeting monospecific molecules (except for bivalent MUC1-fluo1 on MUC1++ OVCAR3 cells at high concentrations) was observed (see **Figures 12A****,** **12B****,** and **12C**).

**Table 10: Summary of mono-specific comparative molecules of different valencies**

| **Name** | **Description** | **Unconjugated antibody or conjugate** | **Conjugated compound** | **DAR and conjugation** | **SEQ ID No HC** | **SEQ ID No HC2** | **SEQ ID No LC** |
|---|---|---|---|---|---|---|---|
| **EGFR/MUC-fluo6** | Conjugate derived from EGFR/MUC-MAB6 | Conjugate | maleimide-alexafluor488 | DAR 4, site-specific* | SEQ ID NO: 17 | SEQ ID NO: 18 | SEQ ID NO: 19 |
| **monoEGFR** | monovalent (one-armed) anti EGFR IgG (KIH2) | Unconjugated | - | - | SEQ ID NO: 18 | SEQ ID NO: 40 | SEQ ID NO: 19 |
| **monoEGFR-ADC1** | | Conjugate | exatecan drug linker compound of formula (I) in Table 1 | DAR 4, site-specific* | | | |
| **monoEGFR-fluo1** | | Conjugate | maleimide-alexafluor488 | | | | |
| **monoMUC** | monovalent (one-armed) anti MUC1 scFv-Fc (SEED) | Unconjugated | - | - | SEQ ID NO: 41 | SEQ ID NO: 42 | - |
| **monoMUC-ADC1** | | Conjugate | exatecan drug linker compound of formula (I) in Table 1 | DAR 4, site-specific | | | |
| **monoMUC-fluo1** | | Conjugate | maleimide-alexafluor488 | | | | |
| **monoMUC** | monovalent (one-armed) anti MUC1 scFv-Fc (KIH2) | Unconjugated | - | - | SEQ ID NO: 43 | SEQ ID NO: 44 | - |
| **monoMUC-ADC2** | | Conjugate | exatecan drug linker compound of formula (I) in Table 1 | DAR 4, site-specific | | | |
| **bivEGFR** | bivalent (two-armed) anti EGFR IgG | Unconjugated | - | - | SEQ ID NO:45 | - | SEQ ID NO: 19 |
| **bivEGFR-ADC1** | | Conjugate | exatecan drug linker compound of formula (I) in Table 1 | DAR 4, site-specific* | | | |
| **bivEGFR-fluo1** | | Conjugate | maleimide-alexafluor488 | | | | |
| **bivMUC** | bivalent (two-armed) anti-MUC1 scFv-Fc | Unconjugated | - | - | SEQ ID NO: 46 | - | SEQ ID NO: 47 |
| **bivMUC-ADC1** | | Conjugate | exatecan drug linker compound of formula (I) in Table 1 | DAR 4, site-specific* | | | |
| **bivMUC-fluo1** | | Conjugate | maleimide-alexafluor488 | | | | |
| **iso-MAB1** | anti-digoxigenin isotype control IgG | Unconjugated | - | - | SEQ ID NO: 48 | - | SEQ ID NO: 49 |
| **iso-ADC1** | | Conjugate | exatecan drug linker compound of formula (I) in Table 1 | DAR 4, partial disulfide reduction | | | |
| **iso-fluo1** | | Conjugate | maleimide-alexafluor488 | | | | |
| **iso-MAB2** | anti-CD20 isotoype control IgG | Unconjugated | - | - | SEQ ID NO: 50 | - | SEQ ID NO: 51 |
| **iso-ADC2** | | Conjugate | exatecan drug linker compound of formula (I) in Table 1 | DAR 4, partial disulfide reduction | | | |
| **iso-fluo2** | | Conjugate | maleimide-alexafluor488 | | | | |
| *total number of interchain disulfide-forming cysteines reduced to four by mutating Fab cysteines to serine | | | | | | | |

### Example 12 - In vivo efficacy studies

### Example 12.1 EGFR/MUC-ADC1 v/s EGFR/MUC-ADC6

### Method - In vivo efficacy experiments

Anti-tumor efficacy of ADCs has been evaluated in several human cell-line-derived or patient-derived xenografts (CDX/PDX). In general, tumor cells or fragments were transplanted subcutaneously in the flank of immunodeficient female mice (athymic nude (Charles River), CD1 nude (Charles River), or BRG (Taconic)). When tumors reached a mean volume between 100-300 mm³, the mice were allocated in treatment groups with a similar mean tumor volume and variance (randomization) and were treated with the respective compound. Mice were either treated with a single dose intravenously (i.v.), or with an i.v. injection every 14 days for 3 treatments in total. Tumor length (L) and width (W) were measured with calipers and tumor volumes were calculated using the formula Lx(W^2)/2.

EGFR/MUC-ADC6 was evaluated in several patient derived or cell line-derived xenograft efficacy studies.

### Efficacy of EGFR/MUC-ADC6 in Squamous Cell Carcinoma Head and Neck (SCCHN):

Tumor fragments from evaluated PDX models were harvested from host animals and implanted into immune-deficient (athymic nude or CB-17 SCID) mice and studies initiated at a mean tumor volume of approximately 175-300 mm3 (XenoSTART, US). Mice were treated with a single dose (i.v.) of 8 mg/kg EGFR/MUC-ADC1 or EGFR/MUC-ADC6.

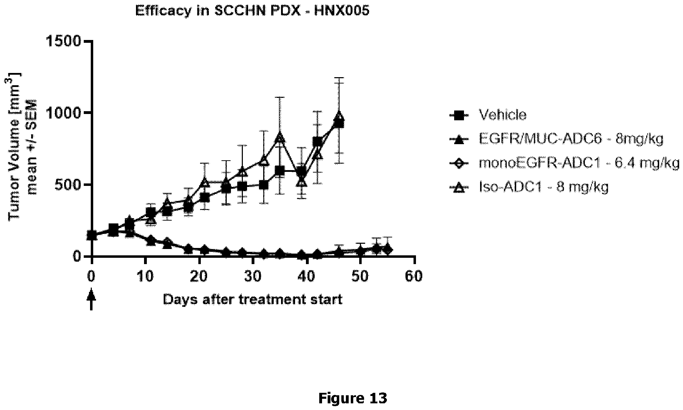

Results are shown in **Figure 13****.** No notable model-related weight loss was reported in the tested models based on lack of weight loss in control animals. In this study, single agent EGFR/MUC-ADC1 and EGFR/MUC-ADC6 were tolerated with a maximum weight loss of 9% in any group and no drug-related deaths; three total deaths occurred in these studies unrelated to drug tolerability.

EGFR/MUC-ADC1 showed in 5 models tumor regression (rel. Tumor growth <-66%), stable disease in 3 (rel. Tumor growth between -66% and +73%) and 2 progressive disease (rel. Tumor growth >73%) as assessed by best response (%tumor volume median).

EGFR/MUC-ADC6 showed a tumor regression in 4 models, stable disease in 5 models, and progressive disease in 1 model.

Overall, the efficacy of EGFR/MUC-ADC1 and EGFR/MUC-ADC6 are comparable in SCCHN PDX models.

### Efficacy of EGFR/MUC-ADC6 in Non-small cell lung cancer (NSCLC):

Tumor fragments from evaluated NSCLC model LUX003 were harvested from host animals and implanted into female NU/NU mice (Beijing Vital River Lab) and studies initiated at a mean tumor volume of approximately 175 (Chempartner, China). Mice were treated with a single dose (i.v.) of 4 mg/kg EGFR/MUC-ADC1 or 8 mg/kg EGFR/MUC-ADC6. Taking into consideration PK and the estimated clinical dose of EGFR/MUC-ADC1 with its tubulin inhibitor payload, 4 mg/kg was considered to be the relevant dose in xenograft studies.

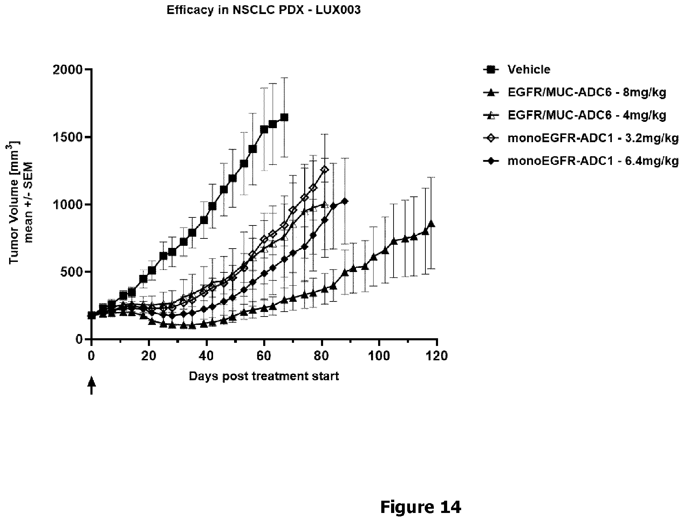

Results are shown in **Figure 14****.** Both ADCs show a similar efficacy in the NSCLC PDX model LUX003.

### Example 12.2 EGFR/MUC-ADC6 v/s one-armed EGFR ADCs

EGFR/MUC-ADC6 was compared against a one-armed EGFR-targeting ADC (monoEGFR-ADC1, same DAR and drug-linker as EGFR/MUC-ADC6) in in-vivo efficacy studies. Equimolar doses were applied to account for the different molecular weights, and single treatment (i.v. injection) was applied to all compounds.

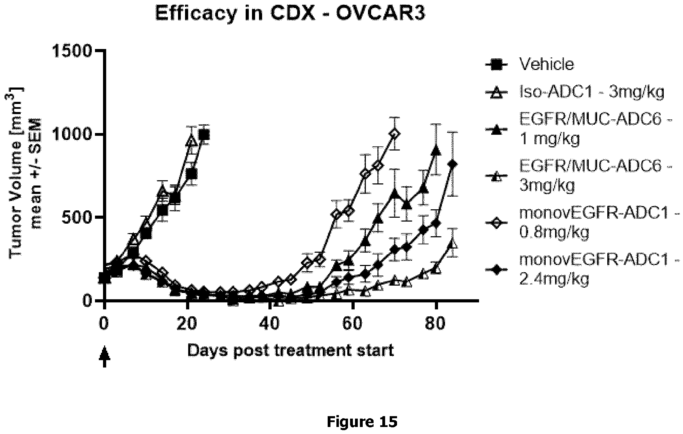

Surprisingly, EGFR/MUC-ADC6 showed enhanced efficacy in two out of three models (OVCAR3, LUX003) demonstrating the advantage of the bispecific mechanism of action. An isotype control ADC (irrelevant target, same DAR and drug-linker) did not show anti-tumor effects (see **Figures 14** **and** **15**)

Overall, comparison against one-armed EGFR ADC (same DAR and drug-linker) at equimolar doses in CDX and PDX in vivo efficacy studies, suggests that EGFR/MUC-ADC6 response is driven by its EGFR-arm while the MUC1-arm increases efficacy in certain MUC1-high expressing tumors.

### Example 12.3 EGFR/MUC-ADC6 v/s one-armed and two-armed EGFR-targeting ADC

EGFR/MUC-ADC6 was compared against a one-armed EGFR-targeting ADC (monoEGFR-ADC1, same DAR and drug-linker as EGFR/MUC-ADC6) and a two-armed EGFR-targeting ADC (bivEGFR-ADC1) in in-vivo efficacy studies. Equimolar doses were applied to account for the different molecular weights, and single treatment (i.v. injection) was applied to all compounds.

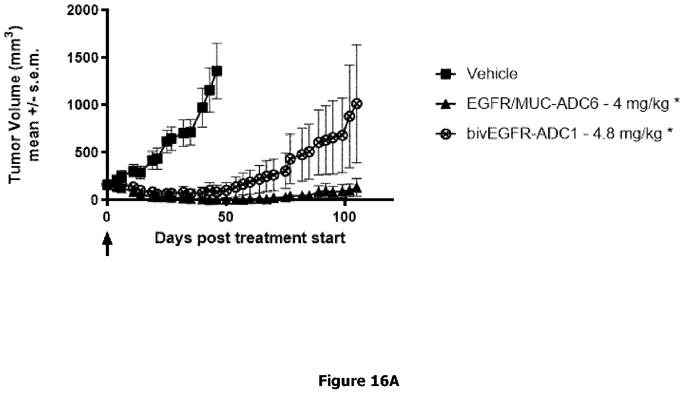

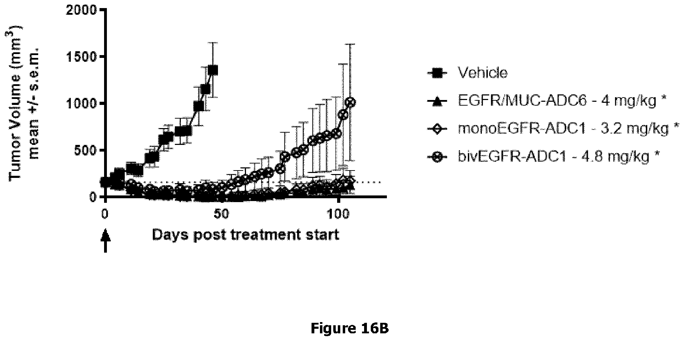

The SCCHN model HNX005 (with high EGFR expression) was chosen to assess whether a one-armed EGFR ADC would be inferior to a two-armed EGFR ADC. At equimolar doses of the compounds, a similar efficacy of all 3 tested compounds was observed. There was a slightly faster regrowth in bivEGFR-ADC1 (see **Figure 16**).

This suggests that a one-armed EGFR ADC is not inferior compared to a two-armed EGFR ADC in this in-vivo experiment.

### Example 13: Comparison of EGFR/MUC-ADC6 with EGFR-ADC-MMAE, EGFR/cMet-ADC, EGFR/HER3-ADC

Alternative EGFR-targeting ADCs with the following key properties were produced:
- **EGFR-ADC-MMAE:** Monospecific, bivalent anti-EGFR ADC conjugated at the antibody interchain cysteines with tubulin inhibiting drug-linker mc-vc-PAB-MMAE to a DAR of ~4 (antibody sequences and drug-linker structure according to WHO Drug Information, Vol. 38, No. 2, 2024 Proposed INN: List 131, Becotatug vedotin)
- **EGFR/cMet-ADC:** Bivalent, bispecific anti-EGFRxcMET ADC conjugated at the interchain cysteines with a topoisomerase-1 inhibitor drug-linker to a DAR of ~6 (antibody sequences and drug-linker structure according to WHO Drug Information, Vol. 37, No. 4, 2023 Proposed INN: List 130, Tilatamig samrotecan)
- **EGFR/HER3-ADC:** Tetravalent, bispecific anti-EGFRxcMET ADC conjugated at the interchain cysteines with a topoisomerase-1 inhibitor drug-linker to a DAR of ~8 (antibody sequences and drug-linker structure according to WHO Drug Information, Vol. 37, No. 2, 2023 Proposed INN: List 129, Zalontamab brengitecan)
- **Iso-ADC3:** Anti-digoxigenin isotype control IgG iso-MAB1 conjugated at the interchain cysteines with the same drug-linker as EGFR/HER3-ADC to a DAR of ~8

### Example 13.1 - Comparative PK study

The PK properties of EGFR/cMet-ADC and EGFR/HER3-ADC were characterized in transgenic humanized FcRn TG32 mice model.

The PK study was performed following single intravenous administration of 3 mg/Kg of the molecules. Samples were taken 0.167, 4, 24, 48, 96, 168, 336, 504 and 672 h after dosing for 9 animals per group. Total antibody was measured as analyte using a generic anti human IgG MSD (Meso Scale Discovery) method. Data were pooled for PK analysis. Pharmacokinetic analyses were performed using Phoenix WinNonlin^{®} software (Pharsight Corporation, USA). A non-compartmental (model independent) approach was applied.

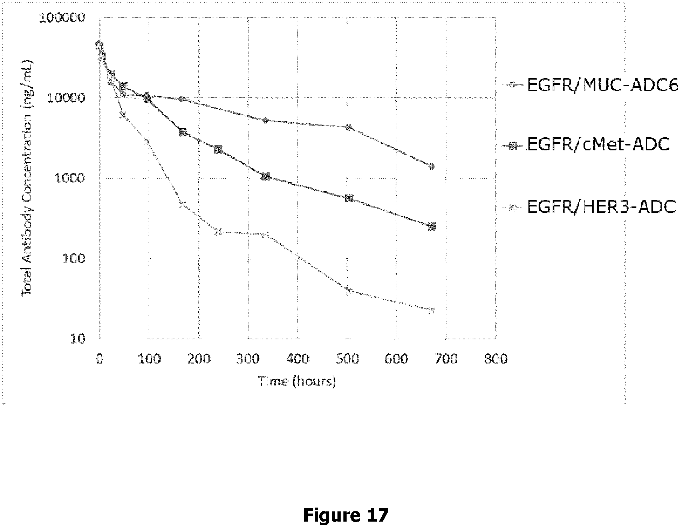

**Figure 17** shows the results. EGFR/MUC-ADC6 shows a better PK profile than EGFR/cMet-ADC and EGFR/HER3-ADC in PK studies in Tg32 mice (clearance 0.575, 1.13, 3.53 mL/hr/kg, respectively).

### Example 13.2 - In vivo comparison of EGFR/MUC-ADC6 vs EGFR-MMAE ADC

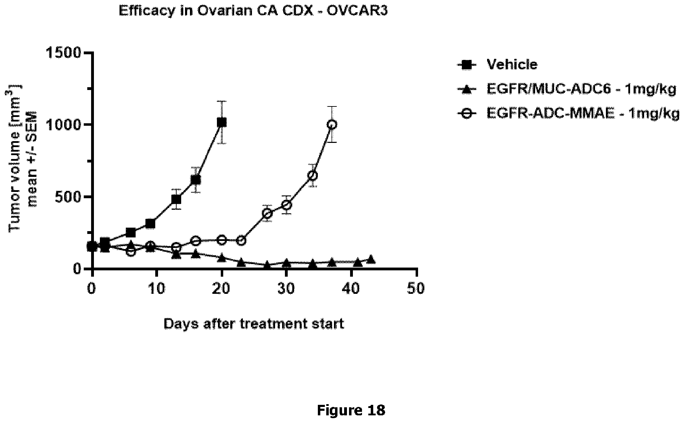

EGFR-ADC-MMAE and EGFR/MUC-ADC6 were compared at the same dose with single injection in the ovarian cancer CDX model OVCAR3. Surprisingly, a stronger and longer lasting anti-tumor effect of EGFR/MUC-ADC6 compared to the EGFR targeting ADC with MMAE payload was observed (see **Figure 18**).

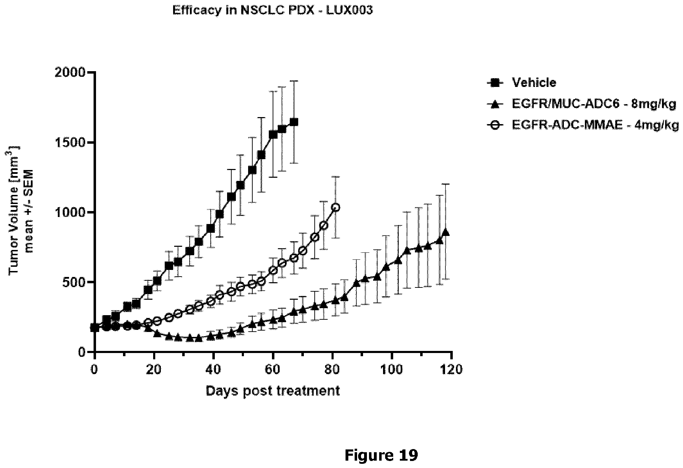

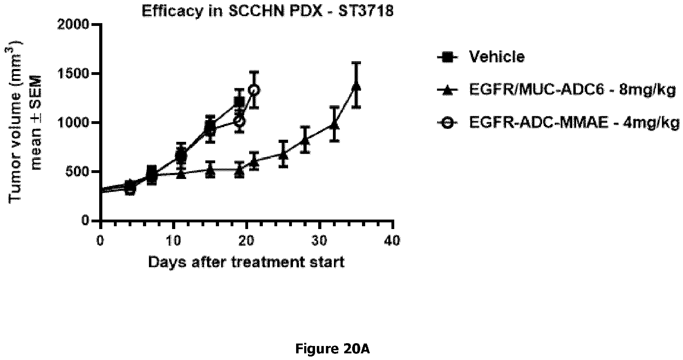

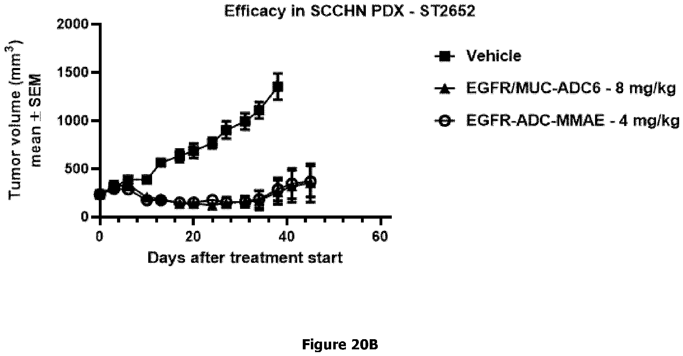

Further comparison of these two molecules in PDX SCCHN and NSCLC PDX showed a stronger effect in 2/4 models (LUX003 and ST3718) of EGFR/MUC-ADC6 compared to EGFR-ADC-MMAE. In one model ST2652, both ADCs showed very similar responses. The EGFR-ADC-MMAE showed a stronger effect in one model, ST497B compared to the EGFR/MUC-ADC6 (see **Figures 19****,** **20A****, and** **20B**).

Overall EGFR/MUC-ADC6 revealed a better efficacy in 3/5 models, compared to a EGFR-ADC-MMAE.

### Example 13.3 - In vitro comparison of EGFR/MUC-ADC6 vs. EGFR/HER3-ADC

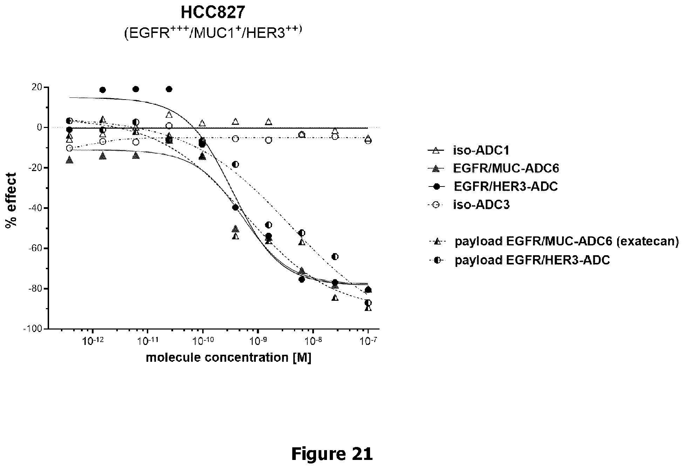

The in vitro potency of EGFR/MUC-ADC6 was compared to that of an EGFR/HER3 ADC (DAR8, TOP1i payload). In EGFR-high expressing HCC827 cells, which have low MUC1 and intermediate HER3 levels, both EGFR/MUC-ADC6 and EGFR/HER3-ADC exhibit comparable inhibitory effects, with IC50 values of 5.3E-10 for EGFR/MUC-ADC6 versus 3.5E-10 for EGFR/HER3-ADC, and inhibitory effects of -77.8% versus -78.4%, respectively (**Figure 21**).

The effect of EGFR/MUC-ADC6 and EGFR/HER3-ADC on cell viability was compared using the HCC827 cancer cell line. Additionally, cells were treated with respective isotype control ADCs (iso-ADC1 and iso-ADC3), as well as with the free payloads of EGFR/MUC-ADC6 (payload EGFR/MUC-ADC6 (exatecan)) and of EGFR/HER3-ADC (payload EGFR/HER3-ADC). The HCC827 cells were plated at a defined cell number one day prior to treatment. After overnight incubation at 37°C and 5% CO2, treatment was performed with a serial dilution of the molecules for 144 hours. Control cells were treated with an equivalent amount of DMSO (for payload controls) or the respective cell culture medium (RPMI with 2 mM stable glutamine, 10% FBS, 2.5 g/L glucose, and 1 mM sodium pyruvate). Background wells (no-cell control) contained medium only. To assess cell viability, the CellTiter-Glo^{®} Luminescent Cell Viability Assay was performed according to the manufacturer's instructions.

### Example 13.4 - In vivo comparison of EGFR/MUC-ADC6 vs. EGFR/HER3-ADC

EGFR/MUC-ADC6 was compared to an EGFR/HER3 ADC (DAR8, TOP1i payload).

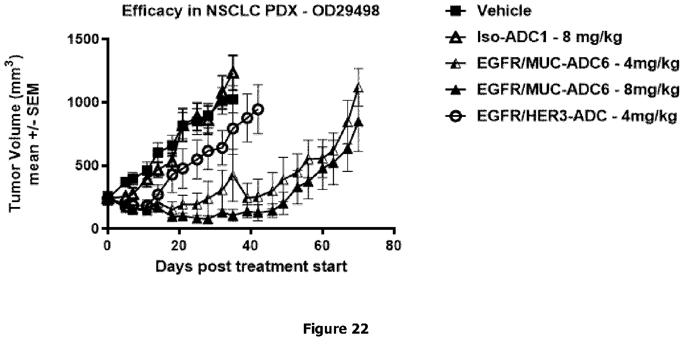

Surprisingly, it was found that in the NSCLC PDX model OD29498, a long-lasting tumor suppression of EGFR/MUC-ADC6 at 4 and at 8 mg/kg (single treatment, i.v. injection) was observed as compared to EGFR/HER3-ADC, which showed a short tumour suppression and fast outgrowth of the tumour (see **Figure 22**)

### Example 14 - Dose-range finding toxicity studies in NHPs (cynomolaus monkeys)

EGFR/MUC-ADC1 and EGFR/MUC-ADC6 were evaluated in dose range finding toxicity studies in non-human primate (cynomolgus monkeys).

### EGFR/MUC-ADC1 - skin toxicity observed at 12 mg/kg or higher doses

Following repeat-dose iv administration of EGFR/MUC-ADC1 to cynomolgus monkeys with a 3-week interval (Q3W), the drug was not tolerated at and above dosages of 12 mg/kg mainly due to formation of multi-focal severe skin lesions. The main effect at planned or premature sacrifices induced by EGFR/MUC-ADC1 was dose-dependent skin and eyelid (Meibomian glands) lesions. Only at 12 mg/kg or higher dose levels, other test article-induced adverse morphological alterations appeared in the cornea, ovaries, thymus, and bone marrow.

At the tolerated dose of 8 mg/kg, pharmacologically mediated skin alterations included hyperkeratosis (up to moderate) and epidermal hyperplasia (up to marked) in all animals, and in a few animals (4 out of 6) erosions and ulcerations (up to moderate), which were fully reversible. Treatment-related changes also occurred in the Meibomian glands (eyelid) and sebaceous glands (skin) at 8 mg/kg and above. At 12 mg/kg or higher only the eye conjunctiva and cornea were affected in addition in both toxicology studies. At 4 mg/kg, pharmacologically mediated minor skin alterations occurred, namely up to mild hyperkeratosis and up to moderate epidermal hyperplasia with up to moderate atrophy of the sebaceous glands in some animals (4 out of 6). These lesions did not impair the integrity of the skin epidermis and were therefore regarded as not adverse and were reversible. Therefore, the highest non-severely toxic dose (HNSTD) was established at 8 mg/kg and the no-observed adverse effect level (NOAEL) at 4 mg/kg Q3W.

The skin and eye toxicities of EGFR/MUC-ADC1 are regarded as on-target EGFR-related toxicities as they are highly similar to EGFR-inhibiting agents such as mAb (e.g. cetuximab) and tyrosine kinase inhibitors (refer to Lacouture 2006, Ho 2013, Guggina 2017 and Lacouture 2018).

### EGFR/MUC-ADC6 - surprisingly no signs of toxicities up to 32 mg/kg doses

To evaluate the safety profile of EGFR/MUC-ADC6, cynomolgus monkeys were dosed with 16, 32 or 64 mg/kg of EGFR/MUC-ADC6 (2x iv administrations, Q3W) in a dose range-finding (DRF) study.

As a result, there was no unscheduled death at 16 and 32 mg/kg, whereas both animals given 64 mg/kg were euthanized for ethical reasons on Day 28 instead of Day 29. For both animals, slight to severe liquid feces, decreased activity, low body temperature and changes in clinical pathology parameters were noted before sacrifice. At 16 and 32 mg/kg, no remarkable effects on body weight were observed. Very surprisingly, in contrast to EGFR/MUC-ADC1, no effects on skin were observed by visual inspection in any group.

Changes in hematology parameters were mainly observed at 64 mg/kg throughout the study period, with decreases in neutrophil, lymphocyte and monocyte counts.

Microscopic findings were mainly noted at 64 mg/kg in the gastrointestinal tract (esophagus, stomach, and small and large intestine). In the hematopoietic/lymphoid organs decreased cellularity was observed in bone marrow, thymus, spleen and lymph nodes. There was no clear evidence of microscopic treatment-related findings in gastrointestinal tract and/or lymphoid organs at 32 mg/kg. Minimal, non-adverse skin findings (epidermal hyperkeratosis) were seen at 32 and 64 mg/kg mainly at injection sites. No toxicological evidence of treatment-related findings was seen at 16 mg/kg.

### PK study of EGFR/MUC-ADC1 and ADC6

From a TK perspective, to evaluate the exposure to EGFR/MUC-ADC6 total antibody, conjugated exatecan and unconjugated exatecan were quantified in plasma samples.

Total antibody was determined using a three-step immunoassay sandwich method performed on the Gyrolab platform. Anti-idiotypes generated against the two arms of the bispecific antibody were used as detection and capture reagents, respectively. Free exatecan was assessed by a LC-MS/MS method and conjugated exatecan by using an Hybrid immunocapture LC-MS/MS method. It consists of a Immunoaffinity capture step on magnetic beads followed by linkerspecific papain+B-glucuronidase digestion. PK/TK analyses were performed using Phoenix WinNonlin^{®} software (Pharsight Corporation, USA). A non-compartmental (model independent) approach was applied.

After intravenous infusion, peak concentration of both total antibody and conjugated exatecan was generally observed at the end of the infusion (i.e., 0.5 h after the start of dosing) with individual values ranging from 0.5 to 6 hours. Nonlinear pharmacokinetics was observed from 16 to 64 mg/kg with slower elimination in the high dose group: CL decreased, t1/2 increased and Vss did not change with the increasing dose. This finding could potentially indicate a target mediated clearance.

The peak concentration of free exatecan was reached quickly after infusion with the Cmax generally observed 2 h after administration. After the peak concentration, free exatecan plasma profile was parallel to conjugated exatecan suggesting formation-limited pharmacokinetics. No accumulation of total antibody and conjugated exatecan was observed in any dose group.

EGFR/MUC-ADC6 showed similar PK/TK properties in the NHP DRF study compared to EGFR/MUC-ADC1 as depicted in the table below, where total antibody data are shown.

**Table 11: Pharmacokinetics of EGFR/MUC-ADC6 and EGFR/MUC-ADC1 in NHP DRF toxicity studies (Total Antibody data).**

| MBE | Dose (mg/kg) | t1/2 (h) | *Cl (mL/h/kg) |
|---|---|---|---|
| **EGFR/MUC-ADC6** | 16 | 77 | 0.804 |
| **EGFR/MUC-ADC1** | 15 | 69 | 0.687 |
| **EGFR/MUC-ADC6** | 32 | 89 | 0.518 |
| **EGFR/MUC-ADC1** | 30 | 89 | 0.438 |
| **EGFR/MUC-ADC6** | 64 | 132 | 0.348 |

### Concluding remarks

In conclusion, 2 iv administrations (Q3W) of EGFR/MUC-ADC6 were well tolerated in cynomolgus monkeys at dose levels of 16 and 32 mg/kg, whereas 64 mg/kg was not tolerated. Adverse target organ effects were observed at 64 mg/kg and consisted of intestinal tract and hematopoietic/lymphoid tissues findings associated with changes in hematology parameters. The low dose of 16 mg/kg was considered to be the NOEL, and the NOAEL was set at 32 mg/kg.

Thus, the results of the DRF with EGFR/MUC-ADC6 surprisingly show a strongly improved safety profile compared to EGFR/MUC-ADC1.

## Claims

1. An immunoconjugate comprising:
(a) a bispecific antibody that binds to EGFR and MUC1,
(b) a growth inhibiting agent, and
(c) a linker covalently linking (a) and (b).

2. The immunoconjugate of claim 1, wherein the bispecific antibody comprises
- an EGFR binding portion comprising
o a heavy chain variable region (VH) comprising
a heavy chain complementary determining region (HCDR) 1 of SEQ ID NO: 1,
a HCDR 2 of SEQ ID NO: 2, and
a HCDR 3 of SEQ ID NO: 3, and
∘ a light chain variable region (VL) comprising
a light chain complementarity determining region (LCDR) 1 of SEQ ID NO: 4,
a LCDR 2 of SEQ ID NO: 5, and
a LCDR 3 of SEQ ID NO: 6, and
- a MUC1 binding portion comprising
o a heavy chain variable region (VH) comprising
a heavy chain complementary determining region (HCDR) 1 of SEQ ID NO: 7,
a HCDR 2 of SEQ ID NO: 8, and
a HCDR 3 of SEQ ID NO: 9, and
o a light chain variable region (VL) comprising
a light chain complementarity determining region (LCDR) 1 of SEQ ID NO: 10,
a LCDR 2 of SEQ ID NO: 11, and
a LCDR 3 of SEQ ID NO: 12.

3. The immunoconjugate of claim 1 or 2, wherein the bispecific antibody comprises
- an EGFR binding portion comprising
o a heavy chain variable region (VH) comprising
an amino acid sequence that is at least 95%, 96%, 97%, 98%, 99% or 100% identical to the amino acid sequence of SEQ ID NO: 13, and
∘ alight chain variable region (VL) comprising
an amino acid sequence that is at least 95%, 96%, 97%, 98%, 99% or 100% identical to the amino acid sequence of SEQ ID NO: 14,
- a MUC1 binding portion comprising
o a heavy chain variable region (VH) comprising
an amino acid sequence that is at least 95%, 96%, 97%, 98%, 99% or 100% identical to the amino acid sequence of SEQ ID NO: 15, and
∘ a light chain variable region (VL) comprising
an amino acid sequence that is at least 95%, 96%, 97%, 98%, 99% or 100% identical to the amino acid sequence of SEQ ID NO: 16.

4. The immunoconjugate of any one of claims 1 to 3, wherein the bispecific antibody binds to EGFR such as human EGFR with a binding affinity (K_{D}), preferably wherein the K_{D} is less than 10 nM such as less than 5 nM.

5. The immunoconjugate of any one of claims 2 to 4, wherein the bispecific antibody comprises:
(i) a first polypeptide comprising a first Fc domain and a single-chain Fv fragment (scFv), wherein the scFv comprises the VH and VL of the MUC1 binding portion,
(ii) a second polypeptide comprising a second Fc domain and a heavy chain of a Fab fragment comprising the VH of the EGFR binding portion, and
(iii) a third polypeptide comprising a light chain of the Fab fragment comprising the VL of the EGFR binding portion.

6. The immunoconjugate of claim 5, wherein
- the first polypeptide comprises a T366W substitution, and the second polypeptide comprises at least one of T366S, L368A, and Y407V substitutions, or
- the second polypeptide comprises a T366W substitution, and the first polypeptide comprises at least one of T366S, L368A, and Y407V substitutions,
and/or
- the first polypeptide comprises a S354C substitution, and the second polypeptide comprises a Y349C substitution, or
- the second polypeptide comprises a S354C substitution, and the first polypeptide comprises a Y349C substitution,
wherein EU numbering is used for said amino acid substitutions.

7. The immunoconjugate of claim 5 or 6, wherein the first and/or the second polypeptide comprises
- a C226S substitution,
- a L234A and L235A (LALA) substitution, and/or
- a D356E and L358M substitution,
wherein EU numbering is used for said amino acid substitutions.

8. The immunoconjugate of any one of claims 5 to 7, wherein the
- the first polypeptide comprises
an amino acid sequence that is at least 90%, 95%, 96%, 97%, 98%, 99% or 100% identical to the amino acid sequence of SEQ ID NO: 17,
- the second polypeptide comprises
an amino acid sequence that is at least 90%, 95%, 96%, 97%, 98%, 99% or 100% identical to the amino acid sequence of SEQ ID NO: 18, and
- the third polypeptide comprises
an amino acid sequence that is at least 90%, 95%, 96%, 97%, 98%, 99% or 100% identical to the amino acid sequence of SEQ ID NO: 19.

9. The immunoconjugate of any one of claims 1 to 8, wherein the growth inhibitory agent is a topoisomerase I inhibitor such as exatecan.

10. The immunoconjugate of any one of claims 1 to 9, wherein the linker is a cleavable linker, preferably a β-glucuronide linker.

11. The immunoconjugate of any one of claims 1 to 10, wherein the linker and the growth inhibiting agent in combination represents a drug-linker compound, preferably wherein the drug-linker compound is conjugated to the bispecific antibody at an average drug-to-antibody ratio (DAR) from 2 to 8 or about 4 such as 3.5 to 4.5 or 3.8 to 4.2.

12. An immunoconjugate of any one of claims 1 to 11 comprising
(a) a bispecific antibody that binds to EGFR and MUC1 comprising an EGFR binding portion and a MUC1 binding portion,
(b) a growth inhibiting agent, and
(c) a linker covalently linking (a) and (b),
wherein the bispecific antibody comprises
- a first polypeptide comprising a first Fc domain and a single-chain Fv fragment (scFv), wherein the scFv comprises a VH and a VL of the MUC1 binding portion, wherein said first polypeptide comprises the amino acid sequence of SEQ ID NO: 17,
- a second polypeptide comprising a second Fc domain and a heavy chain of a Fab fragment comprising a VH of the EGFR binding portion, wherein said second polypeptide comprises the amino acid sequence of SEQ ID NO: 18, and
- a third polypeptide comprising a light chain of the Fab fragment comprising a VL of the EGFR binding portion, wherein said third polypeptide comprises the amino acid sequence of SEQ ID NO: 19,
wherein the growth inhibiting agent is exatecan, and the linker is a β-glucuronide linker comprising a maleimide moiety, wherein the immunoconjugate has the following formula **(II):** 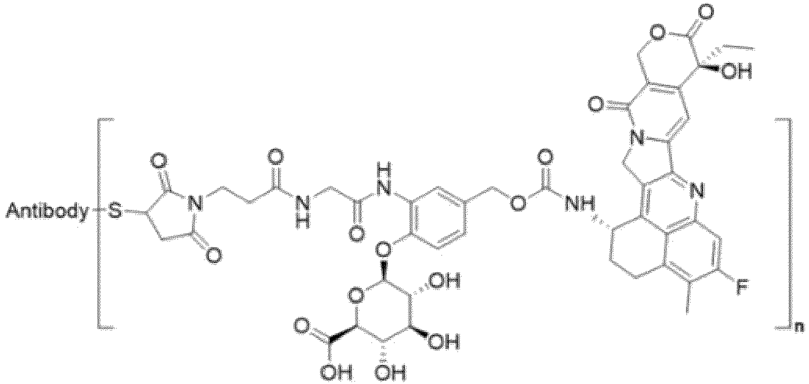
wherein "n" represents an average drug-to-antibody ratio (DAR), wherein n is 3.8 to 4.2,
wherein the "S" is a sulfur atom of a cysteine of the bispecific antibody, wherein the cysteine of the bispecific antibody is one of the cysteines capable of forming an interchain disulfide bond.

13. A pharmaceutical composition comprising the immunoconjugate of any one of claims 1 to 12, and a pharmaceutically acceptable excipient.

14. The immunoconjugate of any one of claims 1 to 12, or the pharmaceutical composition of claim 13, for use in the treatment of cancer in a subject, wherein the subject is administered the pharmaceutical composition or the immunoconjugate, wherein the subject is human, preferably wherein the cancer is a MUC1 and/or EGFR expressing cancer.

15. The immunoconjugate or the pharmaceutical composition for use of claim 14, wherein the subject is administered at least 1 mg/kg of the immunoconjugate, or from 1 mg/kg to 10mg/kg of the immunoconjugate, preferably qw, q2w, or q3w.
